(19) Europäisches Patentamt
European Patent Office
Office européen des brevets

(11) **EP 3 786 166 A1**

(12) **EUROPEAN PATENT APPLICATION**
published in accordance with Art. 153(4) EPC


(43) Date of publication:
**03.03.2021 Bulletin 2021/09**

(21) Application number: **19791541.6**

(22) Date of filing: **25.04.2019**

(51) Int Cl.:
*C07D 498/04* (2006.01) *C07D 513/04* (2006.01)
*C07D 487/04* (2006.01) *A61K 31/542* (2006.01)
*A61P 35/00* (2006.01)

(86) International application number:
**PCT/CN2019/084373**

(87) International publication number:
**WO 2019/206237 (31.10.2019 Gazette 2019/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.04.2018 CN 201810388202**



(71) Applicant: **Etern Biopharma (Shanghai) Co., Ltd.**
**Shanghai 201210 (CN)**

(72) Inventors:
- **ZHU, Jidong**
  **Shanghai 200032 (CN)**
- **CAO, Hengyi**
  **Shanghai 200032 (CN)**
- **SUN, Lin**
  **Shanghai 200032 (CN)**

(74) Representative: **V.O.**
**P.O. Box 87930**
**2508 DH Den Haag (NL)**


(54) **2-AMINOPYRIMIDINE DERIVATIVE, PREPARATION METHOD AND USE THEREOF**


(57) The disclosure provides an aminopyrimidine derivative for preventing and treating diseases related to IDH mutation, a preparation method and use thereof. Specifically, the disclosure provides a compound of Formula I, a stereoisomer, racemate thereof, or pharmaceutically acceptable salt thereof. The compound of the general Formula I has isocitrate dehydrogenase 1 (IDH1) inhibitory activity and can treat cancer induced by IDH1 mutation.

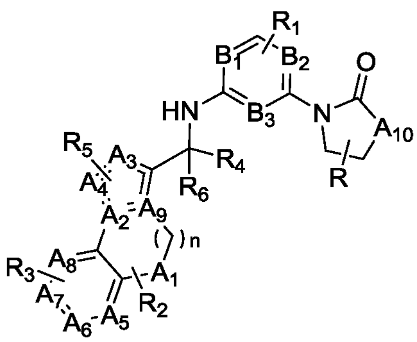

I


EP 3 786 166 A1

## Description

### Technical Field

This disclosure is in the field of chemical synthesis. Specifically, the disclosure relates to 2-aminopyrimidine derivatives with inhibitory activity on mutant IDH, and preparation method and use thereof.

### Background

Malignant tumor is one of the major diseases threatening human health. 8.5 million people died of cancer in 2008 worldwide, and there will be 20 million new cancer cases by 2020 according to current trends, including 12 million deaths. The prevention and treatment of tumor has become an important research topic in the medical field of various countries. Although people have a better understanding of the cause and development of tumor and a large number of anti-tumor drugs are used in clinic, these widely used anti-tumor drugs often have serious side effects and are easy to produce drug resistance, which limits their clinical treatment. Therefore, it is of great significance to develop new, efficient and low toxicity anti-tumor drugs.

Isocitrate dehydrogenases (IDH) catalyze the oxidative decarboxylation of isocitrate to 2-oxoglutarate (a-ketoglutarate), and produce carbon dioxide and NADPH/NADH simultaneously. This process plays an important role in cell metabolism. According to the different electron acceptors, these enzymes can be divided into two subtypes: one using NAD(+), and the other using NADP(+). Among the five isocitrate dehydrogenases reported, three are NAD(+) dependent isocitrate dehydrogenases, which mainly exist in mitochondrial matrix; the other two are NADP(+) dependent isocitrate dehydrogenase 1 and isocitrate dehydrogenase 2. Isocitrate dehydrogenase 1 mainly exists in cytoplasm, and isocitrate dehydrogenase 2 mainly exists in mitochondria. Isocitrate dehydrogenase mutations occur in many types of cancer, including but not limited to: brain glioma, glioblastoma, paraneurocytoma, acute leukemia, prostate cancer, thyroid cancer, colon cancer, chondrosarcoma, cholangiocarcinoma, peripheral T-cell leukosis, melanoma, etc.

Non-mutant IDH1 catalyzes the oxidative decarboxylation of isocitrate to α-ketoglutarate, thereby reducing NAD+ (NADP+) to NADP (NADPH) in the following forward reactions:

$$\text{Isocitrate + NAD+ (NADP+)} \rightarrow \alpha\text{-ketoglutarate} + CO_2 + \text{NADH (NADPH)}.$$

However, the mutant isocitrate dehydrogenases lose the above normal function and instead catalyze the NAPH dependent reduction of α-ketoglutarate to R(-)-2-hydroxyglutarate (2HG). The structure of 2-hydroxyglutaric acid (2-HG) is similar to that of α-KG, which leads to the competition between 2-HG and α-KG. Both of the above two factors will reduce the activity of some α-KG dependent dioxygenases, including proline hydroxylase (PHD), DNA hydroxylase Tet family and histone lysine demethylases (KDMs), and eventually lead to the occurrence of tumor. For example, high concentrations of 2-HG were detected in acute leukemia patients with IDH mutations. High concentration of 2HG is closely related to carcinogenic genes. Therefore, there is an urgent need to develop mutant IDH inhibitors in the art.

### SUMMARY

One object of the disclosure is to provide a compound of Formula I or a pharmaceutically acceptable salt thereof, a pharmaceutical composition comprising the compound or the pharmaceutically acceptable salt thereof, and use of the compound or the pharmaceutical composition in the prevention or treatment of diseases related to IDH mutation.

The first aspect of the disclosure provides a compound of Formula I, or a stereoisomer, racemate, or pharmaceutically acceptable salt thereof, or an isotope-substituted derivative thereof.

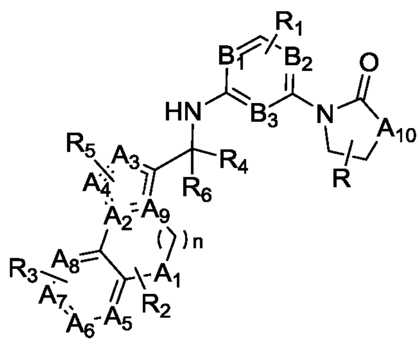

I

wherein,

n is 0, 1, 2 or 3;

$A_1$ is selected from the group consisting of: a bond, $-CH_2-$, -CH(R)-, $-C(R)_2-$, $-CH_2O-$, -CH(R)O-, $-C(R)_2O-$, $-CH_2N(R)-$, -CH(R)N(R)-, $-C(R)_2N(R)-$, -CH=CH-, -C(R)=CH-, -C(R)=C(R)-, -CH=N-, -C(R)=N-, -NR-, -O-, -S-,

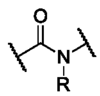 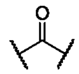 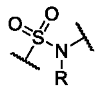 ;

$A_2$, $A_3$, $A_4$, $A_5$, $A_6$, $A_7$, $A_8$, $A_9$, $A_{10}$ are independently selected from the group consisting of: $C(R)_{2-}$, CH(R), NR, O, S, CR or N;

$B_1$, $B_2$, $B_3$ are independently selected from the group consisting of: CR or N;

the dotted line represents a double bond or null;

R is selected from the group consisting of: H, halogen, CN, substituted or unsubstituted C1-C4 alkyl, substituted or unsubstituted C1-C4 alkoxy, substituted or unsubstituted $C_3$-$C_{10}$ cycloalkyl, substituted or unsubstituted $C_6$-$C_{12}$ aryl, and substituted or unsubstituted 3 to 12-membered heteroaryl; or two R groups together form a group selected from the group consisting of: substituted or unsubstituted $-(CH_2)_m-$ structure, substituted or unsubstituted $-CH_2-O-CH_2-$ structure, and substituted or unsubstituted $-O-CH_2-O-$ structure; wherein, m is 2 or 3; or when two R groups are connected to the same carbon atom, the two R groups form a C=O double bond (carbonyl group) with the carbon atom.

$R_4$, $R_6$ are independently selected from the group consisting of: hydrogen, deuterium, methyl, trideuteriomethyl, dideuteriomethyl, monodeuteriomethyl, ethyl, trifluoromethyl, difluoromethyl, monofluoromethyl, substituted or unsubstituted C1-C4 alkyl, and substituted or unsubstituted C1-C4 alkoxy; or $R_4$ and $R_6$ together form a group selected from the group consisting of: =O (forming a carbonyl group with the carbon atom it connects thereto), substituted or unsubstituted $-(CH_2)_m-$ structure, and substituted or unsubstituted $-CH_2-O-CH_2-$structure; wherein, m is 2 or 3;

$R_1$, $R_2$, $R_3$, $R_5$ are independently one or more substituents that correspond to any position on the five-membered or six-membered ring, and are selected from the group consisting of: H, hydroxyl, cyano, halogen, trifluoromethyl, substituted or unsubstituted C1-C4 alkyl, substituted or unsubstituted C1-C4 alkoxy, substituted or unsubstituted $C_3$-$C_{10}$ cycloalkyl, substituted or unsubstituted $C_6$-$C_{12}$ aryl, and substituted or unsubstituted 3 to 12-membered heteroaryl;

wherein, each chiral center can be independently R configuration or S configuration;

the term "substituted" refers to that one or more hydrogen atoms on the group are substituted by substituent(s) selected from the group consisting of: halogen, amino, cyano, $C_1$-$C_6$ alkyl, $C_1$-$C_6$ alkoxy, $C_1$-$C_6$ halogenated alkyl, carbonyl($C_{2-10}$)alkoxy, carbonyl($C_{7-10}$)aryloxy, acylamino($C_{2-10}$)alkyl, unsubstituted $C_6$-$C_{12}$ aryl or 3 to 12-membered heteroaryl, or $C_6$-$C_{12}$ aryl or 3 to 12-membered heteroaryl substituted by 1-3 substituents selected from the group consisting of: halogen, unsubstituted or halogenated $C_1$-$C_6$ alkyl, and $C_1$-$C_6$ alkoxy.

In another preferred embodiment, the compound has a structure as presented in the following Formula II:

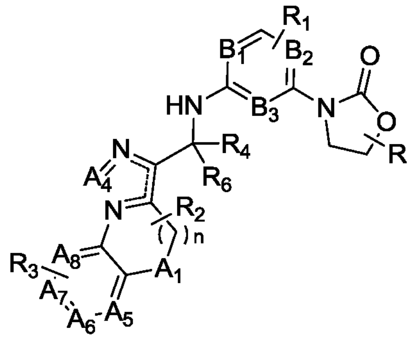

II

wherein, the groups are as defined in the first aspect of the disclosure.

In another preferred embodiment, the compound has a structure as presented in the following Formula III:

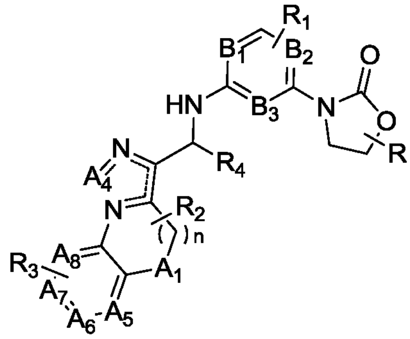

III

wherein, the groups are as defined in claim 1.

In another preferred embodiment, the compound has a structure as presented in the following Formula IV:

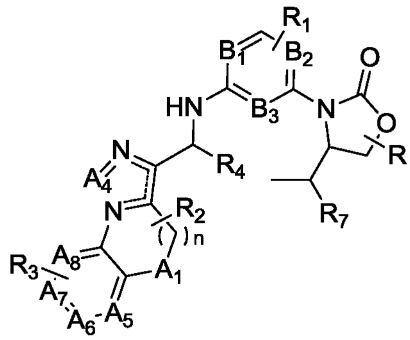

IV

wherein, the groups are as defined in claim 1.

$R_7$ is selected from: H, hydroxyl, cyano, amino, halogen, substituted or unsubstituted C1-C4 alkyl, substituted or unsubstituted C1-C4 alkoxy, substituted or unsubstituted $C_3$-$C_{10}$ cycloalkyl, substituted or unsubstituted $C_6$-$C_{12}$ aryl, and substituted or unsubstituted 3 to 12-membered heteroaryl;

wherein, each chiral center can be independently R configuration or S configuration;

the term "substituted" refers to that one or more hydrogen atoms on the group are substituted by substituent(s) selected from the group consisting of: halogen, amino, cyano, $C_1$-$C_6$ alkyl, $C_1$-$C_6$ alkoxy, $C_1$-$C_6$ halogenated alkyl, carbonyl($C_{2-10}$)alkoxy, carbonyl($C_{7-10}$)aryloxy, acylamino($C_{2-10}$)alkyl, unsubstituted $C_6$-$C_{12}$ aryl or 3 to 12-membered heteroaryl, or $C_6$-$C_{12}$ aryl or 3 to 12-membered heteroaryl substituted by 1-3 substituents selected from the group

consisting of: halogen, unsubstituted or halogenated $C_1$-$C_6$ alkyl, and $C_1$-$C_6$ alkoxy.

In another preferred embodiment, n=1, and $A_1$ is $CR_2$ or O.

In another preferred embodiment, R is selected from the group consisting of: H, halogen, CN, substituted or unsubstituted C1-C4 alkyl, substituted or unsubstituted C1-C4 alkoxy, substituted or unsubstituted $C_3$-$C_{10}$ cycloalkyl, substituted or unsubstituted $C_6$-$C_{12}$ aryl, and substituted or unsubstituted 3 to 12-membered heteroaryl; or two R groups together form a group selected from the group consisting of: substituted or unsubstituted $-(CH_2)_2-$ structure, and substituted or unsubstituted $-CH_2-O-CH_2-$ structure; or when two R groups are connected to the same carbon atom, the two R groups form a C=O double bond (carbonyl group) with the carbon atom.

In another preferred embodiment, $R_1$, $R_2$, $R_3$, $R_4$, $R_5$ are independently one or more groups that correspond to any position on the five-membered or six-membered ring and that are selected from the group consisting of: H, halogen, substituted or unsubstituted C1-C4 alkyl, and substituted or unsubstituted C1-C4 alkoxy.

The second aspect of the disclosure provides a pharmaceutical composition, the pharmaceutical composition comprising: (a) the compound of Formula I as described in the first aspect of the disclosure as an active ingredient, or a racemate, R-isomer, S-isomer or pharmaceutically acceptable salt thereof, or their mixture, or an isotope-substituted derivative thereof, and (b) a pharmaceutically acceptable excipient.

In another preferred embodiment, the pharmaceutical composition further comprises (c) a second active ingredient.

In another preferred embodiment, the pharmaceutical composition is used for the treatment or prevention of solid tumor(s) carrying IDH1 mutation, preferably, the pharmaceutical composition is used for the treatment or prevention of indication(s) selected from the group consisting of: brain glioma, glioblastoma, paraneuroma, acute leukemia, prostate cancer, thyroid cancer, colorectal cancer, chondrosarcoma, cholangiocarcinoma, leukemia, and melanoma.

The third aspect of the disclosure provides use of the compound as decribed in Formula I, or a racemate, R-isomer, S-isomer or pharmaceutically acceptable salt thereof, or an isotope-substituted derivative thereof, or their mixture in: (1) preparing a pharmaceutical composition for the treatment or prevention of diseases related to the activity or expression of mutant IDH; (2) preparing a mutant IDH inhibitor.

In another preferred embodiment, the disease is a solid tumor carrying IDH1 mutation, preferably selected from the group consisting of: brain glioma, glioblastoma, paraneuroma, acute leukemia, prostate cancer, thyroid cancer, colorectal cancer, chondrosarcoma, cholangiocarcinoma, leukemia, melanoma.

In another preferred embodiment, the tumor is selected from the group consisting of: neuroglioma, acute myeloid leukemia, sarcoma, prostate cancer, melanoma, non-small cell lung cancer, articular chondroma, and cholangioma.

It should be understood that, within the scope of the present disclosure, the above technical features of the present disclosure and the technical features specifically described in the following (e.g., Examples) can be combined with each other, thereby forming new or preferred technical solution(s). For purposes of simplicity, they will not be described herein.

## DETAILED DESCRIPTION

After long and intensive research, the inventors prepared compounds having the structure of Formula I, and found that they have inhibitory acitivity on mutant IDH1. The compounds have an inhibitory effect on a series of mutant IDH1 at very low concentrations (as low as ≤100nM/L), and the inhibitory activity is quite excellent, so they can be used to treat mutant IDH1-related diseases, such as tumors. Based on the above discovery, the inventors completed the invention.

### Term description

Unless defined otherwise, all technical and scientific terms used herein have the same meaning commonly understood by those of ordinary skill in the art to which this disclosure belongs.

As used herein, when used in reference to a specific enumerated value, the term "about" means that the value may vary by no more than 1% from the enumerated value. For example, as used herein, the expression "about 100" includes all values between 99 and 101 (e.g., 99.1, 99.2, 99.3, 99.4, etc.).

As used herein, the terms "include", "comprise" and "contain" may be open-ended, semi closed-ended and closed-ended. In other words, the terms also include the meaning of "substantially consist of', or "constit of'.

### Definition of groups

The definitions of standard chemical terms can be found in references (including Carey and Sundberg "ADVANCED ORGANIC CHEMISTRY 4TH ED." Vols. A (2000) and B (2001), Plenum Press, New York). Unless specified

otherwise, conventional methods within the scope of the art, e.g., mass spectroscopy, NMR, IR and UV/VIS spectroscopy and pharmacology methods, are used. Unless specified otherwise, the terms used in the present disclosure related to analytical chemistry, organic synthetic chemistry, medicine and pharmaceutical chemistry herein are known in the art. Standard techniques can be used in chemical synthesis, chemical analysis, medicament preparation, formulations and delivery, and in the treatment of patients. For example, reactions and purifications may be carried out according to the manufacturer’s instructions for use of kit, or in a manner well known in the art, or according to the description of the disclosure. Generally, the above techiques and methods can be implemented in accordance with the conventional methods well known in the art according to the descriptions in the multiple general and more specific literatures cited and discussed in the present description. In the present description, groups and substituents may be selected by a person skilled in the art to provide stable structural moieties and compounds.

When a substituent is described by a conventional formula written from left to right, the substituent also includes the chemically equivalent substituent obtained when the structural formula is written from right to left. For example, $-CH_2O-$ is equal to $-OCH_2-$.

Some chemical groups defined herein are preceded by simplified symbols to represent the total number of carbon atoms in the group. For example, $C_1$-$C_6$ alkyl refers to an alkyl as defined below having a total of 1 to 6 carbon atoms. The total number of carbon atoms in the simplified symbols does not comprise carbon atoms that may exist in the substituents of the group.

In addition to the foregoing, when used in the specification and claims of the disclosure, unless specifically indicated otherwise, the following terms have the meanings as follows.

In the disclosure, the term "halogen" refers to fluorine, chlorine, bromine or iodien.

"Hydroxyl" refers to -OH group.
"Hydroxyl alkyl" refers to an alkyl as defined below substituted by hydroxyl (-OH).
"Carbonyl" refers to -C(=O)- group.
"Nitro" refers to $-NO_2$.
"Cyano" refers to -CN.
"Amino" refers to $-NH_2$.
"Substituted amino" refers to an amino substituted by one or two of the alkyl, alkyl carbonyl, aryl alkyl, heteroaryl alkyl as defined below, for exemple, substituted amino may be monoalkyl amino, dialkyl amino, alkyl acylamino, arylalkyl amino, heteroarylalkyl amino.
"Carboxyl" refers to -COOH.

In the disclosure, as a group or moiety of other groups (for example, used in groups such as halogenated alkyl), the term "alkyl" refers to a fully saturated straight or branched hydrocarbon chain group, consisting only of carbon atoms and hydrogen atoms, for example, comprising 1 to 12 (preferably 1 to 8, more preferably 1 to 6) carbon atoms, and connected to the rest of the molecule by a single bond, for example, including but not limited to methyl, ethyl, n-propyl, isopropyl, n-butyl, isobutyl, sec-butyl, tert-butyl, n-amyl, 2-methylbutyl, 2,2-dimethylpropyl, n-hexyl, heptyl, 2-methylhexyl, 3-methylhexyl, octyl, nonyl and decyl, etc. In the case of the present disclosure, the term "alkyl" refers to an alkyl group containing 1 to 6 carbon atoms.

In the disclosure, as a group or moiety of other groups, the term "alkenyl" refers to a straight or branched hydrocarbon chain group, consisting only of carbon atoms and hydrogen atoms, for example, comprising 2 to 14 (preferably 2 to 10, more preferably 2 to 6) carbon atoms, comprsing at least one double bond, and connected to the rest of the molecule by a single bond, for example, including but not limited to vinyl, propenyl, allyl, but-1-enyl, but-2-enyl, pent-1-enyl, pent-1,4-dienyl, etc.

In the disclosure, as a group or moiety of other groups, the term "cyclic hydrocarbyl" refers to a stable non-aromatic monocyclic or polycyclic hydrocarbyl consisting only of carbon atoms and hydrogen atoms, which may comprise fused ring system, bridged ring system or spiro ring system, comprise 3 to 15 carbon atoms, preferably comprise 3 to 10 carbon atoms, more preferably comprise 3 to 8 carbon atoms, and which is saturated or unsaturated and may be connected to the rest of the molecule via any suitable carbon atom by a single bond. Unless otherwise specifically indicated in the description, the carbon atoms in the cyclic hydrocarbyl may be optionally oxidized. Embodiments of cyclic hydrocarbyl include but are not limited to cyclopropyl, cyclobutyl, cyclopentyl, cyclopentenyl, cyclohexyl, cyclohexenyl, cyclohexadienyl, cycloheptyl, cyclooctyl, 1H-indenyl, 2,3-dihydroindenyl, 1,2,3,4-tetrahydro-naphthyl, 5,6,7,8-tetrahydro-naphthyl, 8,9-dihydro-7H-benzocyclohepten-6-yl, 6,7,8,9-tetrahydro-5H-benzocycloheptenyl, 5,6,7,8,9,10-hexahydro-benzocyclooctenyl, fluorenyl, bicyclo[2.2.1]heptyl, 7,7-dimethyl-bicyclo[2.2.1]heptyl, bicyclo[2.2.1]heptenyl, bicyclo[2.2.2]octyl, bicyclo[3.1.1]heptyl, bicyclo[3.2.1]octyl, bicyclo[2.2.2]octenyl, bicyclo[3.2.1]octenyl, adamantyl, octahydro-4,7-methylene-1H-indenyl and octahydro-2,5-methylene-dicyclopentadienyl, etc.

In the disclosure, as a group or moiety of other groups, the term "heterocyclyl" refers to a stable 3 to 20-membered non-aromatic cyclic group consisting of 2 to 14 carbon atoms and 1 to 6 heteroatoms selected from nitrogen,

phosphorus, oxygen or sulfur. Unless otherwise specifically indicated, a heterocyclyl may be a monocyclic ring system, a dicyclic ring system, a tricyclic ring system or a ring system with more rings, and may comprise fused ring system, bridged ring system or spiro ring system; the nitrogen, phosphorus or sulfur atoms in the heterocyclyl may be optionally oxidized; the nitrogen atoms in the heterocyclyl may be optionally quaternized; and the heterocyclyl may be partially or fully saturated. The heterocyclyl may be connected to the rest of the molecule via a carbon atom or a heteroatom by a single bond. In a heterocyclyl containing fused rings, one or more rings may be aryl or heteroaryl as defined below, provided that the connection point between the group and the rest of the molecule is a non-aromatic ring atom. For the purpose of the present disclosure, heterocyclyl is preferably a stable 4 to 11-membered non-aromatic monocyclic, dicyclic, bridged or spiro ring group comprising 1 to 3 heteroatoms selected from nitrogen, oxygen or sulfur, more preferably is a stable 4 to 8-membered non-aromatic monocyclic, dicyclic, bridged or spiro ring group comprising 1 to 3 heteroatoms selected from nitrogen, oxygen or sulfur. Exemplary heterocyclyls include but are not limited to: pyrrolidinyl, morpholinyl, piperazinyl, homopiperazinyl, piperidinyl, thiomorpholinyl, 2,7-diaza-spiro[3.5]nonan-7-yl, 2-oxa-6-aza-spiro[3.3]heptan-6-yl, 2,5-diaza-bicyclo[2.2.1]heptan-2-yl, azacyclobutanyl, pyranyl, tetrahydropyranyl, thiopyranyl, tetrahydrofuranyl, oxazinyl, dioxolanyl, tetrahydroisoquinolinyl, decahydroisoquinolinyl, imidazolinyl, imidazolidinyl, quinolizinyl, thiazolidinyl, isothiazolidinyl, isoxazolidinyl, dihydroindolyl, octahydroindolyl, octahydroisoindolyl, pyrrolidinyl, pyrazolidinyl, phthalimido, etc.

In the disclosure, as a group or moiety of other groups, the term "aryl" refers to a conjugated hydrocarbon ring system group comprising 6 to 18 carbon atoms (preferably comprising 6 to 10 carbon atoms). For the purpose of the present disclosure, aryl may be a monocyclic ring system, a dicyclic ring system, a tricyclic ring system or a ring system with more rings, and may be fused with the cycloalkyl or heterocyclyl as defined above, provided that the aryl and the rest of the molecule are connected via an atom on the aromatic ring by a single bond. Exemplary aryls include but are not limited to phenyl, naphthyl, anthracyl, phenanthrenyl, fluorenyl, 2,3-dihydro-1H-isoindolyl, 2-benzoxazolinone, 2H-1,4-benzoxazin-3(4H)-one-7-yl, etc.

In the disclosure, the term "aryl alkyl" refers to an alkyl as defined above which is substituted by an aryl as defined above.

In the disclosure, as a group or moiety of other groups, the term "heteroaryl" refers to a 5 to 16-membered conjugated ring system group comprising 1 to 15 carbon atoms (preferably comprising 1 to 10 carbon atoms) and 1 to 6 heteroatoms selected from nitrogen, oxygen or sulfur. Unless otherwise specifically indicated, a heteroaryl may be a monocyclic ring system, a dicyclic ring system, a tricyclic ring system or a ring system with more rings, and may be fused with the cycloalkyl or heterocyclyl, provided that the heteroaryl and the rest of the molecule are connected via an atom on the aromatic ring by a single bond. The nitrogen, carbon or sulfur atoms in the heteroaryl may be optionally oxidized; the nitrogen atoms in the heteroaryl may be optionally quaternized. For the purpose of the disclosure, heteroaryl is preferably a stable 5 to 12-membered aromatic group comprising 1 to 5 heteroatoms selected from nitrogen, oxygen or sulfur, and is more preferably a stable 5 to 10-membered aromatic group comprising 1 to 4 heteroatoms selected from nitrogen, oxygen or sulfur, or a 5 to 6-membered aromatic group comprising 1 to 3 heteroatoms selected from nitrogen, oxygen or sulfur. Exemplary heteroaryls include but are not limited to thiophenyl, imidazolyl, pyrazolyl, thiazolyl, oxazolyl, oxadiazolyl, isoxazolyl, pyridyl, pyrimidinyl, pyrazinyl, pyridazinyl, benzimidazolyl, benzopyrazolyl, indolyl, furanyl, pyrrolyl, triazolyl, tetrazolyl, triazinyl, indolizinyl, isoindolyl, indazolyl, isoindazolyl, purinyl, quinolinyl, isoquinolinyl, diazanaphthalenyl, naphthyridinyl, quinoxalinyl, pteridinyl, carbazolyl, carbolinyl, phenanthridinyl, phenanthrolinyl, acridinyl, phenazinyl, isothiazolyl, benzothiazolyl, benzothiophenyl, oxatriazolyl, cinnolinyl, quinazolyl, phenylthio, indolizinyl, phenanthrolinyl, isoxazolyl, phenoxazinyl, phenothiazinyl,
4,5,6,7-tetrahydrobenzo[b]thiophenyl, naphthopyridinyl, [1,2,4]triazolo[4,3-b]pyridazine, [1,2,4]triazolo[4,3-a]pyrazine, [1,2,4]triazolo[4,3-c]pyrimidine, [1,2,4]triazolo[4,3-a]pyridine, imidazo[1,2-a]pyridine, imidazo[1,2-b]pyridazine, imidazo[1,2-a]pyrazine, etc.

In the disclosure, the term "heteroaryl alkyl" refers to an alkyl as defined above which is substituted by heteroaryl as defined above.

The terms "moiety", "structural moiety", "chemical moiety", "group", "chemical group" as employed herein refer to specific part or functional group in the molecular. Chemical moiety is generally considered to be chemical entity embedded or attached to molecule.

"Stereisomer" refers to a compound composed of the same atoms, bonded by the same bonds, but having different three-dimensional structures. The present disclosure will cover various stereoisomers and mixtures thereof.

Unless specified otherwise, when a compound of the present disclosure contains an olefinic double bond, the compound is intended to include E- and Z-geometric isomers.

A compound of the disclosure or pharmaceutically acceptable salt thereof may comprise one or more chiral carbon atoms, and thus may produce enantiomers, diastereomers and other stereoisomeric forms. Each chiral carbon atom can be defined as (R)- or (S)- based on stereochemistry. The disclosure is intended to include all possible isomers, as well as racemates and optically pure forms thereof. A compound of the disclosure can be prepared by using racemate, diastereomer or enantiomer as raw material or intermediate. The optically active isomers can be prepared by using chiral

synthons or chiral reagents, or separated by conventional techniques, such as crystallization and chiral chromatography.

Conventional techniques for preparing/separating individual isomers include chiral synthesis from suitable optically pure precursors, or separation of racemates (or racemates of salts or derivatives) using, for example, chiral high performance liquid chromatography.

In the disclosure, the term "pharmaceutically acceptable salt" includes pharmaceutically acceptable acid addition salts and pharmaceutically acceptable base addition salts.

"Pharmaceutically acceptable acid addition salt" refers to a salt formed with an inorganic acid or an organic acid that can retain the biological effectiveness of the free base without other side effects. Inorganic acid salts include but are not limited to hydrochloride, hydrobromide, sulfate, nitrate, phosphate, etc.; organic acid salts include but are not limited to formate, acetate, 2,2-dichloroacetate, trifluoroacetate, propionate, hexanoate, octanoate, decanoate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate , alginate, ascorbate, salicylate, 4-aminosalicylate, naphthalene disulfonate, etc. These salts can be prepared by methods known in the art.

"Pharmaceutically acceptable base addition salts" refers to a salt formed with an inoragnic base or an organic base that can retain the biological effectiveness of the free acid without other side effects. Salts derived from inorganic bases include but are not limited to sodium salt, potassium salt, lithium salt, ammonium salt, calcium salt, magnesium salt, iron salt, zinc salt, copper salt, manganese salt, aluminum salt, etc. Preferred inorganic salts are ammonium salt, sodium salt, potassium salt, calcium satl and magnesium salt. Salts derived from organic bases include but are not limited to the following salts: primary amines, secondary amines, and tertiary amines, substituted amines, including natural substituted amines, cyclic amines and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucosamine, theobromine, purine, piperazine, piperidine, N-ethylpiperidine, polyamine resin, etc. Preferred organic bases include isopropylamine, diethylamine, ethanolamine, trimethylamine, dicyclohexylamine, choline and caffeine. These salts can be prepared by methods known in the art.

In the disclosure, "pharmaceutical compostion" refers to a formulation of a compound of the present disclosure and a medium generally accepted in the art for delivering a biologically active compound to a mammal (e.g., human). The medium includes a pharmaceutically acceptable carrier. The purpose of the pharmaceutical composition is to promote the administration of living organisms, which facilitates the absorption of active ingredients and thus exerts biological activity. The pharmaceutical composition generally contains an effective amount of the compounds of the disclosure (for example, containing 0.01-100 mg of the compounds of the disclosure).

The term "pharmaceutically acceptable" as used herein refers to a substance (such as a carrier or diluent) that does not affect the biological activity or properties of the compound of the present disclosure, and is relatively non-toxic, that is, the substance can be administered to an individual without causing adverse biological responses or interacting with any components contained in the composition in an undesirable manner.

In the disclosure, "pharmaceutically acceptable excipient" includes but is not limited to any adjuvant, carrier, excipient, glidant, sweetener, diluent, preservative, dye/colorant, corrigent, surfactant, wetting agent, dispersant, suspending agent, stabilizer, isotonic agent, solvent or emulsifier that is approved by relevant government regulatory agency to be acceptable for human or domestic animal use.

"Tumour" in the disclosure includes but is not limited to neuroglioma, sarcoma, melanoma, articular chondroma, cholangioma, leukemia, gastrointestinal stromal tumor, histiocytic lymphoma, non-small cell lung cancer, small cell lung cancer, pancreatic cancer, lung squamous cell carcinoma, lung adenocarcinoma, breast cancer, prostate cancer, liver cancer, skin cancer, epithelioma, cervical cancer, ovarian cancer, colorectal cancer, nasopharyngeal carcinoma, brain cancer, bone cancer, esophageal cancer, melanoma, renal carcinoma, oral cancer and other diseases.

The terms "preventive", "prevent" and "prevention" as employed herein include reducing the possiblity of the occurrence or deterioration of a disease or condition in a patient.

The term "treatment" and other similar synonyms as employed herein include the following meanings:

(i) prevention of the occurrence of a disease or condition in mammals, especially when such mammals are susceptible to the disease or condition but have not been diagnosed with the disease or condition;
(ii) suppression of a disease or condition, that is, inhibition of the development of the disease or condition;
(iii) alleviation of a disease or condition, that is, abatement of the status of the disease or condition; or
(iv) relief of the symptoms caused by the disease or condition.

The term "effective amount", "therapeutically effective amount" or "pharmaceutically effective amount" as used herein refers to the amount of at least one medicament or compound sufficient to relieve one or more symptoms of the disease or condition being treated to a certain extent after administration. The result may be the reduction and/or remission

of signs, symptoms or causes, or any other desired changes in the biological system. For example, an "effective amount" for treatment is the amount of a composition comprising a compound as disclosed herein required to provide a clinically significant disease relief effect. Techniques such as dose escalation tests may be used to determine the effective amount suitable for any individual case.

The terms "taking", "administrated", "administration" and the like refer to a method capable of delivering a compound or composition to a desired site for biological action. These methods include but are not limited to oral route, transduodenal route, parenteral injection (including intravenous, subcutaneous, intraperitoneal, intramuscular, intraarterial injection or infusion), local administration, and transrectal administration. In a preferred embodiment, the compounds and compositions discussed herein are administered orally.

The terms "medicament combination", "medicament co-administration", "combined medication", "administration of other treatments", "administration of other therapeutic agents" and the like as employed herein refer to medical treatment obtained by mixing or combining more than one active ingredient, and include fixed and unfixed combinations of active ingredients. The term "fixed combination" refers to the simultaneous administration of at least one compound described herein and at least one synergistic medicament to a patient in the form of a single entity or a single dosage form. The term "unfixed combination" refers to simultaneous administration, co-administration, or sequential administration at variable intervals of at least one compound described herein and at least one synergistic formulation to a patient in the form of separate entities. These also apply to cocktail therapy, such as the administration of three or more active ingredients.

One skilled in the art should also understand that in the method described below, the functional group of an intermediate compound may need to be protected by an appropriate protecting group. Such functional groups include hydroxyl, amino, sulfydryl and carboxylic acid. Suitable hydroxyl-protecting groups include trialkylsilyl or diarylalkylsilyl (e.g. tert-butyldimethylsilyl, tert-butyldiphenylsilyl or trimethylsilyl), tetrahydropyranyl, benzyl, etc. Suitable protecting groups for amino, amidino and guanidino include tert-butoxycarbonyl, benzyloxycarbonyl, etc. Suitable sulfydryl-protecting groups include -C(O)-R" (wherein R" is alkyl, aryl or aralkyl), p-methoxybenzyl, trityl, etc. Suitable carboxy-protecting groups include alkyl, aryl or aralkyl esters.

Protecting groups may be introduced and removed according to standard techniques known to one skilled in the art and as described herein.

**Compound of Formula I**

The disclosure provides a compound of Formula I, or a stereoisomer, racemate thereof, or pharmaceutically acceptable salt thereof, or an isotope-substituted derivative thereof:

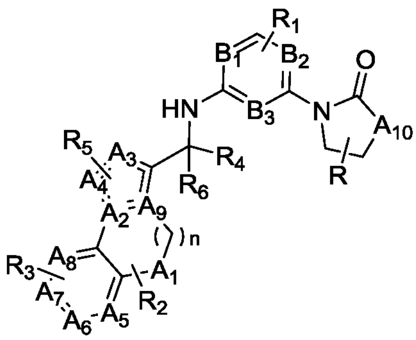

I

wherein,

n is 0, 1, 2 or 3;

$A_1$ is selected from the group consisting of: bond, $-CH_2-$, -CH(R)-, $-C(R)_2-$, $-CH_2O-$, -CHRO-, $-CR_2O-$, $-CH_2N(R)-$, -CH(R)N(R)-, $-CR_2NR-$, -CH=CH-, -CR=CH-, -C(R)=C(R)-, -CH=N-, -C(R)=N-, -NR-, -O-, -S-,

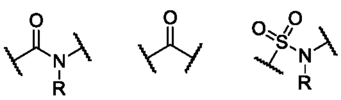;

$A_2$, $A_3$, $A_4$, $A_5$, $A_6$, $A_7$, $A_8$, $A_9$, $A_{10}$ are independently selected from the group consisting of: $C(R)_{2-}$, CH(R), NR, O, S, CR or N;

$B_1$, $B_2$, $B_3$ are independently selected from the group consisting of: CR or N;

the dotted line represents a double bond or null;

R is selected from the group consisting of: H, halogen, CN, substituted or unsubstituted C1-C4 alkyl, substituted or unsubstituted C1-C4 alkoxy, substituted or unsubstituted $C_3$-$C_{10}$ cycloalkyl, substituted or unsubstituted $C_6$-$C_{12}$ aryl, and substituted or unsubstituted 3 to 12-membered heteroaryl; or two R groups together form a group selected from the group consisting of: substituted or unsubstituted $-(CH_2)_m-$ structure, substituted or unsubstituted $-CH_2-O-CH_2-$ structure, and substituted or unsubstituted $-O-CH_2-O-$ structure; wherein, m is 2 or 3; or when two R groups are connected to the same carbon atom, the two R groups form a C=O double bond (carbonyl group) with the carbon atom.

$R_4$, $R_6$ are independently selected from the group consisting of: hydrogen, deuterium, methyl, trideuteriomethyl, dideuteriomethyl, monodeuteriomethyl, ethyl, trifluoromethyl, difluoromethyl, monofluoromethyl, substituted or unsubstituted C1-C4 alkyl, and substituted or unsubstituted C1-C4 alkoxy; or $R_4$ and $R_6$ together form a group selected from the group consisting of: =O (forming a carbonyl group with the carbon atom it connects thereto), substituted or unsubstituted $-(CH_2)_m-$ structure, and substituted or unsubstituted $-CH_2-O-CH_2-$structure; wherein, m is 2 or 3;

$R_1$, $R_2$, $R_3$, $R_5$ are independently one or more substituents corresponding to any position on the five-membered or six-membered ring, and are selected from the group consisting of: H, hydroxyl, cyano, halogen, trifluoromethyl, substituted or unsubstituted C1-C4 alkyl, substituted or unsubstituted C1-C4 alkoxy, substituted or unsubstituted $C_3$-$C_{10}$ cycloalkyl, substituted or unsubstituted $C_6$-$C_{12}$ aryl, and substituted or unsubstituted 3 to 12-membered heteroaryl;

wherein, each chiral center can be independently R configuration or S configuration;

the term "substituted" refers to that one or more hydrogen atoms on the group are substituted by substituent(s) selected from the group consisting of: halogen, amino, cyano, $C_1$-$C_6$ alkyl, $C_1$-$C_6$ alkoxy, $C_1$-$C_6$ halogenated alkyl, carbonyl($C_{2-10}$)alkoxy, carbonyl($C_{7-10}$)aryloxy, acylamino($C_{2-10}$)alkyl, unsubstituted $C_6$-$C_{12}$ aryl or 3 to 12-membered heteroaryl, or $C_6$-$C_{12}$ aryl or 3 to 12-membered heteroaryl substituted by 1-3 substituents selected from the group consisting of: halogen, unsubstituted or halogenated $C_1$-$C_6$ alkyl, and $C_1$-$C_6$ alkoxy.

Preferably, the compound is selected from the group consisting of:

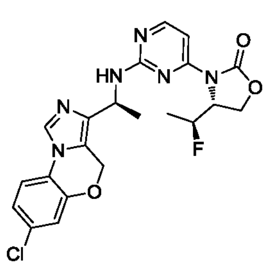
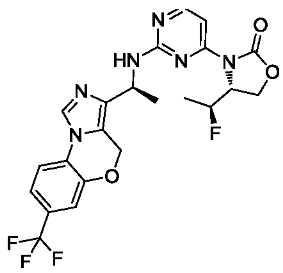
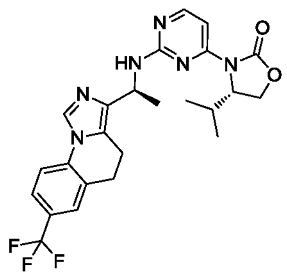
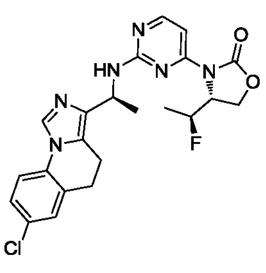
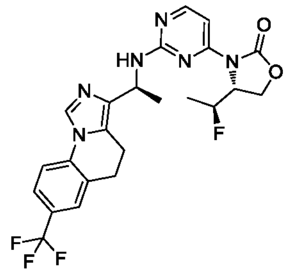
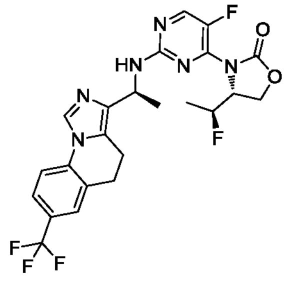

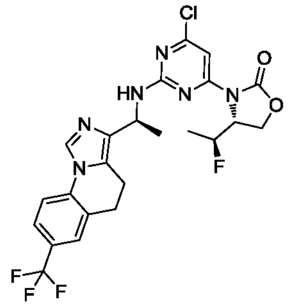
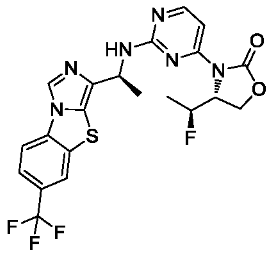
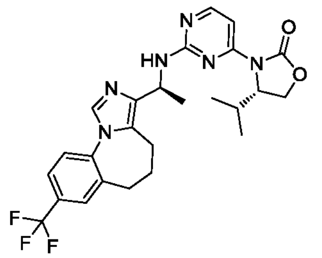

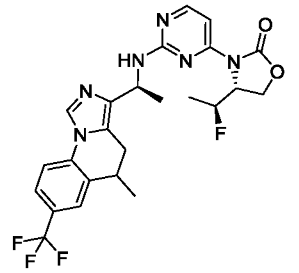
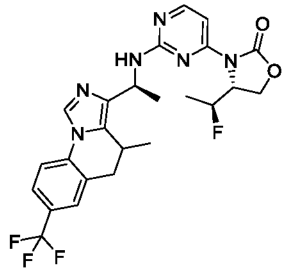
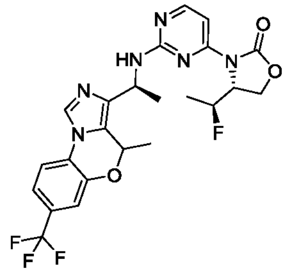

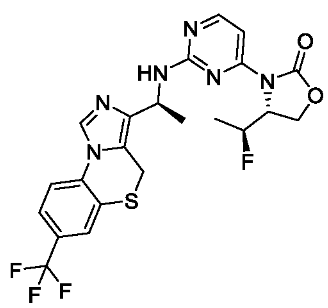
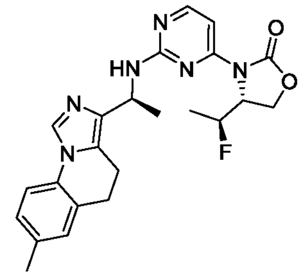
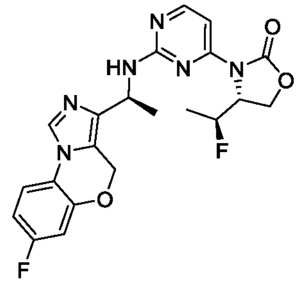

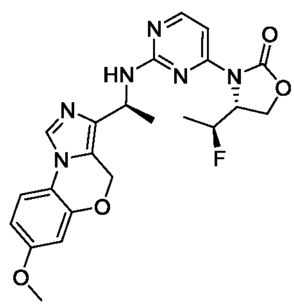
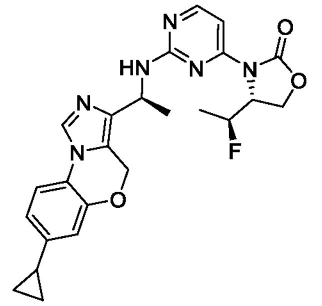
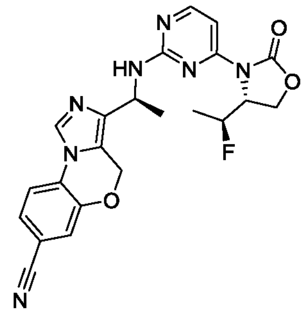

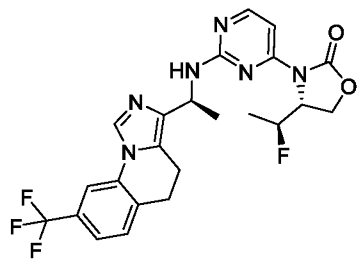
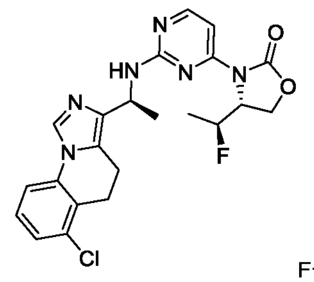
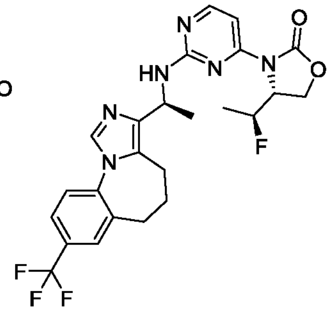

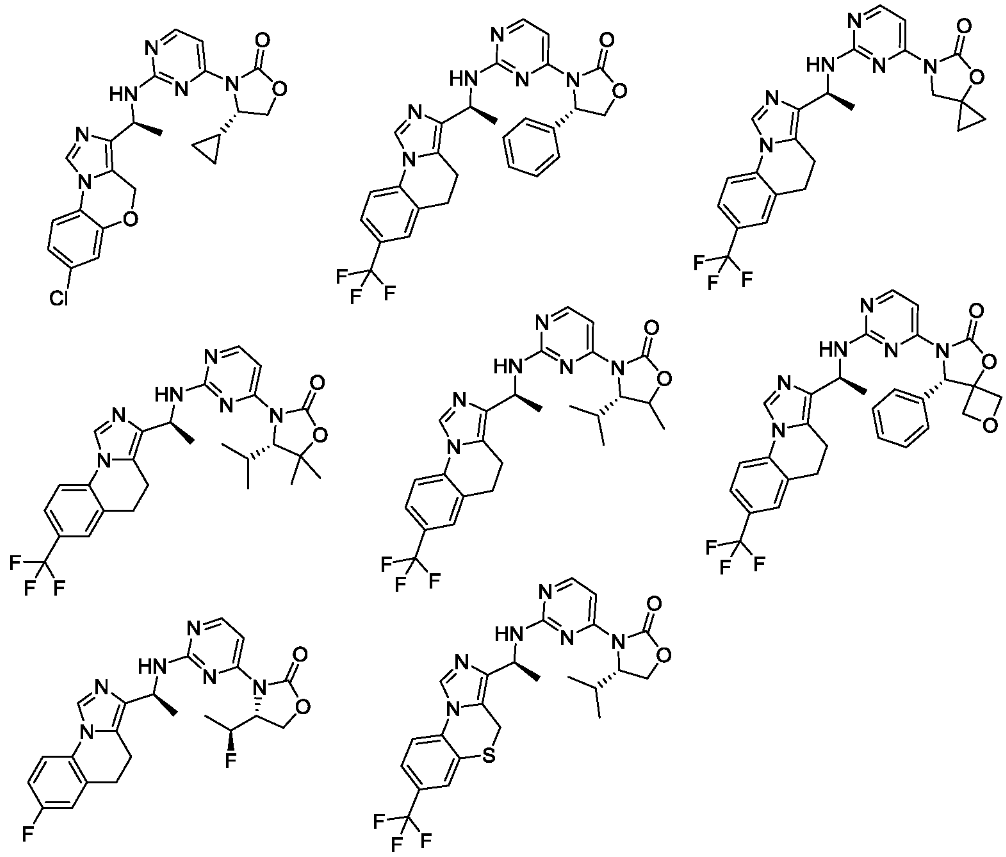

.

## Preparation of the Compound of Formula I

The following reaction schemes illustrate exemplary methods for preparing a compound of Formula I, a stereoisomer thereof or their mixture, or a pharmaceutically acceptable salt thereof, wherein the groups are as described in the embodiments of the compound of Formula I above. It should be understood that in the following reaction schemes, combinations of substituents and/or variables in the general formulas are allowed only if such combinations result in stable compounds.

The disclosure provides a method for preparing the compound of the disclosure, including the subsequent steps:

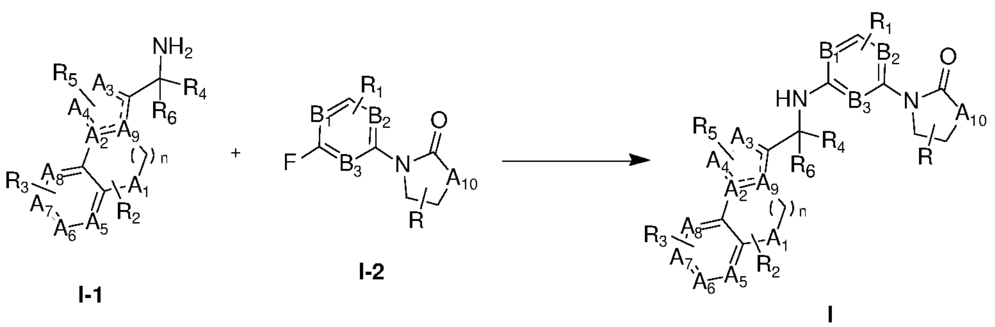

I-1 I-2 I

performing reaction between the compound of Formula 1-1 and the compound of Formula 1-2 to obtain the compound of Formula I.

wherein, the definition of each group is as described above.

## The main advantages of the disclosure include:

1. A compound of Formula I is provided.
2. A novel pharmaceutical composition for the prevention and treatment of IDH mutation-related diseases is provided.
3. A method for preparing the compound of Formula I is provided.

The disclosure is further described below in combination with specific examples. It should be understood that these examples are only used to illustrate the present disclosure and not to limit the scope of the present disclosure. The experimental methods without specific conditions in the following examples generally follow the conventional conditions or the conditions recommended by the manufacturer. Unless otherwise stated, percentages and parts are calculated by weight.

In the examples:

LCMS instrument: Pump Agilent 1100 UV detector: Agilent 1100 DAD
Mass Spectrometer API 3000
Chromatographic column: Waters sunfire C18, 4.6×50mm, 5um
Mobile phase: A-acetonitrile B-$H_2O$ (0.1%FA)

**Synthesis of intermediate A2:**

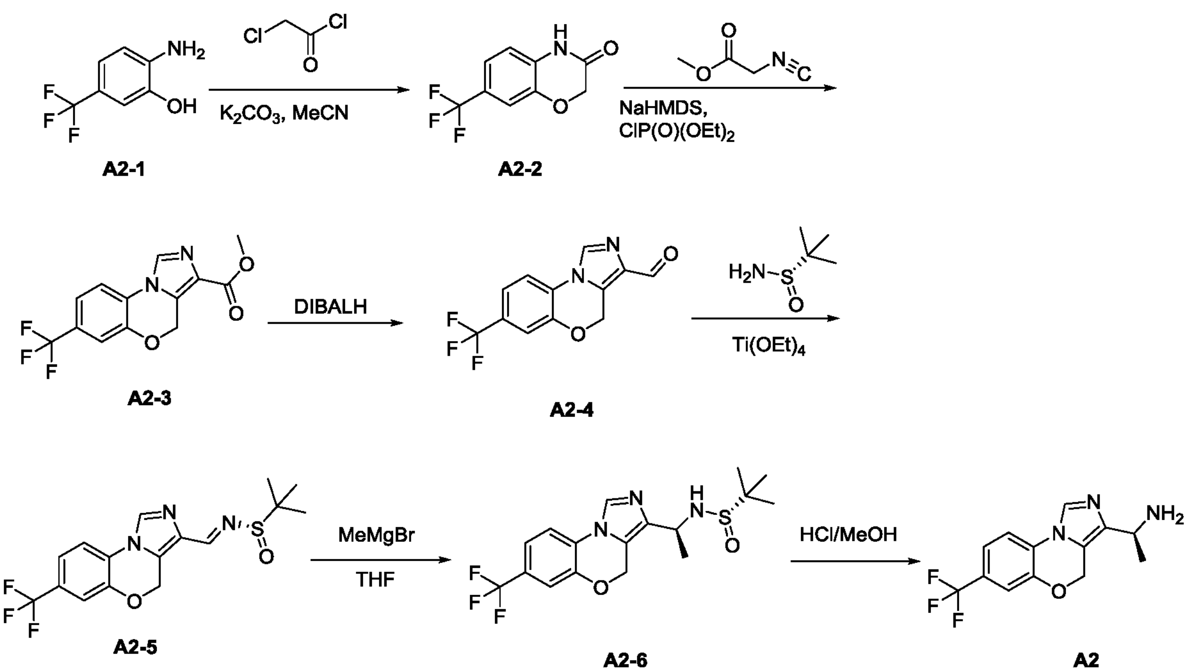


A2-1 A2-2 A2-3 A2-4 A2-5 A2-6 A2

**Step 1: Synthesis of 7-trifluoromethyl-2*H*-benzo[*b*][1,4]oxazin-3(4*H*)-one (A2-2)**

2-amino-5-trifluoromethylphenol (A2-1, 2.0 g, 11.29 mmol) and potassium carbonate (4.7 g, 34.00 mmol) were added to 20 mL acetonitrile. Under the protection of nitrogen, chloroacetyl chloride (1.4 g, 12.40 mmol) was added dropwise. After the adding, the mixture was raised to 85 °C and the reaction solution was stirred under reflux for 2 hours. After the reaction finished, the reaction solution was concentrated, and dissolved with 30 mL ethyl acetate and 20 mL water. The phases were separated, and the organic phase was washed with 20 mL water once. The organic phase was dried with anhydrous sodium sulfate, concentrated, and purified by Combi-Flash flash silica gel chromatography to obtain 1.7g 7-trifluoromethyl-2*H*-benzo[*b*][1,4]oxazin-3(4*H*)-one (A2-2) as a powder with a yield of 69.3%. LCMS: m/z= 218.0$(M+H)^+$, RT= 4.55min.

**Step 2: Synthesis of methyl 7-trifluoromethyl-4*H*-benzo[*b*]imidazo[1,5-*d*][1,4]oxazine-3-carboxylate (A2-3)**

A2-2 (1.6g, 7.37mmol) was added into a round bottomed flask containing 24mL ultra dry tetrahydrofuran, and after cooling to -30 °C under nitrogen protection, NaHMDS (4.42ml, 8.84mmol, 2.0M tetrahydrofuran solution) was slowly

added dropwise thereto. After 30 minutes of reaction, diethyl chlorophosphate (2.54 g, 14.74 mmol) was slowly added dropwise to the mixture, and the mixture was stirred at -30 °C for 30 minutes, then stirred at room temperature for 30 minutes, and then cooled to -30 °C again. Methyl isocyanoacetate (1.46 g, 14.74 mmol) and NaHMDS (7.37 ml, 14.74 mmol, 2.0M tetrahydrofuran solution) were successively added to the mixture, and after the mixture was stirred for 2 hours with the temperature being slowly rised to room temperature, the reaction was comfirmed to be complete by TLC. The reaction mixture was quenched with saturated ammonium chloride solution, diluted with water, and extracted with ethyl acetate. The organic phase was washed with water and saturated sodium chloride solution successively, dried with anhydrous sodium sulfate, filtered, concentrated and purified by Combi-Flash flash silica gel chromatography to obtain 1.25g methyl 7-trifluoromethyl-4*H*-benzo[*b*]imidazo[1,5-*d*][1,4]oxazine-3-carboxylate (A2-3) as a yellow solid with a yield of 57.1%. LCMS: m/z= 299.0$(M+H)^+$, RT= 4.79min.

**Step 2: Synthesis of 7-trifluoromethyl-4*H*-benzo[*b*]imidazo[1,5-*d*][1,4]oxazine-3-formaldehyde (A2-4)**

A2-3 (1.15g, 3.87mmol) was dissolved in 35ml ultra dry dichloromethane, the solution was cooled to -78 °C under the protection of nitrogen, and DIBALH (10.32ml, 15.48mmol, 1.5M toluene solution) was slowly added dropwise thereto, and the mixture was stirred at -78 °C for 40 minutes. After that, methanol which was precooled to -78 °C was added dropwise to quench the reaction until no bubbles were produced. The reaction mixture was warmed to room temperature, diluted with dichloromethane, added with water and saturated potassium sodium tartrate aqueous solution, and stirred for 3 hours until stratification. The extracted organic layer was dried with anhydrous sodium sulfate, filtered and purified by Combi-Flash flash silica gel chromatography to obtain 503 mg 7-trifluoromethyl-4*H*-benzo[*b*]imidazo[1,5-*d*][1,4]oxazine-3-formaldehyde (A2-4) with a yield of 48.6%. LCMS: m/z= 269.0$(M+H)^+$, RT= 4.55min.

**Step 4: Synthesis of (S, E)-2-methyl-N-((7-trifluoromethyl-4*H*-benzo[*b*]imidazo[1,5-*d*][1,4]oxazin-3-yl)methylene)propane-2-sulfinamide (A2-5)**

A2-4 (480 mg, 1.79 mmol) and (S)-tert-butylsulfinamide (390 mg, 3.22 mmol) were dissolved in 3 ml tetrahydrofuran solution, tetraethyl titanate (1.47 g, 6.44 mmol) was added and the mixture was stirred overnight at room temperature. Saturated sodium chloride solution and ethyl acetate were added for extraction. The organic layer was dried with anhydrous sodium sulfate, filtered and purified by Combi-Flash flash silica gel chromatography to obtain 550mg (S, E)-2-methyl-N-((7-trifluoromethyl-4*H*-benzo[*b*]imidazo[1,5-*d*][1,4]oxazin-3-yl)methylene)pr opane-2-sulfinamide (A2-5) with a yield of 82.7%. LCMS: m/z= 372.0$(M+H)^+$, RT= 5.12 min.

**Step 5: Synthesis of (S)-2-methyl-N-((S)-1-(7-trifluoromethyl-4*H*-benzo[*b*]imidazo[1,5-*d*][1,4]oxazin-3-yl)ethyl)propane-2-sulfinamide (A2-6)**

Under the protection of nitrogen at -78 °C, A2-5 (550mg, 1.48mmol) was dissolved in 30ml ultra-dry tetrahydrofuran, methyl magnesium bromide (8.88ml, 8.88mmol, 1.0M ether solution) was slowly added dropwise, the mixture was stirred at -78 °C for 1 hour, and then saturated ammonium chloride solution was added for quenching. The mixture was extracted with dichloromethane and water. The organic layer was dried with anhydrous sodium sulfate, filtered and purified by Combi-Flash flash silica gel chromatography to obtain 453mg (S)-2-methyl-N-((S)-1-(7-trifluoromethyl-4*H*-benzo[*b*]imidazo[1,5-*d*][1,4]oxazin-3-yl)ethyl) propane-2-sulfinamide (A2-6) with a yield of 78.9%. LCMS: m/z= 388.0$(M+H)^+$, RT= 4.64 min.

**Step 2: Synthesis of (S)-1-(7-trifluoromethyl-4*H*-benzo[*b*]imidazo[1,5-*d*][1,4]oxazin-3-yl)ethylamine (A2)**

Under the protection of nitrogen at 0 °C, A2-6 (453mg, 1.17mmol) was dissolved in 4.5ml methanol, 0.45ml concentrated hydrochloric acid was dropwise added, and the mixture was stirred for 2 hours at room temperature. The mixture was diluted with water, part of methanol was removed by rotary evaporation, 1N sodium hydroxide solution was added to adjust pH to 11, and the mixture was extracted with ethyl acetate for 3 times. The organic layer was dried with anhydrous sodium sulfate, filtered and dried by rotary evaporation to obtain 287mg (S)-1-(7-trifluoromethyl-4*H*-benzo[*b*]imidazo[1,5-*d*] [1,4]oxazin-3-yl)ethylamine (A2) with a yield of 86.7%. Viscous liquid, was dissolved and packed, dried by rotary evaporation and weighed for later use. LCMS: m/z= 267.0$(M+H)^+$, RT= 3.71min.

**Synthesis of intermediate A4**

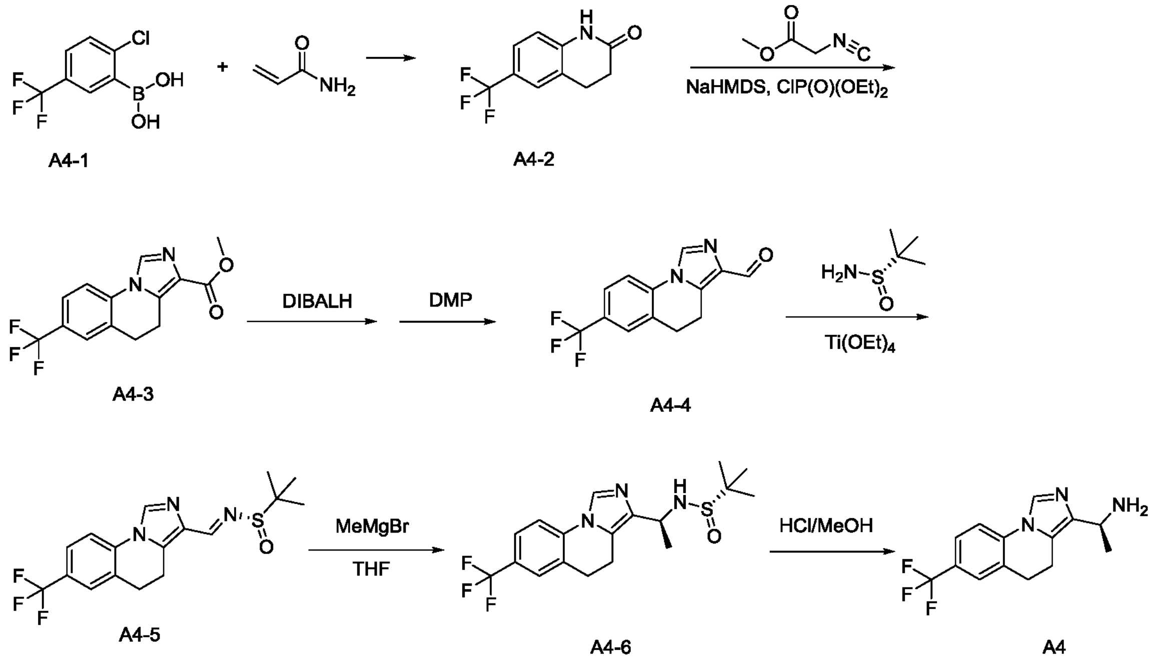


A4-1 A4-2 A4-3 A4-4 A4-5 A4-6 A4

2-chloro-5-trifluoromethyl phenylboronic acid (A4-1, 1.65 g, 7.35 mmol), acrylamide (0.52 g, 7.32 mmol), allylpalladium(II) chloride dimer (134 mg, 0.37 mmol), chloride(1,5-cyclooctadiene)rhodium(I) dimer (145mg, 0.29mmol), XPhos (350mg, 0.73mmol) and potassium phosphate (3.40g, 16.02mmol) were added to a mixed solvent of 20mL tert-amyl alcohol and 2mL methanol, and the mixture was stirred at 110 °C for 18 hours under the protection of nitrogen. After the reaction was completed, the reaction liquid was filtered by silica gel pad, the filter cake was washed with ethyl acetate, and the filtrate was concentrated and purified by Combi-Flash to obtain 1.2g 6-trifluoromethyl-3,4-dihydroquinolin-2(1*H*)-one (A4-2) as a light yellow solid with a yield of 73.8%. LCMS: m/z= 216.0(M+H)$^+$, RT= 4.46min.

A4-2 was used as raw material, the subsequent steps were the same as the synthesis steps for intermediate A2 (in step 3, if reduced to alcohol, the mixture could be treated by appropriate amount of Dess-Martin periodinane), and (S)-1-(7-trifluoromethyl-4,5-dihydroimidazo[1,5-*a*]quinolin-3-yl)ethylamine (A4) was obtained. LCMS: m/z=265.1 [M-NH]$^+$, RT=3.53 min.

**Synthesis of intermediate A5:**

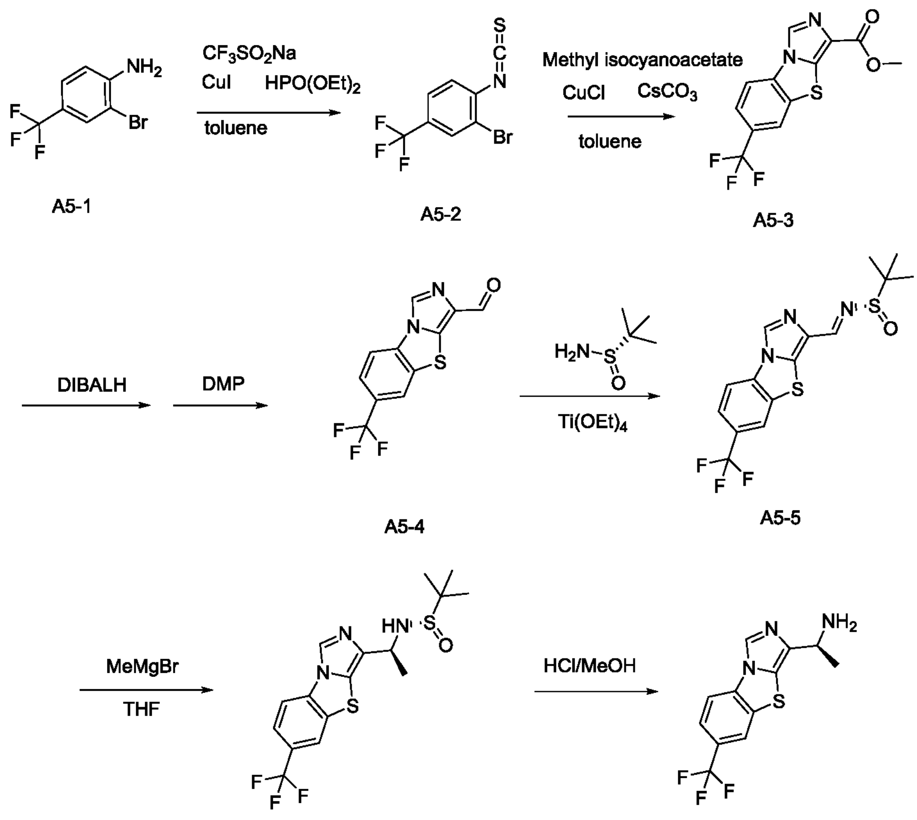


A5-1 A5-2 A5-3 A5-4 A5-5 A5-6 A5

**Step 1: Synthesis of 2-bromo-1-isothiohydro-4-trifluoromethylbenzene (A5-2)**

2-bromo-4-trifluoromethylaniline (8 g, 33.33 mmol), cuprous iodide (318 mg, 1.67 mmol) and sodium trifluoromethanesulfonate (10.4 g, 66.66 mmol) were dissolved in 100 ml toluene in a sealed tube, and diethyl phosphate (9.21 g, 66.66 mmol) was added after nitrogen bubbling, the reaction solution was stirred at 110 °C for 16 hours and then poured into water. The mixture was extracted with ethyl acetate, and the organic phase was washed with saturated sodium chloride solution, dried with anhydrous sodium sulfate, filtered, concentrated and separated by combi-flash flash silica gel chromatography to obtain 5.04g 2-bromo-1-isothiohydro-4-trifluoromethylbenzene (A5-2) as a light green solid with a yield of 53.6%.

**Step 2: Synthesis of methyl 6-trifluoromethylbenzo[*d*]imidazo[5,1-*b*]thiazole-3-carboxylate (A5-3)**

Under the protection of nitrogen, A5-2 (5.04g, 17.87mmol), cuprous chloride (177mg, 1.79mmol) and cesium carbonate (11.64g, 35.74mmol) were dissolved in toluene, methyl isocyanoacetate (3.54g, 35.74mmol) was added, and the mixture was stirred at 110 °C for 3 hours. After concentration, ethyl acetate and water were added for extraction, the organic layer was washed with saturated sodium chloride solution, dried with anhydrous sodium sulfate, filtered, concentrated and separated by combi-flash flash silica gel chromatography to obtain 2.33 g methyl 6-trifluoromethylbenzo[*d*]imidazo[5,1-*b*]thiazole-3-carboxylate (A5-3) as a yellow solid with a yield of 43.5%. LCMS: m/z= 301.0 $(M+H)^+$, RT=4.73min.

The subsequent steps were the same as those for intermediate A2 (in step 3, if reduced to alcohol, the mixture could be treated by appropriate amount of Dess -Martin periodinane), and (S)-1-(6-trifluoromethylbenzo[d]imidazo[1,5-b]thiazol-3-yl)ethylamine (A5) was obtained. LCMS: m/z=269.0 $[M-NH]^+$, RT=3.67 min.

**Synthesis of intermediate A6**

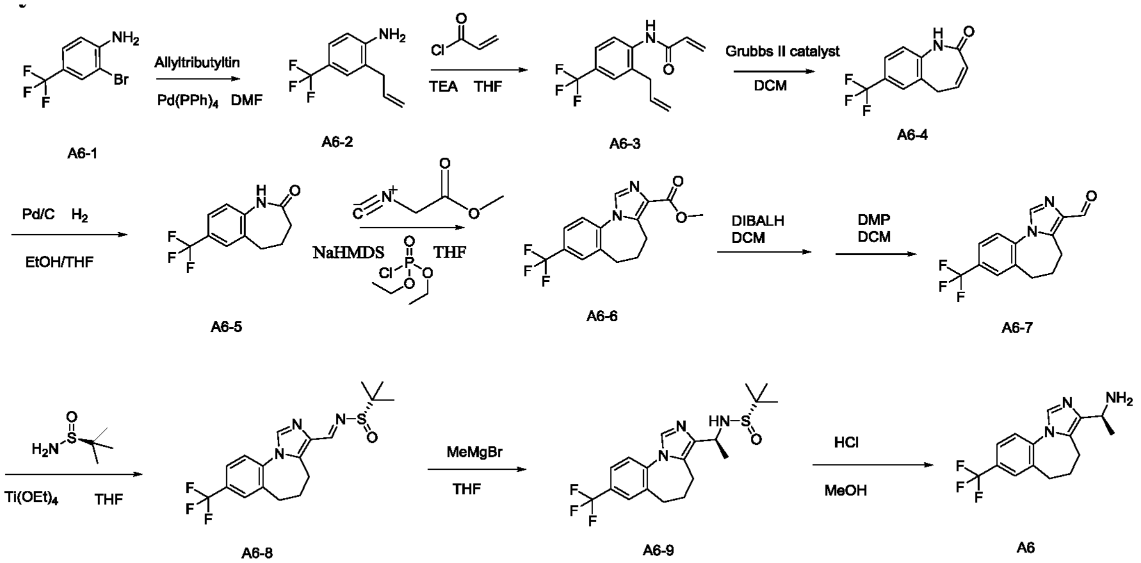


**Step 1: Synthesis of 2-allyl-4-trifluoromethylaniline (A6-2)**

Under the protection of nitrogen, 4-amino-3-bromotrifluoromethylbenzene (15g, 62.49mmol) and tetrakis(triphenylphosphine)palladium (2.16g, 1.87mmol) were dissolved in 100ml ultra dry DMF, AIBN (22.92g, 69.23mmol) was added, and the mixture was stirred at 80 °C for 19 hours. After cooling, ethyl acetate and water were added for extraction. The organic layer was washed with saturated sodium chloride solution, dried with anhydrous sodium sulfate, filtered, concentrated, and separated by combi-flash flash silica gel chromatography to obtain 8.147g 2-allyl-4-trifluoromethylaniline (A6-2) as a brown oily substance with a yield of 65%. LCMS: m/z= 202.1 $(M+H)^+$, RT=5.18min.

**Step 2: Synthesis of N-(2-allyl-4-trifluoromethylphenyl)acrylamide (A6-3)**

Under the protection of nitrogen, 2-allyl-4-trifluoromethylaniline (A6-2, 8g, 39.76mmol) was dissolved in 80ml ultra-dry tetrahydrofuran, triethylamine (6.84g, 67.59mmol) was added, and acryloyl chloride (4.32g, 47.71mmol) was dropwise added at 0 °C. The reaction solution was slowly raised to room temperature in 1 hour and continued to be stirred for 1 hour, and then the mixture was concentrated, and extracted with ethyl acetate and water. The organic layer was washed with saturated sodium chloride solution, dried with anhydrous sodium sulfate, filtered, concentrated and separated by combi-flash flash silica gel chromatography to obtain 5.9 g N- (2-allyl-4-trifluoromethylphenyl)acrylamide (A6-3) was as a white solid with a yield of 58.1%. LCMS: m/z= 256.1 $(M+H)^+$, RT=5.00min.

**Step 3: Synthesis of 7-trifluoromethyl-1,5-dihydro-2*H*-benzo[*b*]azepin-2-one (A6-4)**

N-(2-allyl-4-trifluoromethylphenyl)acrylamide (A6-3, 5.9g, 23.11mmol) was dissolved in 150ml ultra dry dichloromethane, Grubbs II catalyst (1.18g, 1.39mmol) was added, and the mixture was stirred for 18 hours at room temperature. The mixture was concentrated and separated by combi-flash flash silica gel chromatography to obtain 3.51 7-trifluoromethyl-1,5-dihydro-2*H*-benzo[*b*]azepin-2-one (A6-4) as a purple solid with a yield of 66.8%. LCMS: m/z=228.0 $(M+H)^+$, RT=4.15min.

**Step 4: Synthesis of 7-trifluoromethyl-1,3,4,5-tetrahydro-2*H*-benzo[*b*]azepin-2-one (A6-5)**

Under the protection of nitrogen, 7-trifluoromethyl-1,5-dihydro-2*H*-benzo[*b*]azepin-2-one (A6-4, 3.51g, 15.45mmol) was dissolved in 30ml ultra dry tetrahydrofuran and 30ml anhydrous ethanol, 10% palladium/carbon (512mg) was added, the atmosphere was replaced with hydrogen, and the mixture was stirred for 18 hours at room temperature. The mixture was filtered with diatomite, concentrated and purified by combi-flash flash silica gel chromatography to obtain 3.15g 7-trifluoromethyl-1,3,4,5-tetrahydro-2*H*-benzo[*b*]azepin-2-one (A6-5) as a white solid with a yield of 98.0%. LCMS: m/z= 230.1 $(M+H)^+$, RT=4.47min.

The subsequent steps were the same as those for intermediate A2 (in step 3, if reduced to alcohol, the mixture could be treated with appropriate amount of Dess-Martin periodinane), and(S)-1-(8-trifluoromethyl-5,6-dihydro-4*H*-benzo[*f*]imidazo[1,5-*a*]azepin-3-yl)ethylamine (A6) was obtained. LCMS: m/z=279.1 $[M-NH]^+$, RT=3.49 min.

**Synthesis of intermediate A9**

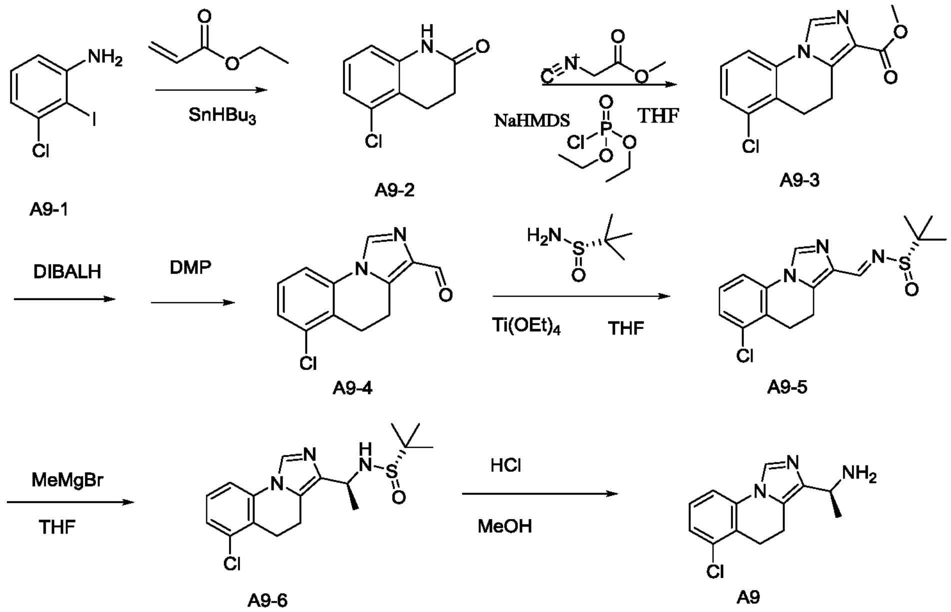


3-chloro-2-iodoaniline (A9-1, 5g, 19.73mmol), AIBN (1.29g, 7.89mmol), tri-n-butyltin hydride (8.6g, 29.60mmol) were dissolved in 30ml ultra dry DMSO, ethyl methyl acrylate (8.58ml, 31.88mmol) was added dropwise at 0 °C, and the mixture was stirred at 120 °C for 12h. After being cooled to room temperature, the mixture was quenched with ice water and extracted with ethyl acetate. The organic phase was washed with saturated sodium chloride solution, dried with anhydrous sodium sulfate, concentrated and purified by combi-flash silica gel chromatography to obtain 1.78g 5-chloro-3,4-dihydroquinolin-2(1*H*)-one (A9-2) as a brown white solid with a yield of 49.7%. LCMS: m/z= 182.1 $(M+H)^+$, RT=4.29min.

The subsequent steps were the same as those for intermediate A2 (in step 3, if reduced to alcohol, the mixture could be treated by appropriate amount of Dess -Martin periodinane), and (S)-1-(6-chloro-4,5-dihydroimidazo[1,5-*a*]quinolin-3-yl)ethylamine (A9) was obtained. LCMS: m/z=231.0 $[M-NH]^+$, RT=4.22 min.

**Synthesis of intermediates A13, A14**

(S)-N-((S)-1-(7-bromo-4*H*-benzo[*b*]imidazo[1,5-*d*][1,4]oxazin-3-yl)ethyl)-2-methylprop ane-2-sulfinamide (A13-6) was synthesized thought the synthesis method similar to that of intermediate A2 using 2-amino-5-bromophenol (A13-1) as raw material. LCMS: m/z=398.0$(M+H)^+$, RT=4.68 min.

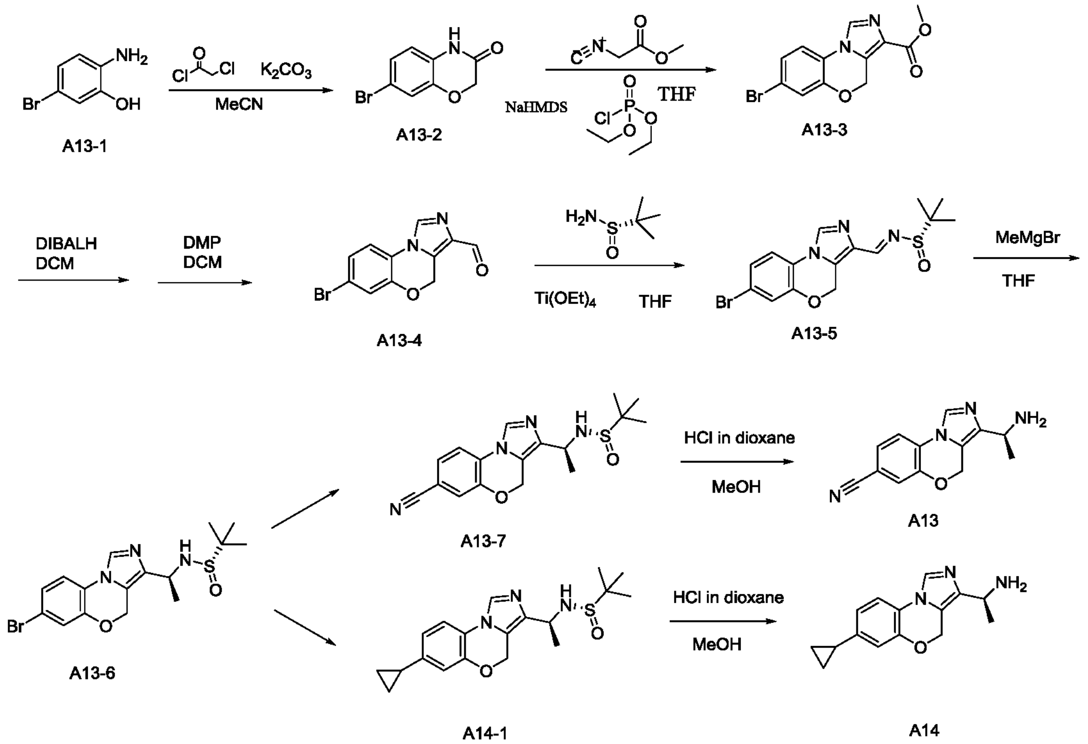


(S)-N-((S)-1-(7-bromo-4*H*-benzo[*b*]imidazo[1,5-*d*][1,4]oxazin-3-yl)ethyl)-2-methylprop ane-2-sulfinamide (A13-6, 200mg, 0.50mmol), tetrakis(triphenylphosphine)palladium (35mg, 0.03mmol) and zinc cyanide (35mg, 0.30mmol) were dissolved in 1ml ultra dry DMF, and the mixture was stirred at 80 °C for 6 hours. After being cooled, the mixture was extracted with 2N ammonia water and ethyl acetate. The organic layer was washed with saturated sodium chloride solution, dried with anhydrous sodium sulfate, filtered, concentrated, and then separated by combi-flash flash silica gel chromatography to obtain 166.7mg (S)-N-((S)-1-(7-cyano-4*H*-benzo[*b*]imidazo[1,5-*d*][1,4]oxazin-3-yl)ethyl)-2-methylpropane-2 -sulfinamide as a light yellow liquid with a yield of 97.1%. LCMS: m/z= 345.1 $(M+H)^+$, RT=4.30min. The subsequent steps were the same as those for intermediate A2 (the same volume of hydrochloride dioxane solution was used to replace hydrochloric acid), and (S)-1-(7-cyano-4*H*-benzo[*e*]imidazo[1,5-*d*][1,4]oxazin-3-yl)ethylamine (A13) was obtained. LCMS: m/z=224.1 $[M-NH]^+$, RT=0.57 min.

(S)-N-((S)-1-(7-bromo-4*H*-benzo[*b*]imidazo[1,5-*d*][1,4]oxazin-3-yl)ethyl)-2-methylprop ane-2-sulfinamide (A13-6, 140mg, 0.35mmol), palladium acetate (11mg, 0.018mmol), n-butylbis(1-adamantyl)phosphine (10 mg, 0.028 mmol), potassium cyclopropyltrifluoroborate (78 mg, 0.53 mmol) and cesium carbonate (342 mg, 1.05 mmol) were dissolved in 5ml toluene: water (10: 1) solution, and the mixture was stirred at 100 °C for 7 hours. The mixture was extracted with water and ethyl acetate, dried with anhydrous sodium sulfate, filtered, concentrated, and then separated by combi-flash flash silica gel chromatography to obtain 86.9mg

(S)-N-((S)-1-(7-cyclopropyl-4*H*-benzo[*b*]imidazo[1,5-*d*][1,4]oxazin-3-yl)ethyl)-2-methylpro pane-2-sulfinamide as a yellow solid with a yield of 69.0%. LCMS: m/z= 360.1 $(M+H)^+$, RT=4.84min. The subsequent steps were the same as those for intermediate A2 (the same volume of hydrochloride dioxane solution was used to replace hydrochloric acid), and (S)-1-(7-cyclopropyl-4*H*-benzo[*e*]imidazo[1,5-*d*][1,4]oxazin-3-yl)ethylamine (A14) was obtained. LCMS: m/z=239.2 $[M-NH]^+$, RT=4.02 min.

**Synthesis of intermediate A18**

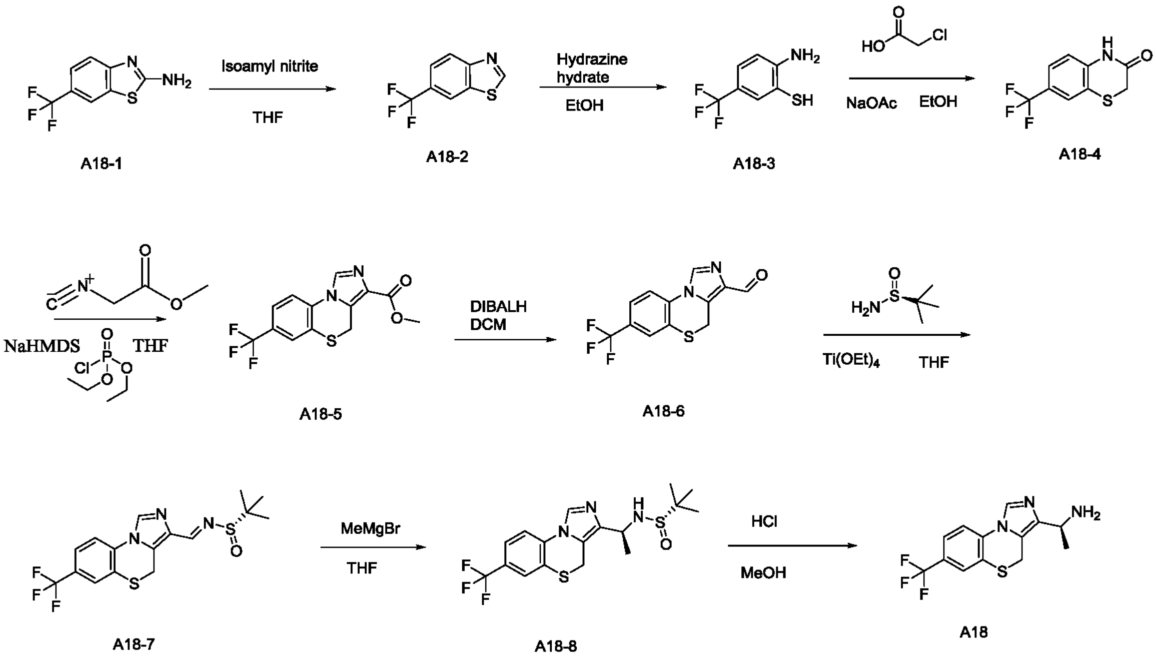


**Step 1: Synthesis of 6-trifluoromethylbenzo[*d*]thiazole (A18-2)**

6-trifluoromethylbenzo[*d*]thiazol-2-amine (A18-1, 6.8 g, 31.16 mmol) was dissolved in 60 ml THF under nitrogen protection, and isoamyl nitrite (8.03 g, 68.55 mmol) was added dropwise. The mixture was refluxed for 1.5 hours. The reaction liquid was poured into 60 ml ice water, and extracted with ethyl acetate. The organic phase was washed with anhydrous sodium sulfate, filtered, concentrated and purified by silica gel chromatography to obtain 6.0g 6-trifluoromethylbenzo[*d*]thiazole (A18-2) as a yellow oily liquid with a yield of 43.2%. LCMS: m/z=204.0 $(M+H)^+$, RT=4.88 min.

**Step 2: 2-amino-5-trifluoromethylthiophenol (A18-3)**

Under the protection of nitrogen, A18-2 (6g, 29.53mmol) was dissolved in 72ml ethanol, 85% hydrazine hydrate (13.2g, 223.25mmol) was added, and the mixture was refluxed for 3 hours. After cooled slightly, the mixture was cooled down to 0~10 °C in ice water bath, added with 60ml water, added slowly with 50% acetic acid aqueous solution to adjust pH to 6-7, and extracted with DCM. The organic phase was washed with saturated sodium chloride solution, dried with anhydrous sodium sulfate, and concentrated to obtain 5.6g yellow oily liquid, which was directly used for the next step. Yield: 98.2%. LCMS: m/z= 194.0 $(M+H)^+$, RT= 4.89min.

**Step 3: Synthesis of 7-trifluoromethyl-2*H*-benzo[*b*][1,4]thiazin-3(4*H*)-one (A18-4)**

A18-3 (5.0g, 25.88mmol) and sodium acetate (3.2g, 23.52mmol) were added to 50mL ethanol, chloroacetic acid (2.9g, 30.69mmol) was added dropwise under the protection of nitrogen, and after dropping finished, the mixture was stirred under reflux for 2.5h. After reaction finished, the reaction liquid was poured into 100ml ice water, stirred for 30 minutes, and then filtered. The filter cake was purified by silica gel chromatography to obtain 3.7g 7-trifluoromethyl-2*H*-benzo[*b*][1,4]thiazin-3(4*H*)-one (A18-4) with a yield of 61.4%. LCMS: m/z=234.1 $(M+H)^+$, RT=5.78 min.

A18-4 was used as raw material, the subsequent steps were the same as those for intermediate A2, and (S)-1-(7-trifluoromethyl-4*H*-benzo[*b*]imidazo[1,5-*d*][1,4]thiazin-3-yl)ethylamine (A18) was obtained. LCMS: m/z=283.0 $[M-NH]^+$, RT=3.54 min.

The intermediates were synthesized from corresponding or similar commercial reagents using the above method.

| Intermediate number | Name | Structural formula | Synthesizing route | Analysis data |
|---|---|---|---|---|
| Intermediate $A_1$ | (S)-1-(7-chloro-4*H*-benzo[e]imidazo[1,5-*d*][1,4]oxazin-3-yl)ethylamine | 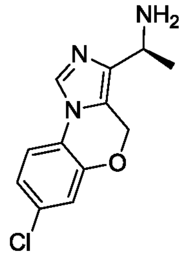 | Refer to the synthesis of intermediate A2 | LCMS: m/z=233.0 $[M-NH]^+$, RT=0.67 min |
| Intermediate A3 | (S)-1-(7-chloro-4,5-dihydroimidazo[1,5-*a*]quinolin-3-yl)ethylamine | 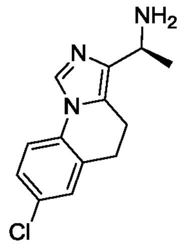 | Refer to the synthesis of intermediate A4 | LCMS: m/z=231.0 $[M-NH]^+$, RT=0.69 min |
| Intermediate A7 | (1S)-1-(5-methyl-7-trifluoromethyl-4,5-dihydroimidazo[1,5-*a*]quinolin-3-yl)ethylamine | 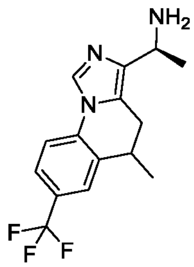 | Refer to the synthesis of intermediate A4 | LCMS: m/z=279.1 $[M-NH]^+$, RT=4.17 min |
| Intermediate A8 | (S)-1-(7-fluoro-4,5-dihydroimidazo[1,5-*a*]quinolin-3-yl)ethylamine | 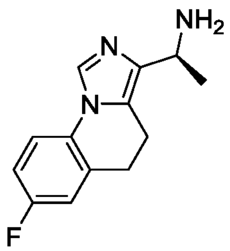 | Refer to the synthesis of intermediate A4 | LCMS: m/z=215.1 $[M-NH]^+$, RT=0.57 min |
| Intermediate A10 | (S)-1-(8-trifluoromethyl-4,5-dihydroimidazo[1,5-*a*]quinolin-3-yl)ethylamine | 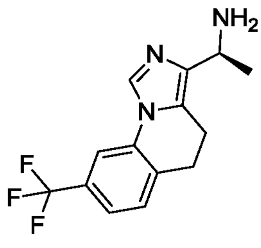 | Refer to the synthesis of intermediate A4 | LCMS: m/z=265.1 $[M-NH]^+$, RT=3.36 min |
| Intermediate A11 | (1S)-1-(4-methyl-7-trifluoromethyl-4,5-dihydroimidazo[1,5-*a*]quinolin-3-yl)ethylamine | 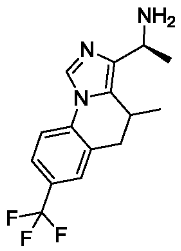 | Refer to the synthesis of intermediate A4 | LCMS: m/z=279.0 [M-NH]+, RT=3.95 min |

(continued)

| Intermediate number | Name | Structural formula | Synthesizing route | Analysis data |
|---|---|---|---|---|
| Intermediate A12 | (S)-1-(4-methyl-7-trifluoromethyl-4*H*-benzo[ *b*] imidazo[1,5-*d*][1,4]o xazin-3-yl) ethylamine | 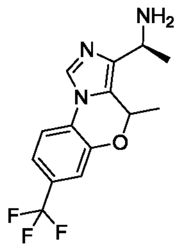 | Refer to the synthesis of intermediat e A2 | LCMS: m/z=281.0 $[M-NH]^+$, RT=4.37 min |
| Intermediate A15 | (S)-1-(7-methyl-4,5-dihydroimidazo[1,5-*a*]q uinolin-3-yl)ethylamin e | 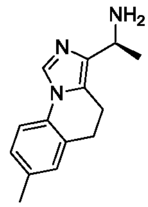 | Refer to the synthesis of intermediat e A4 | LCMS: m/z=211.2 $[M-NH]^+$, RT=0.62 min |
| Intermediate A16 | (S)-1-(7-fluoro-4,5-dihydroimidazo[1,5-*a*]qui nolin-3-yl) ethylamine | 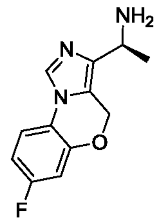 | Refer to the synthesis of intermediat e A2 | LCMS: m/z=217.1 $[M-NH]^+$, RT=3.33 min |
| Intermediate A17 | (S)-1-(7-methoxy-4*H*-benzo[e] imidazo[1,5-*d* ] [1,4]oxazin-3-yl) ethyl amine | 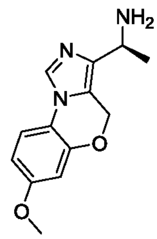 | Refer to the synthesis of intermediat e A2 | LCMS: m/z=229.1 $[M-NH]^+$, RT=0.65 min |

**Synthesis of intermediate B1**

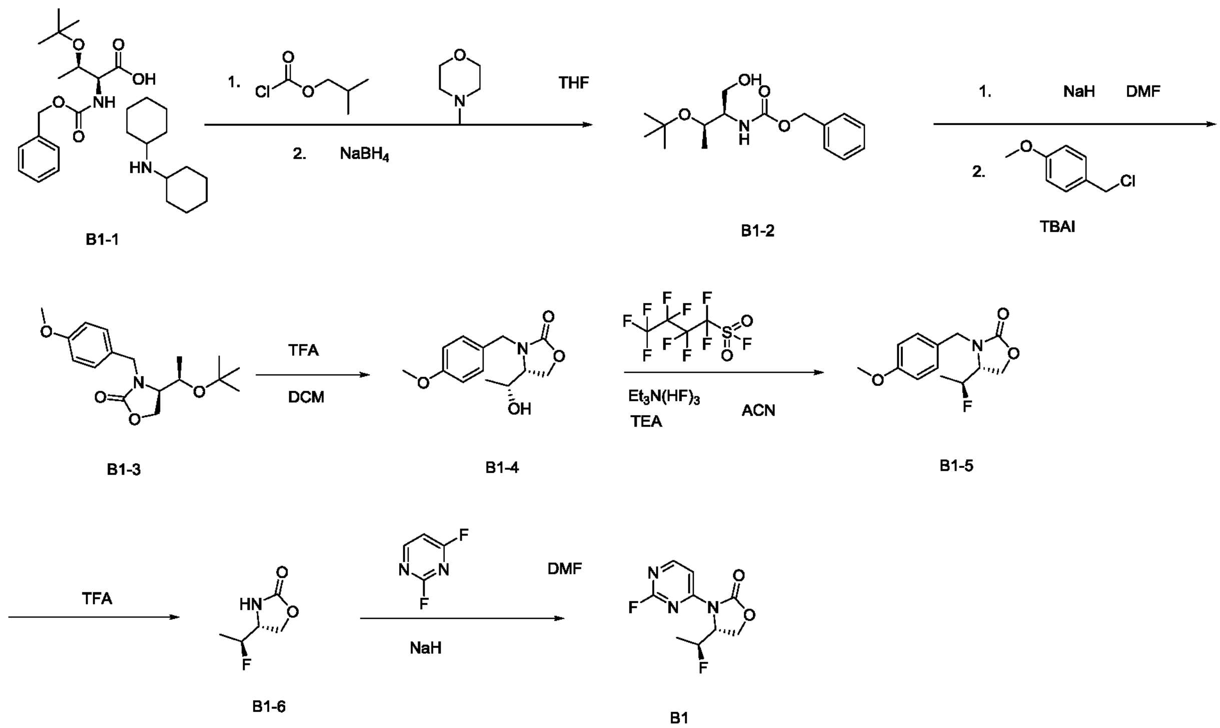


B1-1 B1-2 B1-3 B1-4 B1-5 B1-6 B1

**Step 1: Synthesis of benzyl ((2R,3R)-3-(tert-butoxy)-1-hydroxybutan-2-yl)carbamate (B1-2)**

N-benzyloxycarbonyl-O-tert-butyl-L-threonine dicyclohexylamine salt (B1-1) (10.0g, 20.38mmol) was added to 200mL tetrahydrofuran, and the mixture was cooled to -25 °C and stirred under nitrogen protection. Isobutyl chloroformate (3.3g, 24.16mmol) and N-methylmorpholine (2.5g, 24.71mmol, 1.2eq) were added and the mixture stirred at -25 °C for 15 minutes. After filtration, the filtrate was cooled to - 20 °C, sodium borohydride (1.5g, 39.65mmol) was added, and 40mL water was slowly added dropwise within 0.5-1 hour. After dropping, the reaction solution was raised to room temperature and stirred for 30 minutes. The reaction solution was poured into 200mL ice water, and extracted with ethyl acetate (200mL×2). The organic phases were combined, washed once with 200mL water and washed once with 200ml saturated salt water. The organic phase was dried with anhydrous sodium sulfate and concentrated to obtain benzyl ((2R,3R)-3-(tert-butoxy)-1-hydroxybutan-2-yl)carbamate (B1-2), which was directly used in the next step. LCMS: m/z= 318.2(M+Na)$^+$, RT= 3.55min.

**Step 2: Synthesis of (R)-4-((R)-1-(tert-butoxy)ethyl)-3-(4-methoxybenzyl)oxazolidin-2-one(B1-3)**

B1-2 (6.0 g, 20.31 mmol) was dissolved in 100 mL DMF and the solution was cooled to 0 °C under the protection of nitrogen. Sodium hydride (60%, 1.6g, 40.00mmol) was added in batches and the mixture was stirred for 30 minutes. P-methoxybenzyl chloride (4.8g, 30.65mmol) and tetrabutylammonium iodide (0.8g, 2.16mmol) were added, and the reaction liquid was raised to room temperature and stirred overnight (16 hours). After the reaction finished, the reaction liquid was quenched in 200mL ice water, and extracted with ethyl acetate (100mL×3). The organic phases were combined, washed twice with 100mL water and washed once with 100mL saturated brine. The organic phase was dried with anhydrous sodium sulfate, concentrated, and purified by Combi-Flash flash silica gel chromatography to obtain 5.9g (R)-4-((R)-1-(tert-butoxy)ethyl)-3-(4-methoxybenzyl)oxazolidin-2-one (B1-3) as a light yellow oily substance with a yield of 94.5%. LCMS: m/z= 308.1(M+H)$^+$, RT= 5.19min.

**Step 2: Synthesis of (R)-4-((R)-1-hydroxyethyl)-3-(4-methoxybenzyl)oxazolidin-2-one (B1-4)**

B1-3 (5.9g, 19.19mmol) was dissolved in 40mL dichloromethane, 40mL trifluoroacetic acid was added, and the mixture was stirred for 30 minutes at room temperature under the protection of nitrogen. The reaction liquid was concentrated, and the concentrate was added with 50mL dichloromethane and concentrated again which was repeated three times to remove trifluoroacetic acid. The residue was purified by Combi-Flash flash silica gel chromatography to obtain 3.4g

(R)-4-((R)-1-hydroxyethyl)-3-(4-methoxybenzyl)oxazolidin-2-one (B1-4) as a white solid with a yield of 70.7%. LCMS:

m/z= 252.1(M+H)$^+$, RT= 3.53min.

**Step 4: Synthesis of (R)-4-((S)-1-fluoroethyl)-3-(4-methoxybenzyl)oxazolidin-2-one (B1-5)**

B1-4 (3.4g, 13.53mmol) was dissolved in 45mL acetonitrile and the solution was cooled to 0 °C under the protection of nitrogen. Nonafluorobutanesulfonyl fluoride (7.4mL, 40.59mmol), triethylamine (17.0mL, 121.77mmol) and triethylamine trihydrofluorate (6.8mL, 40.59mmol) were added, and the mixture continued to be stirred at 0 °C for 1 hour. The reaction solution was quenched in 90 mL ice water, and extracted with ethyl acetate (90 mL×3). The organic phases were combined, washed once with 150 mL water and washed once with 150 mL saturated brine. The organic phase was dried with anhydrous sodium sulfate, concentrated, and then purified by Combi-Flash flash silica gel chromatography to obtain 2.8g (R)-4-((S)-1-fluoroethyl)-3-(4-methoxybenzyl)oxazolidin-2-one (B1-5) as an oily substance with a yield of 81.4%. LCMS: m/z=254.1(M+H)$^+$, RT=4.25 min.

**Step 5: Synthesis of (R)-4-((S)-1-fluoroethyl)oxazolidin-2-one (B1-6)**

B1-5 (2.8g, 11.05mmol) was added to 56mL trifluoroacetic acid, and the mixture was heated to 65 °C under nitrogen protection and stirred overnight (16 hours). After the reaction finished, the reaction liquid was concentrated, and the residue was purified by silica gel chromatography to obtain 1.4g (R)-4-((S)-1-fluoroethyl)oxazolidin-2-one (B1-6) as a light blue oily substance with a yield of 95.1%. LCMS: m/z=134.1(M+H)$^+$, RT= 0.95min.

**Step 6: Synthesis of (R)-4-((S)-1-fluoroethyl)-3-(2-fluoropyrimidin-4-yl)-oxazolidin-2-one (B1)**

B1-6 (850mg, 6.38mmol) and 2,4-difluoropyrimidine (740mg, 6.37mmol) were added to 10mL DMF, and the mixture was cooled to 0 °C under nitrogen protection. Sodium hydride (310mg, 7.75mmol) was added, and after stirring for 30 minutes, the reaction liquid was raised to room temperature and continued to be stirred for 2 hours. After the reaction finished, the reaction liquid was quenched in 30mL ice water, and extracted with ethyl acetate (30 mL×3). The organic phases were combined, washed once with 60mL water and washed once with 60mL saturated brine. The residue was purified by Combi-Flash flash silica gel chromatography to obtain 1.1g (R)-4-((S)-1-fluoroethyl)-3-(2-fluoropyrimidin-4-yl)-oxazolidin-2-one (B1) as a white crystalline solid with a yield of 75.2%. LCMS: m/z=230.1(M+H)$^+$, RT=3.90 min.

**Synthesis of intermediate B5**

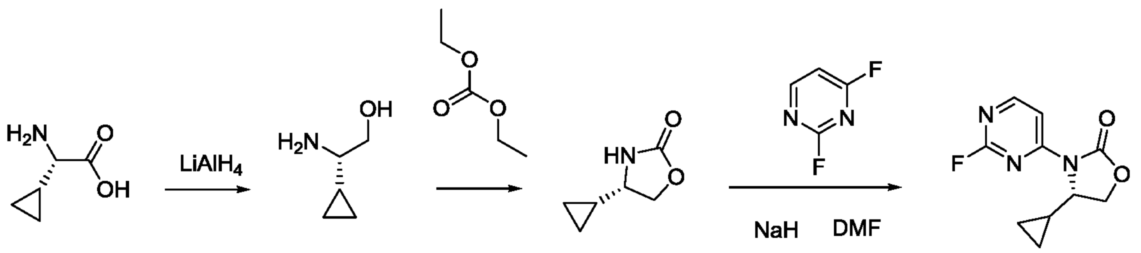


B5-1 B5-2 B5-3 B5

**Step 1: Synthesis of (S)-2-amino-2-cyclopropylethanol (B5-2)**

Under the protection of nitrogen, L-cyclopropylglycine (1g, 8.69mmol) was dissolved in 10ml ultra dry tetrahydrofuran, and the solution was cooled to 0 °C in ice bath, slowly added dropwise with 1.0M lithium aluminum hydride tetrahydrofuran solution (17.55ml, 17.55mmol), and then slowly raised to room temperature and stirred for 16 hours. Water was slowly added dropwise to quech the reaction. The mixture was filtered with diatomite and washed with tetrahydrofuran, and the filtrate was dried by rotary evaporation to obtain 546mg crude product of (S)-2-amino-2-cyclopropylethanol (B5-2) as a yellow liquid. LCMS: m/z=102.2 (M+H)$^+$, RT=0.55min.

**Step 2: Synthesis of (S)-4-cyclopropyloxazolidin-2-one (B5-3)**

Potassium carbonate (75mg, 0.54mmol) and diethyl carbonate (1.595g, 13.5mmol) were added to the product of step 1 (B5-2), and the spinous fractionation column and distillation unit were set up. The mixture was stirred and

slowly heated to 130 °C for 5 h. After cooling to room temperature, the residue was dissolved with dichloromethane and filtered, and the filtrate was extracted and washed with dichloromethane and saturated sodium bicarbonate solution. The organic layer was dried with anhydrous sodium sulfate, filtered and concentrated to obtain 673mg (S)-4-cyclopropyloxazolidin-2-one, which was directly used in the next step. LCMS: m/z= 128.1(M+H)+ , RT=1.65min.

The subsequent step was the same as the synthesis step 6 for intermediate B1, and (S)-4-cyclopropyl-3-(2-fluoropyrimidin-4-yl)oxazolidin-2-one (B5) was obtained. LCMS: m/z= 224.1[M+H]+, RT= 4.51min.

**Synthesis of intermediate B7**

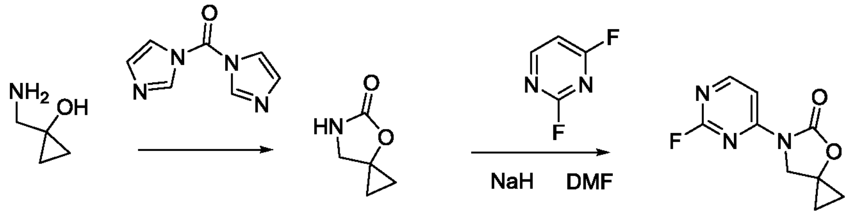


B7-1 B7-2 B7

1-aminomethyl-1-cyclopropanol (B7-1, 5g, 57.39mmol) and carbonyldiimidazole (9.31g, 57.39mmol) were dissolved in 170ml ultra-dry tetrahydrofuran. The mixture was stirred overnight at room temperature, and then dried by rotary evaporation to obtain 7.24g crude product of 4-oxa-6-azaspiro[2.4]heptan-5-one (B7-2) as a yellow oily liquid, which was directly used in the next step. LCMS: m/z=114.2 (M+H)+, RT= 1.15min.

The subsequent step was the same as the synthesis step 6 for intermediate B1, and 6-(2-fluoropyrimidin-4-yl)-4-oxa-6-azaspiro[2.4]heptan-5-one (B7) was obtained. LCMS: m/z= 210.1[M+H]+, RT= 4.40min.

**Synthesis of intermediate B8**

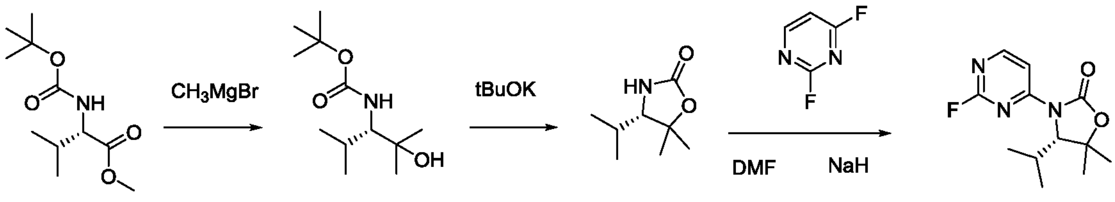


B8-1 B8-2 B8-3 B8

Under the protection of nitrogen, N-(tert-butoxycarbonyl)-L-valine methyl ester (B8-1, 5g, 21.62mmol) was dissolved in 50ml ultra dry tetrahydrofuran, magnesium methyl bromide (25.2ml, 75.67mmol, 3.0M ether solution) was added dropwise at 0 °C, and after 10 minutes, the mixture was stirred for 48 hours at room temperature. Saturated ammonium chloride solution was added dropwise at 0 °C to quench and layers were separated. The aqueous phase was extracted with EA, dried with anhydrous sodium sulfate and dried by rotary evaporation. The residue was added with potassium tert butyl alcohol (222mg, 1.99mmol) and dissolved in 13 mL ultra dry tetrahydrofuran, and the mixture was stirred for 3 hours at room temperature. The mixture was added with saturated ammonium chloride solution and extracted with ethyl acetate. The organic layer was washed with saturated sodium chloride, drying with anhydrous sodium sulfate, and separated by Combiflash flash silica gel chromatography to obtain 148.7mg (S)-4-isopropyl-5,5-dimethyl-oxazolidin-2-one (B8-3) as a white solid. LCMS: m/z= 158.1 (M+H)+, RT=3.55 min.

The subsequent step was the same as the synthesis step 6 for intermediate B1. Finally, (S)-3-(2-fluoropyrimidin-4-yl)-4-isopropyl-5,5-dimethyl-oxazolidin-2-one (B8) was obtained. LCMS: m/z= 254.1[M+H]+, RT= 4.85min.

**Synthesis of intermediate B9**

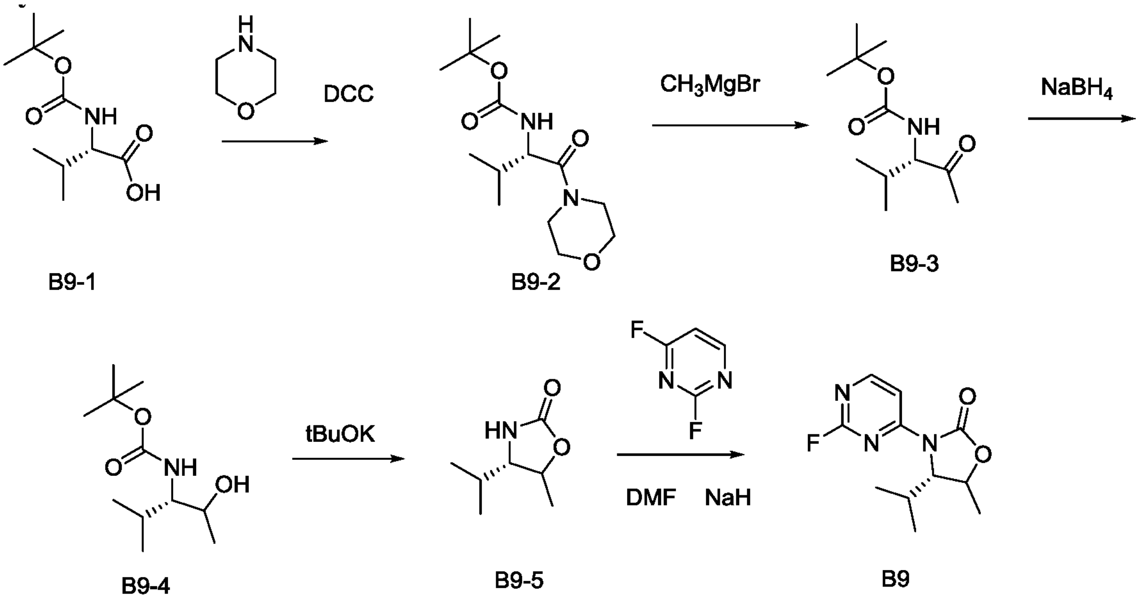


B9-1 B9-2 B9-3

B9-4 B9-5 B9

**Step 1: Synthesis of tert-butyl (S)-(3-methyl-1-morpholino-1-oxybutan-2-yl)carbamate (B9-2)**

(S)-Boc valine (5g, 23.01mmol) was dissolved in 100ml ultra dry dichloromethane, DCC (6.17g, 29.91mmol) and HOBT (3.42g, 25.31mmol) were added, and then after stirring for 10min, the mixture was added dropwise with morpholine (3g, 34.52mmol) and stirred at room temperature for 17h. The mixture was filtered, and the filtrate was washed with 1M dilute hydrochloric acid, washed with saturated sodium bicarbonate, dried with anhydrous sodium sulfate, filtered, and purified by combi-flash flash silica gel chromatography to obtain 5.43g tert-butyl (S)-(3-methyl-1-morpholino-1-oxybutan-2-yl)carbamate (B9-2) as a transparent viscous liquid.

**Step 2: Synthesis of tert-butyl (S)-(2-methyl-4-oxo-3-amyl)carbamate (B9-3)**

Under the protection of nitrogen, B9-2 (5.43g, 18.96mmol) was dissolved in 90ml ultra-dry tetrahydrofuran, methyl magnesium bromide (47.4ml, 47.40mmol, 1.0M tetrahydrofuran solution) was dropwise added at 0 °C, and the mixture was stirred at 0 °C for 1 hour and at room temperature for 1 hour. 1M dilute hydrochloric acid was added to quench at 0 °C, and the mixture was extracted with ether and purified by Combiflash flash silica gel chromatography to 728mg tert butyl (S)-(2-methyl-4-oxo-3-amyl)carbamate (B9-3) as a light yellow liquid. LCMS: m/z= 116.2(M+H)$^+$, RT=4.72min.

**Step 3: Synthesis of tert-butyl ((3S)-2-hydroxy-4-methylpentan-3-yl)carbamate (B9-4)**

At 0 °C, B9-3 (728mg, 3.38mmol) was dissolved in 7ml anhydrous methanol, sodium borohydride (256mg, 6.76mmol) was added in batches, and the mixture was stirred for 1 hour at room temperature, quenched by 1M dilute hydrochloric acid and extracted by ethyl acetate and water. The organic layer was dried by anhydrous sodium sulfate, filtered and concentrated to obtain 765mg crude product of tert-butyl ((3S)-2-hydroxy-4-methylpentan-3-yl)carbamate (B9-4) as a white colloid, which was directly used in the next step. LCMS: m/z=118.2 (M+H-100)$^+$, RT=4.18min.

The subsequent steps were the same as those for intermediate B8, and (4S)-3-(2-fluoropyrimidin-4-yl)-4-isopropyl-5-methyl-oxazolidin-2-one (B9) was obtained, LCMS: m/z= 240.1[M+H]$^+$, RT= 4.88min.

**Synthesis of intermediate B10**

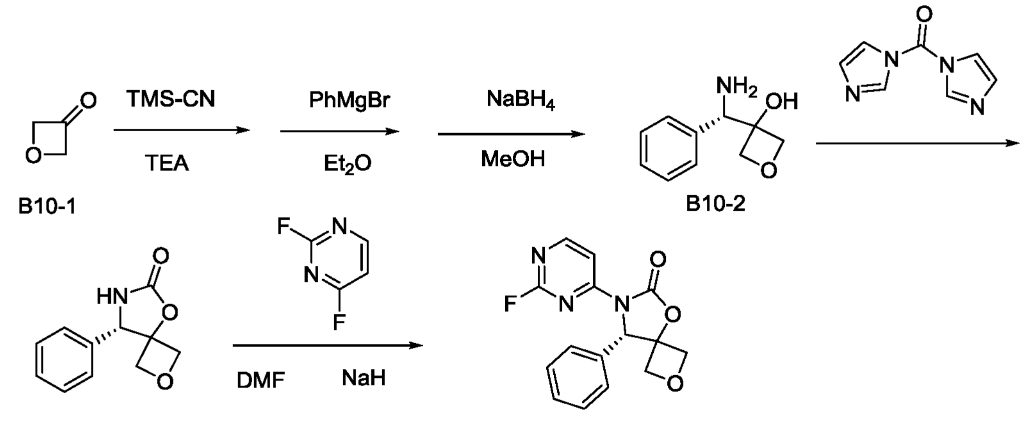


B10-1 B10-2 B10-3 B10

3-oxetanone (B10-1, 1g, 13.88mmol) and triethylamine (281mg, 2.78mmol) were added into an eggplant shaped flask, TMS-CN (1.65g, 16.66mmol) was slowly add (exothermic), and the mixture was stirred for 1 hour and then dried by rotary evaporation. The residue was dissolved in 10ml ether, added dropwise with in phenylmagnesium bromide (6.01ml, 18.04mmol, 3.0M in ether solution) and added with 5ml ether, and the mixture was stirred for 4 hours. 3 ml methanol was slowly added, sodium borohydride (630mg, 16.66 mmol) was slowly added, 12 ml methanol was slowly added (outgassing), the mixture was stirred overnight, 6ml water and 20ml 10% hydrochloric acid was carefully added dropwise, the mixture was stirred vigorously for 4 hours, and ether was add. The organic phase was extracted with 20ml 10% hydrochloric acid, and the aqueous phase was washed with with ether twice. The aqueous phase was added with 6N sodium hydroxide solution, and the milk white solution was extracted with DCM once, EA/THF (1:1) once, and EA twice, then washed with saturated sodium bicarbonate solution, respectively, dried with anhydrous sodium sulfate, and dried by rotary evaporatyion to obtain 1.99g crude product of (S)-3-(amino(phenyl)methyl)epoxypropan-3-ol (B 10-2) as a yellow liquid. LCMS: m/z=180.1 $(M+H)^+$, RT= 2.54min.

The subsequent steps were the same as those for B7, and (S)-7-(2-fluoropyrimidin-4-yl)-8-phenyl-2,5-dioxa-7-azaspiro[3.4]octan-6-one (B 10) was obtained. LCMS: m/z= 302.1$[M+H]^+$, RT= 4.47min.

The intermediates B2-B10 were synthesized from corresponding or similar raw materials using the above method.

| Intermed iate number | Name | Structural formula | Synthesizin g route | Analysis data |
|---|---|---|---|---|
| Intermed iate B2 | (S)-3-(2-chloropyrimid in-4-yl)-4-isopropyl-ox azolidin-2-one | 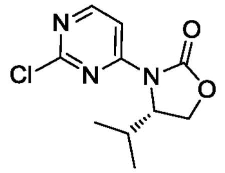 | Refer to the synthesis of intermediat e B1 | LCMS: m/z= 242.0 [M+H]+ RT= 4.72min |
| Intermed iate B3 | (R)-4-((S)-1-fluoroethy l)-3-(2-chloro-5-fluoro pyrimidin-4-yl)-oxazoli din-2-one | 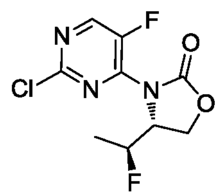 | Refer to the synthesis of intermediat e B1 | LCMS: m/z= 264.0 [M+H]+ RT= 4.28 min |
| Intermed iate B4 | (R)-4-((S)-1-fluoroethy l)-3-(2,4-dichloro-pyri midin-4-yl)-oxazolidin-2-one | 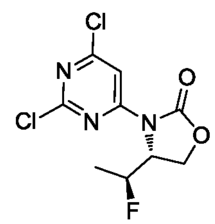 | Refer to the synthesis of intermediat e B1 | LCMS: m/z= 280.0 $[M+H]^+$ RT= 4.83min |

(continued)

| Intermed iate number | Name | Structural formula | Synthesizin g route | Analysis data |
|---|---|---|---|---|
| Intermed iate B6 | (S)-3-(2-fluoropyrimidi n-4-yl)-4-phenyl-oxazo lidin-2-one | 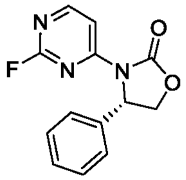 | Refer to the synthesis of intermediat e B5 | LCMS: m/z= 260.0 [M+H]+ RT= 4.66min |

**Synthesis of compound 1:**

(R)-3-(2-((S)-1-(7-chloro-4*H*-benzo[*b*]imidazo[1,5-*d*][1,4]oxazin-3-yl)ethylamino)pyri midin-4-yl)-4-((S)-1-fluoroethyl)oxazolidin-2-one

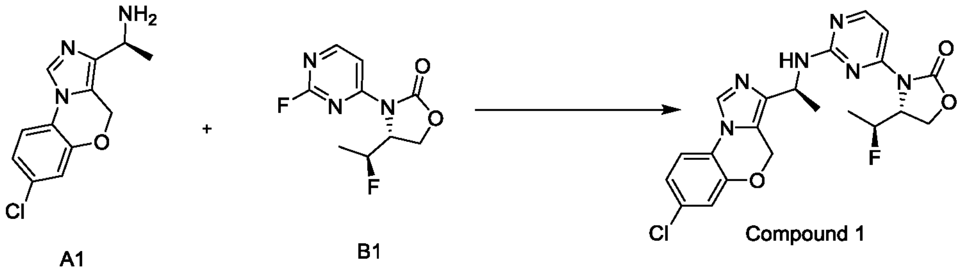

A1 B1 Compound 1

A1 (30mg, 0.12mmol) and B1 (27.5mg, 0.12mmol) were dissolved in 1.4ml DMSO, and DIPEA (46.5mg, 0.36mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-3-(2-(((S)-1-(7-chloro-4*H*-benzo[*b*]imidazo[1,5-*d*][1,4]oxazin-3-yl)ethylamino)pyrimidin -4-yl)-4-((S)-1-fluoroethyl)oxazolidin-2-one (Compound 1, white solid, 45.1 mg, 81.8%).LCMS: m/z= 459.0(M+H)$^+$, RT= 4.44min. $^1$H NMR (400 MHz, Chloroform-*d*) δ 8.19 (d, *J* = 5.8 Hz, 1H), 7.89 (s, 1H), 7.46 (d, *J* = 5.8 Hz, 1H), 7.33 (d, *J* = 8.5 Hz, 1H), 7.07 (d, *J* = 2.1 Hz, 1H), 7.01 (dd, *J* = 8.5, 2.2 Hz, 1H), 5.68 (br, 1H), 5.37 - 4.78 (m, 4H), 4.74 - 4.58 (m, 1H), 4.51 (d, *J* = 3.3 Hz, 1H), 4.36 (t, *J* = 9.0 Hz, 1H), 1.60 (d, *J* = 6.9 Hz, 3H), 1.24 (br, 3H).

**Synthesis of compound 2:**

(R)-4-((S)-1-fluoroethyl)-3-(2-(((S)-1-(7-trifluoromethyl-4*H*-benzo[*b*]imidazo[1,5-*d*][1, 4]oxazin-3-yl)ethyl)amino)pyrimidin-4-yl)oxazolidin-2-one

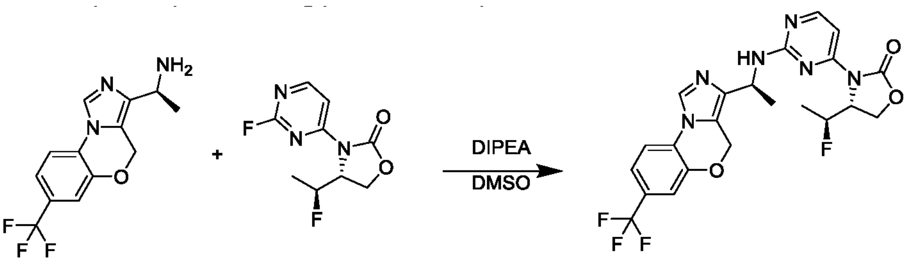


A2 B1 Compound 2

A2 (40mg, 0.14mmol) and B1 (32.1mg, 0.14mmol) were dissolved in 1.5ml DMSO, and DIPEA (54mg,

0.42mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-4-((S)-1-fluoroethyl)-3-(2-(((S)-1-(7-trifluoromethyl-4*H*-benzo[*b*]imidazo[1,5-*d*][1,4]oxa zin-3-yl)ethyl)amino)pyrimidin-4-yl)oxazolidin-2-one (Compound 2, white solid, 49.7mg, 71.5%). LCMS: m/z=493.0$(M+H)^+$, RT=4.77min. $^1H$ NMR (400 MHz, Chloroform-*d*)δ 8.11 (d, *J* = 5.8 Hz, 1H), 7.90 (s, 1H), 7.44 (d, *J* = 8.1 Hz, 1H), 7.38 (d, *J* = 5.8 Hz, 1H), 7.27 - 7.19 (m, 2H), 6.70 - 5.40 (br, 1H), 5.34 - 4.78 (m, 4H), 4.59 (dd, *J* = 26.7, 8.5 Hz, 1H), 4.42 (dd, *J* = 8.9, 3.3 Hz, 1H), 4.29 (t, *J* = 9.0 Hz, 1H), 1.55 (d, *J* = 6.9 Hz, 3H), 1.17 (d, *J* = 17.8 Hz, 3H).

**Synthesis of compound 3**

(S)-4-isopropyl-3-(2-(((S)-1-(7-trifluoromethyl-4,5-dihydroimidazo[1,5-*a*]quinolin-3-yl) ethyl)amino)pyrimidin-4-yl)oxazolidin-2-one

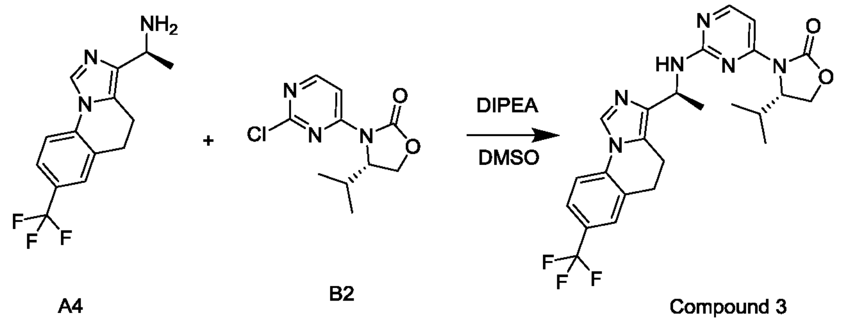


A4 B2 Compound 3

A4 (55.2mg, 0.19mmol) and B2 (46mg, 0.19mmol) were dissolved in 1.5ml DMSO, and DIPEA (74mg, 0.57mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (S)-4-isopropyl-3-(2-(((S)-1-(7-trifluoromethyl-4,5-dihydroimidazo[1,5-*a*]quinolin-3-yl)ethyl )amino)pyrimidin-4-yl)oxazolidin-2-one (Compound 3, white solid, 35.3mg, 37.0%). LCMS: m/z=487.0$(M+H)^+$, RT= 4.48min. δ 8.18 (d, *J* = 5.8 Hz, 1H), 7.99 (s, 1H), 7.59 - 7.46 (m, 3H), 7.43 (d, *J* = 5.8 Hz, 1H), 5.70 (br, 1H), 5.19 (br, 1H), 4.68 (br, 1H), 4.35 - 4.20 (m, 2H), 3.18 - 2.82 (m, 4H), 2.51 (br, 1H), 1.59 (d, *J* = 6.8 Hz, 3H), 0.90 (d, *J* = 6.9 Hz, 3H), 0.81 (d, *J* = 6.5 Hz, 3H).

**Synthesis of compound 4**

(R)-3-(2-(((S)-1-(7-chloro-4,5-dihydroimidazo[1,5-*a*]quinolin-3-yl)ethyl)amino)pyrimid in-4-yl)-4-((S)-1-fluoroethyl)oxazolidin-2-one

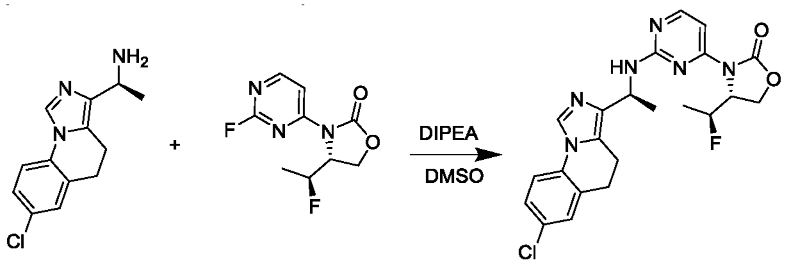


A3 B1 Compound 4

A3 (41.5mg, 0.16mmol) and B1 (37mg, 0.16mmol) were dissolved in 1.6ml DMSO, and DIPEA (62mg, 0.48mmol) was added. The mixture was stirred at 100 °C for 1.5 hours. The mixture was extracted with water and ethyl acetate, and the organic phase was dried with anhydrous sodium sulfate, filtered, concentrated, then separated by

Combi-Flash flash silica gel chromatography, and lyophilized to obtain (R)-3-(2-(((S)-1-(7-chloro-4,5-dihydroimidazo[1,5-*a*]quinolin-3-yl)ethyl)amino)pyrimidin-4-yl)-4-((S)-1-fluoroethyl)oxazolidin-2-one (Compound 4, white solid, 47.3mg, 61.8%). LCMS: m/z=457.0(M+H)$^+$ , RT= 4.22min. $^1$H NMR (400 MHz, Chloroform-*d*)δ 8.21 (d, *J* = 5.7 Hz, 1H), 7.93 (s, 1H), 7.46 (d, *J* = 5.7 Hz, 1H), 7.35 - 7.24 (m, 3H), 5.75 (br, 1H), 5.17 (br, 2H), 4.82 - 4.61 (m, 1H), 4.54 (dd, *J* = 8.8, 3.4 Hz, 1H), 4.39 (t, *J* = 8.9 Hz, 1H), 3.20 - 2.75 (m, 4H), 1.58 (d, *J* = 6.8 Hz, 3H), 1.33 (d, *J* = 19.1 Hz, 3H).

**Synthesis of compound 5**

(R)-4-((S)-1-fluoroethyl)-3-(2-(((S)-1-(7-trifluoromethyl-4,5-dihydroimidazo[1,5-*a*]quin olin-3-yl)ethyl)amino)pyrimidin-4-yl)oxazolidin-2-one

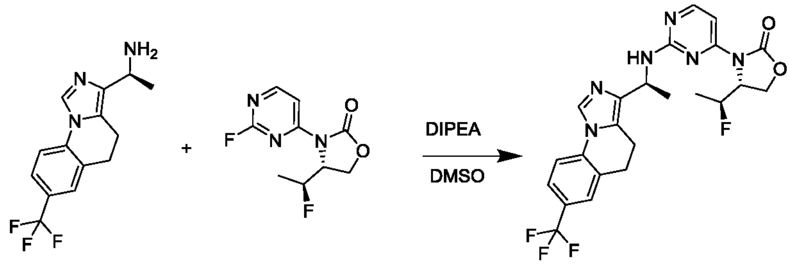


A4 B1 Compound 5

A4 (80mg, 0.28mmol) and B1 (64mg, 0.28mmol) were dissolved in 3 ml DMSO, and DIPEA (108mg, 0.84mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-4-((S)-1-fluoroethyl)-3-(2-(((S)-1-(7-trifluoromethyl-4,5-dihydroimidazo[1,5-*a*]quinolin-3-yl)ethyl)amino)pyrimidin-4-yl)oxazolidin-2-one (Compound 5, white solid, 26mg, 18.6%). LCMS: m/z= 491.0(M+H)$^+$, RT= 4.70min. $^1$H NMR (400 MHz, Chloroform-*d*)δ 8.19 (s, 1H), 7.98 (s, 1H), 7.60 - 7.39 (m, 4H), 5.72 (br, 1H), 5.16 (br, 2H), 4.76 - 4.61 (m, 1H), 4.52 (d, *J* = 8.4 Hz, 1H), 4.37 (t, *J* = 8.6 Hz, 1H), 3.15 - 2.80 (d, *J* = 63.1 Hz, 4H), 1.57 (d, *J* = 6.6 Hz, 3H), 1.32 (d, *J* = 21.3 Hz, 3H).

**Synthesis of compound 6**

(R)-3-(5-fluoro-2-(((S)-1-(7-trifluoromethyl-4,5-dihydroimidazo[1,5-*a*]quinolin-3-yl)eth yl)amino)pyrimidin-4-yl)-4-((S)-1-fluoroethyl)oxazolidin-2-one

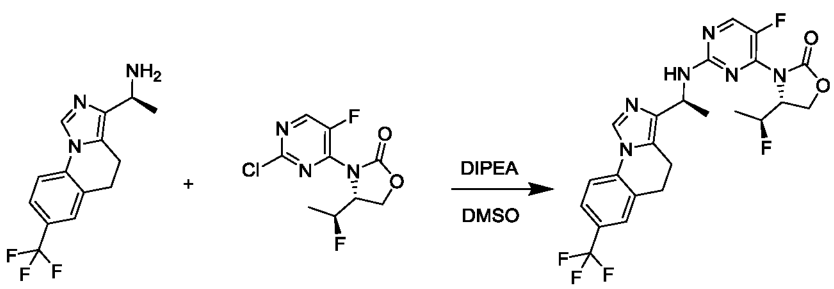


A4 B3 Compound 6

A4 (49.4mg, 0.17mmol) and B3 (44.8mg, 0.17mmol) were dissolved in 1.5ml DMSO, and DIPEA (66mg, 0.51mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain

(R)-3-(5-fluoro-2-(((S)-1-(7-trifluoromethyl-4,5-dihydroimidazo[1,5-*a*]quinolin-3-yl)ethyl)a mino)pyrimidin-4-yl)-4-((S)-1-fluoroethyl)oxazolidin-2-one (Compound 6, white solid, 20.5mg, 22.9%). LCMS: m/z=509.0 $(M+H)^+$, RT= 4.39min. $^1H$ NMR (400 MHz, Chloroform-*d*)δ 8.20 (d, *J* = 2.8 Hz, 1H), 7.98 (s, 1H), 7.58 - 7.46 (m, 3H), 5.86 (br, 1H), 5.15 - 4.87 (m, 2H), 4.75 - 4.61 (m, 1H), 4.50 (p, *J* = 8.6 Hz, 2H), 3.13 - 2.84 (m, 4H), 1.56 (d, *J* = 6.8 Hz, 3H), 1.27 (dd, *J* = 232, 6.3 Hz, 3H).

**Synthesis of compound 7**

(R)-3-(6-chloro-2-(((S)-1-(7-trifluoromethyl-4,5-dihydroimidazo[1,5-*a*]quinolin-3-yl)et hyl)amino)pyrimidin-4-yl)-4-((S)-1-fluoroethyl)oxazolidin-2-one

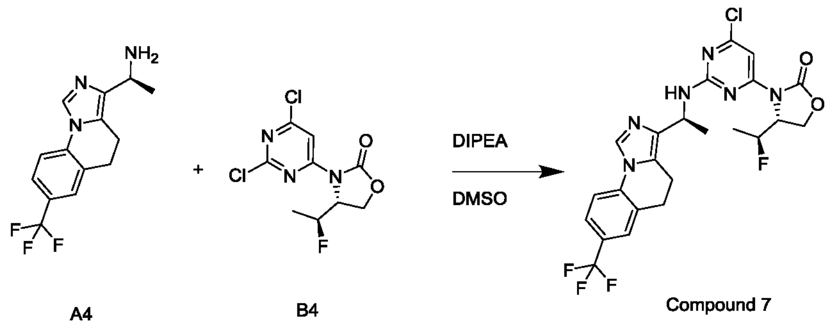


A4 B4 Compound 7

A4 (60.1mg, 0.21mmol) and B4 (70mg, 0.25mmol) were dissolved in 1.7ml DMSO, and DIPEA (81mg, 0.63mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-3-(6-chloro-2-(((S)-1-(7-trifluoromethyl-4,5-dihydroimidazo[1,5-*a*]quinolin-3-yl)ethyl)a mino)pyrimidin-4-yl)-4-((S)-1-fluoroethyl)oxazolidin-2-one (Compound 7, white solid, 15.7mg, 14.0%). LCMS: m/z= 525.0$(M+H)^+$, RT= 5.13min. $^1H$ NMR (400 MHz, Chloroform-*d*) δ 7.97 (s, 1H), 7.60 - 7.45 (m, 4H), 5.92 (d, *J* = 7.3 Hz, 1H), 5.40 - 4.87 (m, 2H), 4.81 - 4.28 (m, 3H), 3.39 - 2.77 (m, 4H), 1.59 (d, *J* = 6.5 Hz, 3H), 1.42 - 1.23 (m, 3H).

**Synthesis of compound 8**

(R)-4-((S)-1-fluoroethyl)-3-(2-(((S)-1-(6-trifluoromethylbenzo[*d*]imidazo[5,1-*b*]thiazol-3-yl)ethyl)amino)pyrimidin-4-yl)oxazolidin-2-one

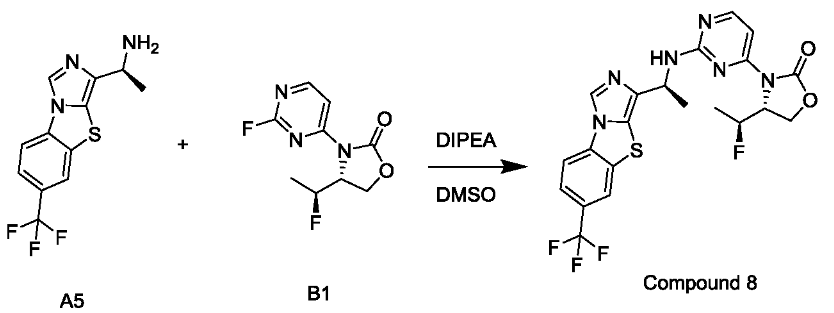


A5 B1 Compound 8

A5 (32.2mg, 0.11mmol) and B1 (25mg, 0.11mmol) were dissolved in 1.5ml DMSO, and DIPEA (43mg, 0.33mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-4-((S)-1-fluoroethyl)-3-(2-(((S)-1-(6-trifluoromethylbenzo[*d*]imidazo[5,1-*b*]thiazol-3-yl) ethyl)amino)pyrimidin-4-yl)oxazolidin-2-one (Compound 8, white solid, 36.5mg, 65.4%). LCMS: m/z= 495.1$(M+H)^+$ , RT= 4.78min. $^1H$ NMR (400 MHz, Chloroform-*d*) δ 8.29 (s, 1H), 8.24 (d, *J* = 5.8 Hz, 1H), 7.78 (s, 1H), 7.73 (d, *J* = 8.4 Hz, 1H), 7.63 (d, *J* = 8.4 Hz,

1H), 7.51 (d, *J* = 5.8 Hz, 1H), 6.21 (br, 1H), 5.17 (br, 1H), 4.65 (dd, *J* = 26.7, 7.4 Hz, 1H), 4.58 - 4.40 (m, 2H), 4.36 (t, *J* = 8.9 Hz, 1H), 1.67 (d, *J* = 6.9 Hz, 3H), 1.06 (br, 3H).

**Synthesis of compound 9**

(S)-4-isopropyl-3-(2-(((S)-1-(8-trifluoromethyl-5,6-dihydro-4*H*-benzo[*f*]imidazo[1,5-*a*] azepin-3-yl)ethyl)amino)pyrimidin-4-yl)oxazolidin-2-one

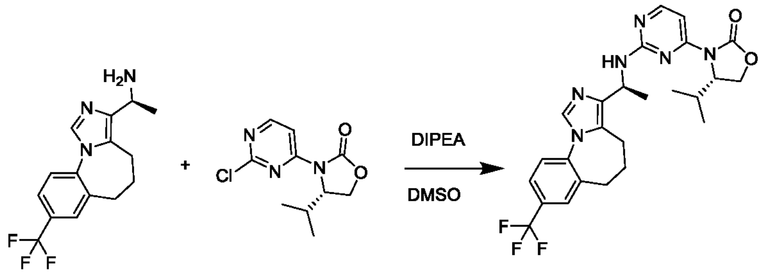


A6 B2 Compound 9

A6 (46.6mg, 0.16mmol) and B2 (46mg, 0.19mmol) were dissolved in 1.7ml DMSO, and DIPEA (62mg, 0.48mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (S)-4-isopropyl-3-(2-(((S)-1-(8-trifluoromethyl-5,6-dihydro-4*H*-benzo[*f*]imidazo[1,5-*a*] azepin-3-yl)ethyl)amino)pyrimidin-4-yl)oxazolidin-2-one (Compound 9, white solid, 58.1mg, 73.5%). LCMS: m/z= 501.2$(M+H)^+$, RT= 4.49min. $^1$H NMR (400 MHz, Chloroform-d) $\delta$ 8.20 (d, J = 5.8 Hz, 1H), 7.71 (s, 1H), 7.64 (d, J = 8.2 Hz, 1H), 7.60 (s, 1H), 7.45 (d, J = 5.8 Hz, 1H), 7.42 (d, J = 8.2 Hz, 1H), 5.79 (br, 1H), 5.24 (br, 1H), 4.72 (br, 1H), 4.35 - 4.25 (m, 2H), 2.75 - 2.50 (m, 5H), 2.12 (br, 2H), 1.62 (d, J = 6.7 Hz, 3H), 0.94 (d, J = 7.0 Hz, 3H), 0.85 (d, J = 6.9 Hz, 3H).

**Synthesis of compound 10**

(4R)-4-((S)-1-fluoroethyl)-3-(2-(((1S)-1-(5-methyl-7-trifluoromethyl-4,5-dihydroimidaz o[1,5-*a*]quinolin-3-yl)ethyl)amino)pyrimidin-4-yl)oxazolidin-2-one

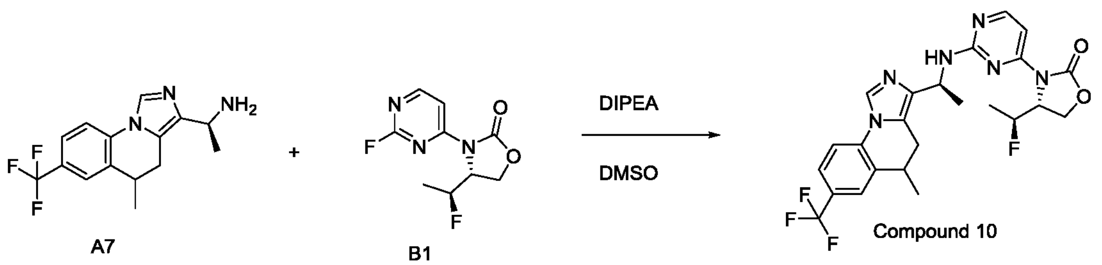


Compound 10

A7 B1

A7 (41.9mg, 0.14mmol) and B1 (34mg, 0.15mmol) were dissolved in 1ml ultra dry DMSO, and DIPEA (54mg, 0.42mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (4R)-4-((S)-1-fluoroethyl)-3-(2-(((1S)-1-(5-methyl-7-trifluoromethyl-4,5-dihydroimidazo[1,5 -*a*]quinolin-3-yl)ethyl)amino)pyrimidin-4-yl)oxazolidin-2-one (Compound 10, white solid, 46.1mg, 65.3%). LCMS: m/z=505.1$(M+H)^+$, RT=4.94 min. $^1$H NMR (400 MHz, Chloroform-*d*)$\delta$ 8.20 (dd, *J* = 5.7, 2.0 Hz, 1H), 7.99 (s, 1H), 7.55 (d, *J* = 5.6 Hz, 2H), 7.49 (d, *J* = 8.8 Hz, 1H), 7.45 (dd, *J* = 5.7, 4.0 Hz, 1H), 5.72 (br, 1H), 5.17 (br, 2H), 4.69 (dd, *J* = 26.7, 7.1 Hz, 1H), 4.59 - 4.44 (m, 1H), 4.37 (t, *J* = 8.9 Hz, 1H), 3.19 - 2.68 (m, 3H), 1.57 (dd, *J* = 6.8, 2.4 Hz, 3H), 1.45 - 1.10 (m, 6H).

**Synthesis of compound 11**

(R)-3-(2-(((S)-1-(7-fluoro-4,5-dihydropyrimidino[1,5-*a*]quinolin-3-yl)ethyl)amino)pyri midin-4-yl)-4-((S)-1-fluoroethyl)oxazolidin-2-one

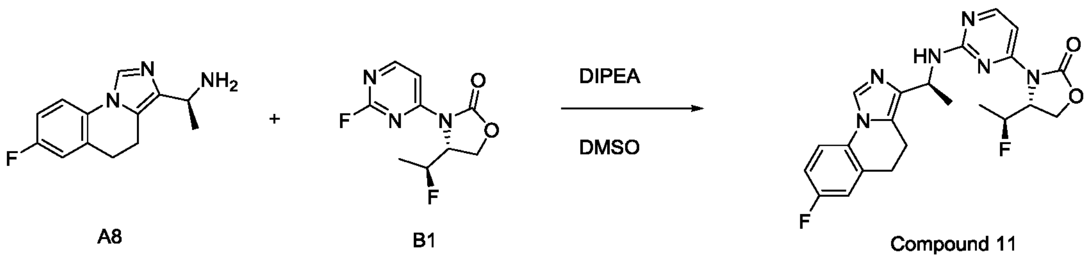


A8 B1 Compound 11

A8 (36.6mg, 0.16mmol) and B1 (41.2mg, 0.18mmol) were dissolved in 1.2ml DMSO, and DIPEA (62mg, 0.48mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-3-(2-(((S)-1-(7-fluoro-4,5-dihydropyrimidino[1,5-*a*]quinolin-3-yl)ethyl)amino)pyrimidin -4-yl)-4-((S)-1-fluoroethyl)oxazolidin-2-one (Compound 11, white solid, 46.6mg, 66.9%). LCMS: m/z=441.2(M+H)$^+$, RT= 3.96min. $^1$H NMR (400 MHz, Chloroform-*d*) δ 8.21 (d, *J* = 5.7 Hz, 1H), 7.91 (s, 1H), 7.46 (d, *J* = 5.7 Hz, 1H), 7.36 (dd, *J* = 8.5, 4.7 Hz, 1H), 7.08 - 6.96 (m, 2H), 5.66 (d, *J* = 7.7 Hz, 1H), 5.17 (br, 2H), 4.70 (dd, *J* = 26.2, 7.4 Hz, 1H), 4.53 (dd, *J* = 8.8, 3.4 Hz, 1H), 4.38 (t, *J* = 8.9 Hz, 1H), 2.95 (d, *J* = 70.2 Hz, 4H), 1.58 (d, *J* = 6.8 Hz, 3H), 1.33 (dd, *J* = 22.9, 5.6 Hz, 3H).

**Synthesis of compound 12**

(S)-4-isopropyl-3-(2-(((S)-1-(7-trifluoromethyl-4*H*-benzo[*b*]imidazo[1,5-*d*][1,4]thiazin-3-yl)ethyl)amino)pyrimidin-4-yl)oxazolidin-2-one

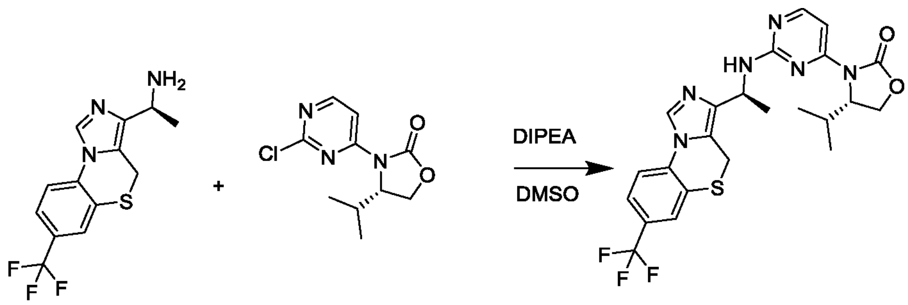


A18 B2 Compound 12

A18 (34.1mg, 0.11mmol) and B2 (29mg, 0.12mmol) were dissolved in 1.5ml DMSO, and DIPEA (43mg, 0.33mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (S)-4-isopropyl-3-(2-(((S)-1-(7-trifluoromethyl-4*H*-benzo[*b*]imidazo[1,5-*d*][1,4]thiazin-3-yl) ethyl)amino)pyrimidin-4-yl)oxazolidin-2-one (Compound 12, white solid, 14.2mg, 24.7%).
LCMS: m/z= 505.1(M+H)$^+$ , RT= 4.92min. $^1$H NMR (400 MHz, Chloroform-d) δ 8.17 (d, J = 5.6 Hz, 1H), 7.97 (s, 1H), 7.72 (s, 1H), 7.56 - 7.45 (m, 3H), 5.23 (br, 1H), 4.67 (br, 1H), 4.35 - 4.15 (m, 4H), 4.03 (d, J = 13.9 Hz, 1H), 2.48 (br, 1H), 1.65 (d, J = 6.7 Hz, 3H), 0.96 - 0.87 (m, 3H), 0.82 (d, J = 5.8 Hz, 3H).

**Synthesis of compound 13**

(R)-4-((S)-1-fluoroethyl)-3-(2-(((S)-1-(8-trifluoromethyl-5,6-dihydro-4*H*-benzo[*f*]imida zo[1,5-*a*]azepin-3-yl)ethyl)amino)pyrimidin-4-yl)oxazolidin-2-one

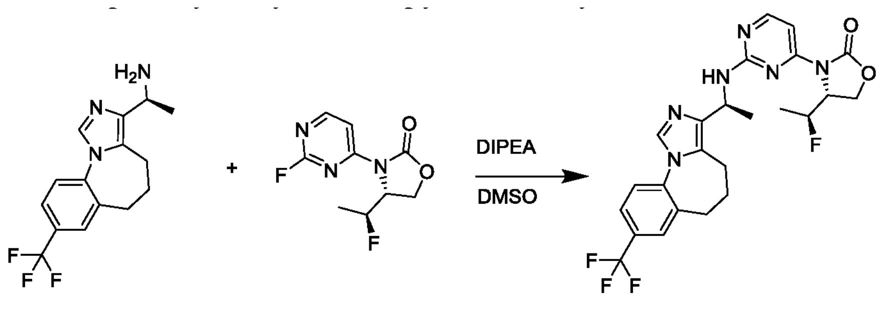


A6 B1 Compound 13

A6 (41.5mg, 0.14mmol) and B1 (34mg, 0.15mmol) were dissolved in 1.6ml DMSO, and DIPEA (54mg, 0.42mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-4-((S)-1-fluoroethyl)-3-(2-(((S)-1-(8-trifluoromethyl-5,6-dihydro-4*H*-benzo[*f*]imidazo[1, 5-*a*]azepin-3-yl)ethyl)amino)pyrimidin-4-yl)oxazolidin-2-one (Compound 13, white solid, 61.4mg, 86.6%). LCMS: m/z= 505.1(M+H)$^+$ , RT= 4.34min. $^1$H NMR (400 MHz, Chloroform-*d*)δ 8.19 (d, *J* = 5.7 Hz, 1H), 7.67 (s, 1H), 7.61 (d, *J* = 8.2 Hz, 1H), 7.57 (s, 1H), 7.45 (d, *J* = 5.7 Hz, 1H), 7.39 (d, *J* = 8.1 Hz, 1H), 5.77 (br, 1H), 5.19 (br, 2H), 4.80 - 4.60 (m, 1H), 4.52 (dd, *J* = 8.8, 3.4 Hz, 1H), 4.38 (t, *J* = 8.7 Hz, 1H), 2.78 - 2.49 (m, 4H), 2.25 - 1.80 (m, 2H), 1.58 (d, *J* = 6.7 Hz, 3H), 1.33 (dd, *J* = 22.9, 5.4 Hz, 3H).

**Synthesis of compound 14**

(4R)-4-((S)-1-fluoroethyl)-3-(2-(((1S)-1-(4-methyl-7-trifluoromethyl-4,5-dihydroimidaz o[1,5-*a*]quinolin-3-yl)ethyl)amino)pyrimidin-4-yl)oxazolidin-2-one

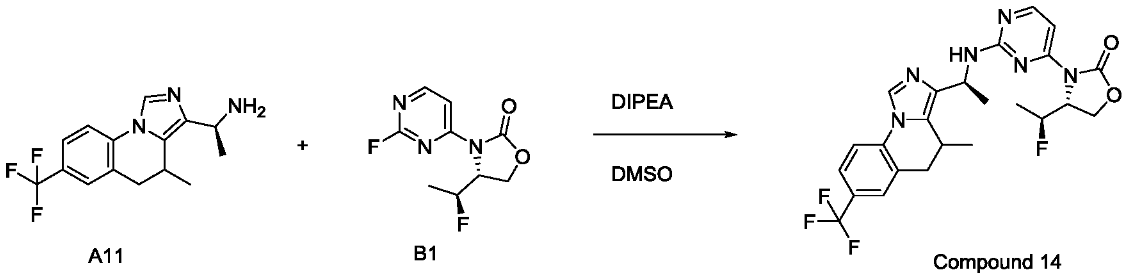


A11 B1 Compound 14

A11 (45.3mg, 0.15mmol) and B1 (39mg, 0.17mmol) were dissolved in 1ml DMSO, and DIPEA (58mg, 0.45mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain
(4R)-4-((S)-1-fluoroethyl)-3-(2-(((1S)-1-(4-methyl-7-trifluoromethyl-4,5-dihydroimidazo[1,5 -*a*]quinolin-3-yl)ethyl)amino)pyrimidin-4-yl)oxazolidin-2-one (Compound 14, white solid, 51.6mg, 68.2%). LCMS: m/z=505.0 (M+H)$^+$, RT=4.81min. $^1$H NMR (400 MHz, Chloroform-*d*) δ 8.20 (d, *J* = 5.7 Hz, 1H), 7.98 (s, 1H), 7.55 (s, 2H), 7.49 (d, *J* = 8.6 Hz, 1H), 7.44 (d, *J* = 5.7 Hz, 1H), 5.76 (br, 1H), 5.45 - 5.00 (m, 2H), 4.69 (d, *J* = 21.5 Hz, 1H), 4.52 (dt, *J* = 8.5, 3.7 Hz, 1H), 4.37 (t, *J* = 8.6 Hz, 1H), 3.70 - 3.35 (d, *J* = 63.6 Hz, 1H), 3.07 (dd, *J* = 15.5, 5.4 Hz, 1H), 2.85 2.70 (m, 1H), 1.58 (d, *J* = 6.7 Hz, 3H), 1.34 (dd, *J* = 23.1, 6.6 Hz, 3H), 1.11 (d, *J* = 7.1 Hz, 3H).

**Synthesis of compound 15**

(4R)-4-((S)-1-fluoroethyl)-3-(2-(((1S)-1-(4-methyl-7-trifluoromethyl-4*H*-benzo[*b*] imidazo[1,5-*d*][1,4]oxazin-3-yl)ethyl)amino)pyrimidin-4-yl)oxazolidin-2-one

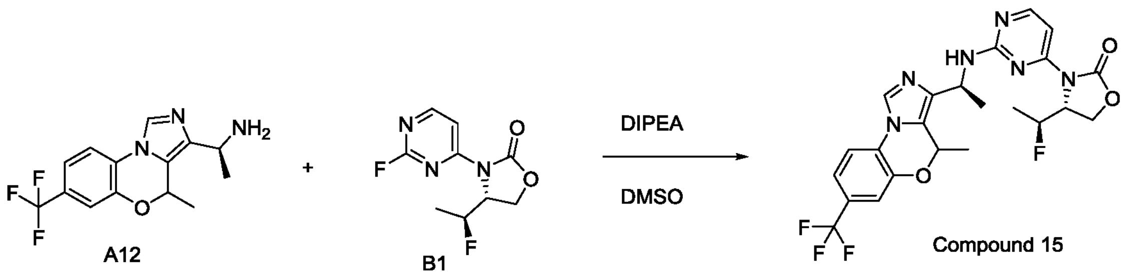


A12 (42.6mg, 0.14mmol) and B1 (34mg, 0.15mmol) were dissolved in 1ml DMSO, and DIPEA (54mg, 0.42mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (4R)-4-((S)-1-fluoroethyl)-3-(2-(((1S)-1-(4-methyl-7-trifluoromethyl-4*H*-benzo[*b*] imidazo[1,5-*d*][1,4]oxazin-3-yl)ethyl)amino)pyrimidin-4-yl)oxazolidin-2-one (Compound 15, white solid, 23.2mg, 32.7%). LCMS: m/z= 507.1 $(M+H)^+$, RT= 5.23min. $^1$H NMR (400 MHz, Chloroform-d) δ 8.22 (d, *J* = 5.8 Hz, 1H), 7.97 (d, *J* = 2.5 Hz, 1H), 7.55 - 7.47 (m, 2H), 7.37 - 7.29 (m, 2H), 5.71 (q, *J* = 6.7 Hz, 1H), 5.59 (q, *J* = 6.5 Hz, 1H), 5.21 (br, 2H), 4.70 (ddd, *J* = 26.5, 9.1, 2.1 Hz, 1H), 4.54 (dt, *J* = 8.3, 4.1 Hz, 1H), 4.39 (t, *J* = 9.0 Hz, 1H), 1.72 - 1.52 (m, 6H), 1.35 (dd, *J* = 23.1, 5.9 Hz, 3H).

**Synthesis of compound 16**

(S)-8-phenyl-7-(2-(((S)-1-(7-trifluoromethyl-4,5-dihydroimidazo[1,5-*a*]quinolin-3-yl)et hyl)amino)pyrimidin-4-yl)-2,5-dioxa-7-azaspiro[3.4]octan-6-one

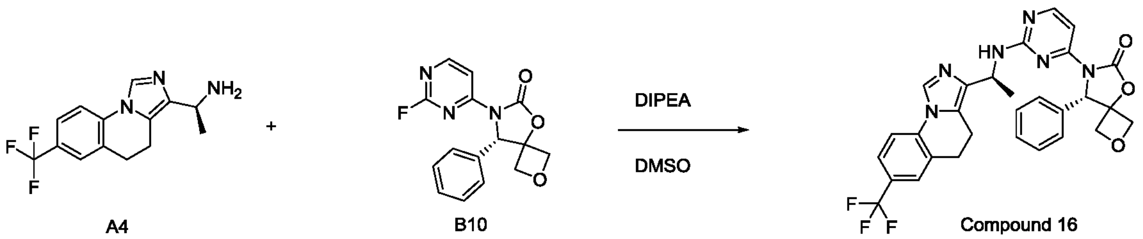


A4 (64.5mg, 0.23mmol) and B10 (75mg, 0.25mmol) were dissolved in 1.6ml DMSO, and DIPEA (89mg, 0.69mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (S)-8-phenyl-7-(2-(((S)-1-(7-trifluoromethyl-4,5-dihydroimidazo[1,5-*a*]quinolin-3-yl)ethyl)a mino)pyrimidin-4-yl)-2,5-dioxa-7-azaspiro[3.4]octan-6-one (Compound 16, white solid, 33.1mg, 25.7%). LCMS: m/z= 563.2 $(M+H)^+$, RT=5.01 min. $^1$H NMR (400 MHz, Chloroform-*d*) δ 8.07 (d, *J* = 5.7 Hz, 1H), 7.92 (s, 1H), 7.46 (dt, *J* = 18.7, 8.4 Hz, 3H), 7.35 (d, *J* = 5.6 Hz, 1H), 7.30 - 7.05 (m, 5H), 5.80 - 5.40 (m, 2H), 5.20 - 4.59 (m, 3H), 4.52 (d, *J* = 8.4 Hz, 1H), 4.03 (d, *J* = 8.2 Hz, 1H), 2.94 - 2.05 (m, 4H), 1.48 (br, 3H).

**Synthesis of compound 17**

(4S)-4-isopropyl-5-methyl-3-(2-(((S)-1-(7-trifluoromethyl-4,5-dihydroimidazo[1,5-*a*]qu inolin-3-yl)ethyl)amino)pyrimidin-4-yl)oxazolidin-2-one

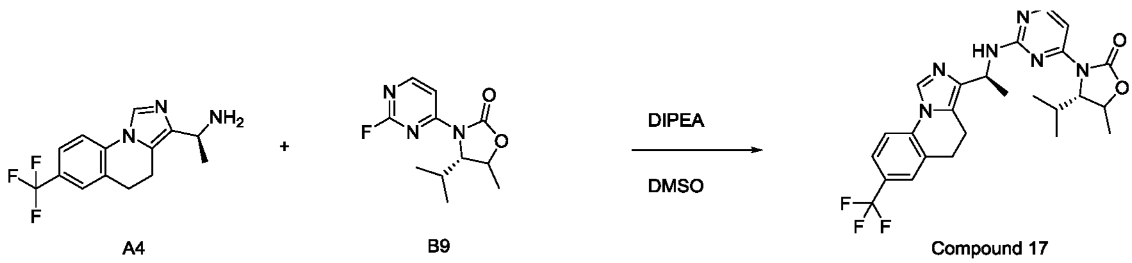


A4 B9 Compound 17

A4 (55.4mg, 0.20mmol) and B9 (53mg, 0.22mmol) were dissolved in 1.3ml DMSO, and DIPEA (78mg, 0.60mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (4S)-4-isopropyl-5-methyl-3-(2-(((S)-1-(7-trifluoromethyl-4,5-dihydroimidazo[1,5-*a*]quinoli n-3-yl)ethyl)amino)pyrimidin-4-yl)oxazolidin-2-one (Compound 17, white solid, 53.1mg, 53.0%). LCMS: m/z= 501.2 $(M+H)^+$, RT= 5.04 min. $^1H$ NMR (400 MHz, Chloroform-*d*)$\delta$ 8.16 (d, $J$ = 5.7 Hz, 1H), 7.98 (s, 1H), 7.57 - 7.45 (m, 3H), 7.41 (d, $J$ = 5.7 Hz, 1H), 5.71 (br, 1H), 5.19 (br, 1H), 4.72 (br, 2H), 3.18 - 2.82 (m, 4H), 2.22 (br, 1H), 1.57 (d, $J$ = 6.8 Hz, 3H), 1.51 (d, $J$ = 6.3 Hz, 3H), 0.93 (d, $J$ = 6.3 Hz, 6H).

**Synthesis of compound 18**

(S)-4-isopropyl-5,5-dimethyl-3-(2-(((S)-1-(7-trifluoromethyl-4,5-dihydroimidazo[1,5-*a*] quinolin-3-yl)ethyl)amino)pyrimidin-4-yl)oxazolidin-2-one

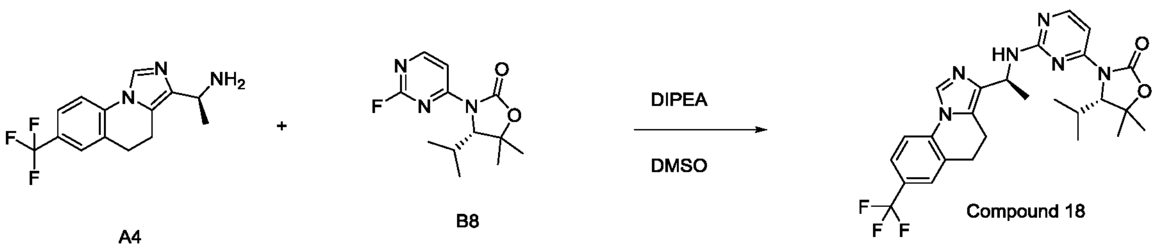


A4 B8 Compound 18

A4 (64.7mg, 0.23mmol) and B8 (63mg, 0.25mmol) were dissolved in 1.6ml DMSO, and DIPEA (89mg, 0.69mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (S)-4-isopropyl-5,5-dimethyl-3-(2-(((S)-1-(7-trifluoromethyl-4,5-dihydroimidazo[1,5-*a*]quin olin-3-yl)ethyl)amino)pyrimidin-4-yl)oxazolidin-2-one (Compound 18, white solid, 60.6mg, 51.2%). CMS: m/z= 515.2$(M+H)^+$, RT=5.06min. $^1H$ NMR (400 MHz, Chloroform-*d*)$\delta$ 8.15 (d, $J$ = 5.7 Hz, 1H), 7.98 (s, 1H), 7.57 - 7.45 (m, 3H), 7.40 (d, $J$ = 5.7 Hz, 1H), 5.70 (br, 1H), 5.18 (br, 1H), 4.47 (s, 1H), 3.15 - 2.80 (m, 4H), 2.18 (br, 1H), 1.56 (d, $J$ = 6.8 Hz, 3H), 1.50 (s, 3H), 1.35 (s, 3H), 0.90 (d, $J$ = 6.4 Hz, 6H).

**Synthesis of compound 19**

(S)-6-(2-((1-(7-trifluoromethyl-4,5-dihydroimidazo[1,5-*a*]quinolin-3-yl)ethyl)amino)pyr imidin-4-yl)-4-oxa-6-azaspiro[2.4] heptan-5-one

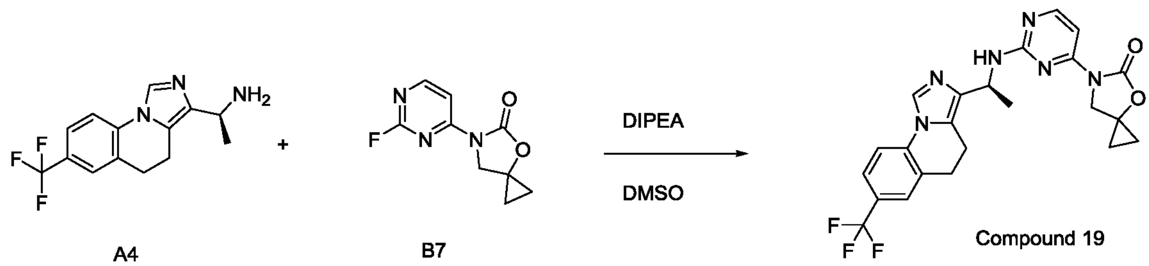


A4 (54.7mg, 0.19mmol) and B7 (43.9mg, 0.21mmol) were dissolved in 1.2ml DMSO, and DIPEA (74mg, 0.57mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (S)-6-(2-((1-(7-trifluoromethyl-4,5-dihydroimidazo[1,5-*a*]quinolin-3-yl)ethyl)amino)pyrimid in-4-yl)-4-oxa-6-azaspiro[2.4] heptan-5-one (Compound 19, white solid, 51.6mg, 57.7%). LCMS: m/z= 471.1 $(M+H)^+$, RT= 5.04min. $^1$H NMR (400 MHz, Chloroform-*d*)δ 8.16 (d, *J* = 5.7 Hz, 1H), 7.98 (s, 1H), 7.58 - 7.46 (m, 3H), 7.40 (d, *J* = 5.7 Hz, 1H), 5.80 (br, 1H), 5.20 (br, 1H), 4.16 (s, 2H), 3.15 - 2.84 (m, 4H), 1.57 (d, *J* = 6.8 Hz, 3H), 1.26 (br, 2H), 0.80 (br, 2H). $^{19}$F NMR (376 MHz, Chloroform-*d*) δ -62.28.

**Synthesis of compound 20**

(S)-4-phenyl-3-(2-(((S)-1-(7-trifluoromethyl-4,5-dihydroimidazo[1,5-*a*]quinolin-3-yl)et hyl)amino)pyrimidin-4-yl)oxazolid-in-2-one

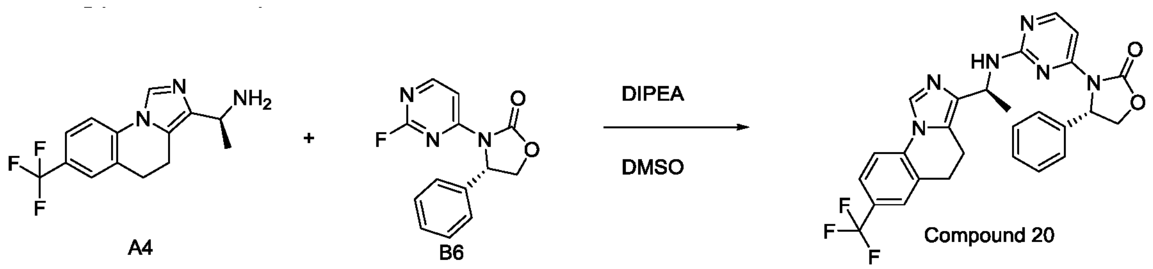


A4 (62.8mg, 0.22mmol) and B6 (62mg, 0.24mmol) were dissolved in 1.5ml DMSO, and DIPEA (85mg, 0.66mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (S)-4-phenyl-3-(2-(((S)-1-(7-trifluoromethyl-4,5-dihydroimidazo[1,5-*a*]quinolin-3-yl)ethyl)a mino)pyrimidin-4-yl)oxazolidin-2-one (Compound 20, white solid, 63mg, 55.0%). LCMS: m/z= 521.1 $(M+H)^+$, RT= 4.80min. $^1$H NMR (400 MHz, Chloroform-*d*)δ 8.15 (d, *J* = 5.7 Hz, 1H), 7.96 (s, 1H), 7.59 - 7.44 (m, 4H), 7.35 - 7.14 (m, 5H), 6.03 - 5.48 (m, 2H), 5.25 - 4.50 (m, 2H), 4.24 (dd, *J* = 8.6, 3.6 Hz, 1H), 3.00 - 2.20 (m, 4H), 1.53 (d, *J* = 6.4 Hz, 3H).

**Synthesis of compound 21**

(R)-4-((S)-1-fluoroethyl)-3-(2-(((S)-1-(7-trifluoromethyl-4*H*-benzo[*b*]imidazo[1,5-*d*][1, 4]thiazin-3-yl)ethyl)amino)pyrimidin-4-yl)oxazolidin-2-one

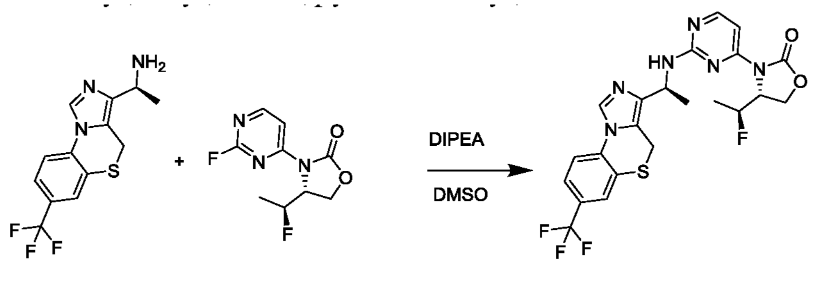


A18 B1 Compound 21

A18 (30.6mg, 0.10mmol) and B1 (25mg, 0.11mmol) were dissolved in 1.2ml DMSO, and DIPEA (39mg, 0.30mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-4-((S)-1-fluoroethyl)-3-(2-(((S)-1-(7-trifluoromethyl-4*H*-benzo[*b*]imidazo[1,5-*d*][1,4]thi azin-3-yl)ethyl)amino)pyrimidin-4-yl)oxazolidin-2-one (Compound 21, white solid, 32.9mg, 63.3%). LCMS: m/z= 509.1(M+H)$^+$, RT= 4.98min. $^1$H NMR (400 MHz, Chloroform-*d*) δ 8.20 (d, *J* = 5.8 Hz, 1H), 7.95 (s, 1H), 7.71 (s, 1H), 7.57 - 7.43 (m, 3H), 5.59 (br, 1H), 5.20 (br, 2H), 4.67 (dd, *J* = 26.6, 7.0 Hz, 1H), 4.52 (dd, *J* = 8.9, 3.4 Hz, 1H), 4.37 (t, *J* = 9.0 Hz, 1H), 4.29 - 3.96 (m, 2H), 1.62 (d, *J* = 6.8 Hz, 3H), 1.31 (d, *J* = 22.4 Hz, 3H).

**Synthesis of compound 22**

(S)-3-(2-(((S)-1-(7-chloro-4*H*-benzo[*b*]imidazo[1,5-*d*][1,4]oxazin-3-yl)ethyl)ami no)pyrimidin-4-yl)-4-cyclopropyloxazolidin-2-one

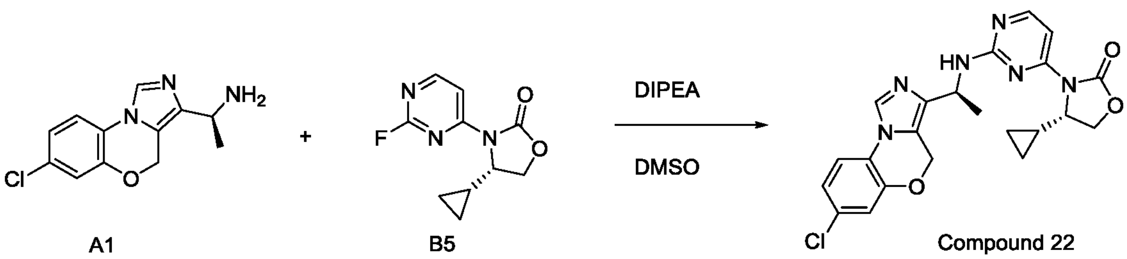


A1 B5 Compound 22

A1 (45mg, 0.18mmol) and B5 (45mg, 0.20mmol) were dissolved in 1.2ml DMSO, and DIPEA (70mg, 0.54mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (S)-3-(2-(((S)-1-(7-chloro-4*H*-benzo[*b*]imidazo[1,5-*d*][1,4]oxazin-3-yl)ethyl)amino)py rimidin-4-yl)-4-cyclopropyloxazolidin-2-one (Compound 22, white solid, 69.1mg, 84.8%). LCMS: m/z= 453.0 (M+H)$^+$, RT= 4.77min. $^1$H NMR (400 MHz, Chloroform-d) δ 8.20 (d, *J* = 5.8 Hz, 1H), 7.90 (s, 1H), 7.44 (d, *J* = 5.8 Hz, 1H), 7.34 (d, *J* = 8.5 Hz, 1H), 7.09 (d, *J* = 2.2 Hz, 1H), 7.03 (dd, *J* = 8.5, 2.2 Hz, 1H), 5.55 (br, 1H), 5.30 (d, *J* = 13.4 Hz, 1H), 5.24 - 5.08 (m, 2H), 4.51 (br, 1H), 4.33 (t, *J* = 8.4 Hz, 1H), 4.09 (dd, *J* = 8.8, 2.4 Hz, 1H), 1.62 (d, *J* = 6.9 Hz, 3H), 1.18 (br, 1H), 0.66 - 0.15 (m, 4H).

**Synthesis of compound 23**

(R)-4-((S)-1-fluoroethyl)-3-(2-(((S)-1-(7-methyl-4,5-dihydroimidazo[1,5-*a*]quinolin-3-y l)ethyl)amino)pyrimidin-4-yl)oxazolidin-2-one

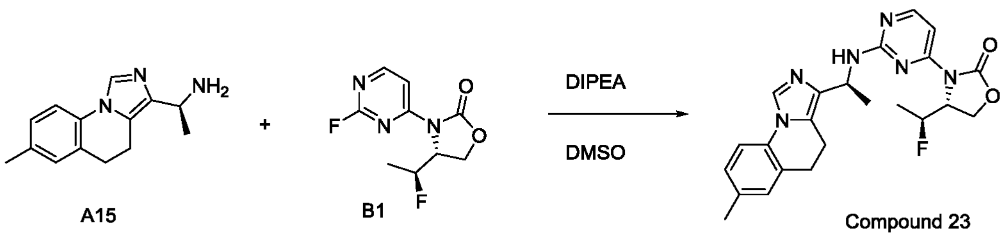


A15 B1 Compound 23

A15 (45.8mg, 0.20mmol) and B1 (50.4mg, 0.22mmol) were dissolved in 1.5ml DMSO, and DIPEA (77.5mg, 0.60mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-4-((S)-1-fluoroethyl)-3-(2-(((S)-1-(7-methyl-4,5-dihydroimidazo[1,5-*a*]quinolin-3-yl)eth yl)amino)pyrimidin-4-yl)oxazolidin-2-one (Compound 23, white solid, 54.7mg, 62.2%). LCMS: m/z= 437.2 $(M+H)^+$, RT= 4.13min. $^1H$ NMR (400 MHz, Chloroform-d) δ 8.18 (d, *J* = 5.7 Hz, 1H), 7.91 (s, 1H), 7.42 (d, *J* = 5.7 Hz, 1H), 7.26 (d, *J* = 8.6 Hz, 1H), 7.10 - 7.03 (m, 2H), 5.83 (br, 1H), 5.14 (br, 2H), 4.79 - 4.60 (m, 1H), 4.51 (dd, *J* = 8.9, 3.4 Hz, 1H), 4.36 (t, *J* = 8.9 Hz, 1H), 3.10 - 2.75 (m, 4H), 2.31 (s, 3H), 1.56 (d, *J* = 6.8 Hz, 3H), 1.30 (d, *J* = 18.9 Hz, 3H).

**Synthesis of compound 24**

(R)-3-(2-(((S)-1-(7-fluoro-4*H*-benzo[*b*]imidazo[1,5-*d*][1,4]oxazin-3-yl)ethyl)amino)pyri midin-4-yl)-4-((S)-1-fluoroethyl)oxazolidin-2-one

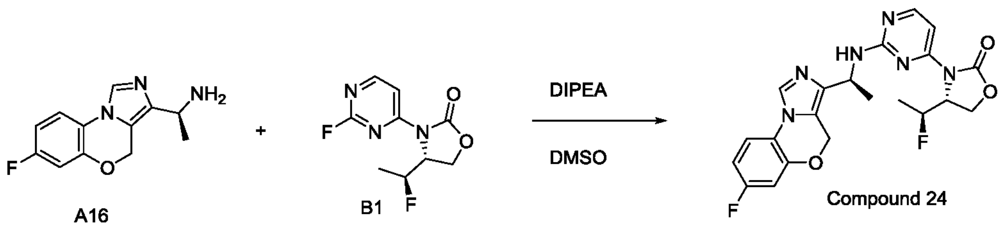


A16 B1 Compound 24

A16 (46.1mg, 0.20mmol) and B1 (50.4mg, 0.22mmol) were dissolved in 1.5ml DMSO, and DIPEA (77.5mg, 0.60mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-3-(2-(((S)-1-(7-fluoro-4*H*-benzo[*b*]imidazo[1,5-*d*][1,4]oxazin-3-yl)ethyl)amino)pyrimidi n-4-yl)-4-((S)-1-fluoroethyl)oxazolidin-2-one (Compound 24, white solid, 55.2mg, 63.1%).
LCMS: m/z=443.1 $(M+H)^+$, RT= 4.63min. $^1H$ NMR (400 MHz, Chloroform-*d*) δ 8.21 (d, *J* = 5.7 Hz, 1H), 7.91 (s, 1H), 7.46 (d, *J* = 5.7 Hz, 1H), 7.36 (dd, *J* = 8.5, 4.7 Hz, 1H), 7.08 - 6.96 (m, 2H), 5.66 (d, *J* = 7.7 Hz, 1H), 5.17 (br, 2H), 4.70 (dd, *J* = 26.2, 7.4 Hz, 1H), 4.53 (dd, *J* = 8.8, 3.4 Hz, 1H), 4.38 (t, *J* = 8.9 Hz, 1H), 2.95 (d, *J* = 70.2 Hz, 4H), 1.58 (d, *J* = 6.8 Hz, 3H), 1.33 (dd, *J* = 22.9, 5.6 Hz, 3H).

**Synthesis of compound 25**

(R)-4-((S)-1-fluoroethyl)-3-(2-(((S)-1-(7-methoxy-4*H*-benzo[*b*]imidazo[1,5-*d*][1,4]oxazi n-3-yl)ethyl)amino)pyrimidin-4-yl)oxazolidin-2-one

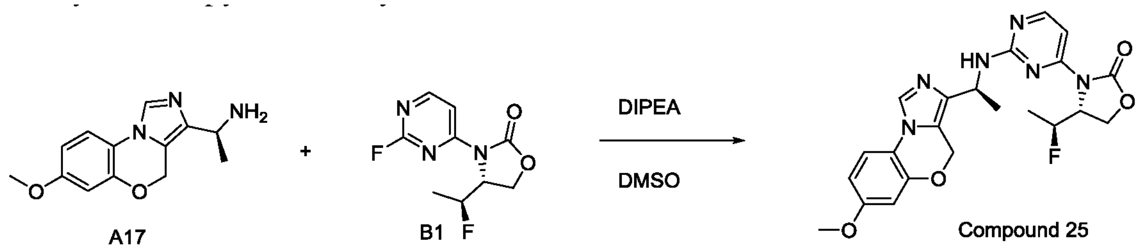


A17 B1 Compound 25

A17 (42.8mg, 0.17mmol) and B1 (43.5mg, 0.19mmol) were dissolved in 1.2ml DMSO, and DIPEA (65.9mg, 0.51mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-4-((S)-1-fluoroethyl)-3-(2-(((S)-1-(7-methoxy-4*H*-benzo[*b*]imidazo[1,5-*d*][1,4]oxazin-3-yl)ethyl)amino)pyrimidin-4-yl)oxazolidin-2-one (Compound 25, white solid, 58.2mg, 75.3%). LCMS: m/z= 455.1 $(M+H)^+$, RT= 4.17min. $^1H$ NMR (400 MHz, Chloroform-*d*) δ 8.18 (d, *J* = 5.8 Hz, 1H), 7.84 (s, 1H), 7.44 (d, *J* = 5.8 Hz, 1H), 7.30 (d, *J* = 8.7 Hz, 1H), 6.62 - 6.54 (m, 2H), 5.82 (br, 1H), 5.34 - 4.92 (m, 4H), 4.75 - 4.56 (m, 1H), 4.48 (dd, *J* = 8.8, 3.3 Hz, 1H), 4.34 (t, *J* = 9.0 Hz, 1H), 3.76 (s, 3H), 1.59 (d, *J* = 6.9 Hz, 3H), 1.21 (br, 3H).

**Synthesis of compound 26**

(R)-3-(2-(((S)-1-(7-cyclopropyl-4*H*-benzo[*b*]imidazo[1,5-*d*][1,4]oxazin-3-yl)ethyl)amin o)pyrimidin-4-yl)-4-((S)-1-fluoroethyl)oxazolidin-2-one

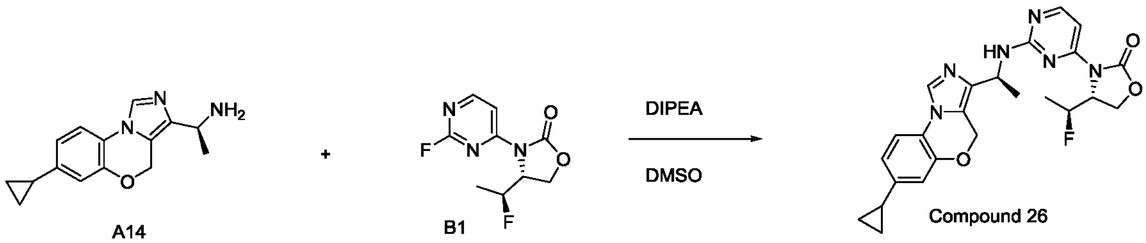


A14 B1 Compound 26

A14 (34.5mg, 0.14mmol) and YUX-A (34.4mg, 0.15mmol) were dissolved in 1.5ml DMSO, and DIPEA (54mg, 0.42mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-3-(2-(((S)-1-(7-cyclopropyl-4*H*-benzo[*b*]imidazo[1,5-*d*][1,4]oxazin-3-yl)ethyl)amino)pyr imidin-4-yl)-4-((S)-1-fluoroethyl)oxazolidin-2-one (Compound 26, white solid, 36.5mg, 56.2%). LCMS: m/z= 465.1 $(M+H)^+$, RT=4.97 min. $^1H$ NMR (400 MHz, Chloroform-*d*)δ 8.19 (d, *J* = 5.7 Hz, 1H), 7.87 (s, 1H), 7.46 (d, *J* = 5.7 Hz, 1H), 7.27 (d, *J* = 8.0 Hz, 1H), 6.79 - 6.70 (m, 2H), 5.71 (br, 1H), 5.31 - 4.89 (m, 4H), 4.65 (dd, *J* = 25.8, 7.7 Hz, 1H), 4.50 (dd, *J* = 8.7, 2.9 Hz, 1H), 4.36 (t, *J* = 9.0 Hz, 1H), 1.84 (tt, *J* = 8.5, 5.1 Hz, 1H), 1.60 (d, *J* = 6.8 Hz, 3H), 1.24 (br, 3H), 0.96 (q, *J* = 6.3 Hz, 2H), 0.66 (q, *J* = 4.9 Hz, 2H).

**Synthesis of compound 27**

3-((S)-1-((4-((R)-4-((S)-1-fluoroethyl)-2-oxyoxazolidin-3-yl)pyrimidin-2-yl)amino)ethy 1)-4*H*-benzo[*b*]imidazo[1,5-*d*][1,4] oxazin-7-nitrile

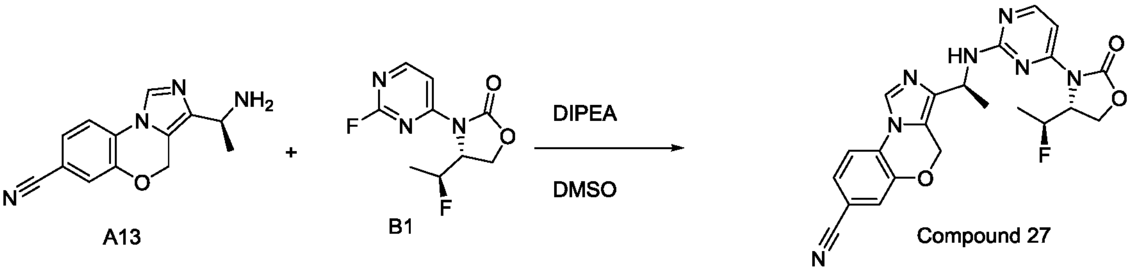


A13 B1 Compound 27

A13 (38.3mg, 0.16mmol) and B1 (41.3mg, 0.18mmol) were dissolved in 1.6ml DMSO, and DIPEA (62mg, 0.48mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain 3-((S)-1-((4-((R)-4-((S)-1-fluoroethyl)-2-oxyoxazolidin-3-yl)pyrimidin-2-yl)amino)ethyl)-4*H* -benzo[*b*]imidazo[1,5-*d*][1,4] oxazin-7-nitrile (Compound 27, white solid, 64.2mg, 89.3%). LCMS: m/z=450.0 $(M+H)^+$, RT= 4.30 min. $^1H$ NMR (400 MHz, Chloroform-*d*) δ 8.17 (d, *J*= 5.8 Hz, 1H), 7.96 (s, 1H), 7.49 (d, *J* = 8.6 Hz, 1H), 7.45 (d, *J* = 5.7 Hz, 1H), 7.38 - 7.31 (m, 2H), 5.64 (br, 1H), 5.40 - 4.95 (m, 4H), 4.72 - 4.56 (m, 1H), 4.49 (dd, *J* = 8.8, 3.2 Hz, 1H), 4.36 (t, *J* = 9.0 Hz, 1H), 1.59 (d, *J* = 6.9 Hz, 3H), 1.25 (d, *J* = 16.6 Hz, 3H).

**Synthesis of compound 28**

(R)-4-((S)-1-fluoroethyl)-3-(2-(((S)-1-(8-trifluoromethyl-4,5-dihydroimidazo[1,5-*a*]quin olin-3-yl)ethyl)amino)pyrimidin-4-yl)oxazolidin-2-one

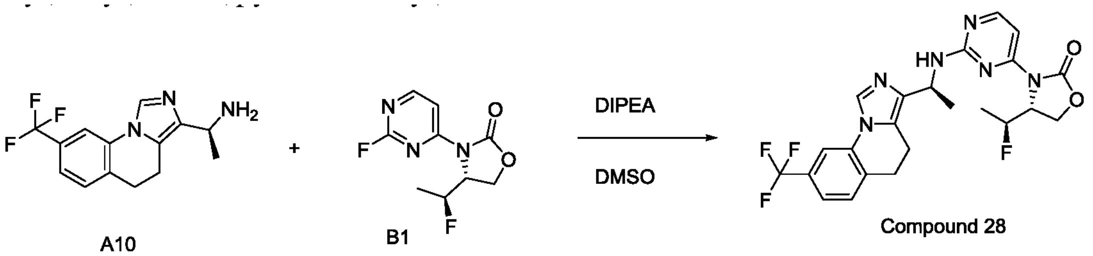


A10 B1 Compound 28

A10 (40.7mg, 0.14mmol) and B1 (35mg, 0.15mmol) were dissolved in 1.2ml DMSO, and DIPEA (54mg, 0.42mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain (R)-4-((S)-1-fluoroethyl)-3-(2-(((S)-1-(8-trifluoromethyl-4,5-dihydroimidazo[1,5-*a*]quinolin-3-yl)ethyl)amino)pyrimidin-4-yl)oxazolidin-2-one (Compound 28, white solid, 52.3mg, 71.0%). LCMS: m/z= 491.1 $(M+H)^+$, RT= 4.43min. NMR (400 MHz, Chloroform-*d*) δ 8.19 (d, *J* = 5.7 Hz, 1H), 7.99 (s, 1H), 7.61 (s, 1H), 7.44 (d, *J* = 5.7 Hz, 1H), 7.41 (s, 2H), 5.74 (br, 1H), 5.17 (br, 2H), 4.80 - 4.58 (m, 1H), 4.52 (dd, *J* = 8.9, 3.4 Hz, 1H), 4.37 (t, *J* = 8.9 Hz, 1H), 3.15 - 2.80 (m, 4H), 1.57 (d, *J* = 6.8 Hz, 3H), 1.41 - 1.23 (m, 3H).

**Synthesis of compound 29**

(R)-3-(2-(((S)-1-(6-chloro-4,5-dihydroimidazo[1,5-*a*]quinolin-3-yl)ethyl)amino)pyrimid in-4-yl)-4-((S)-1-fluoroethyl)oxazolidin-2-one

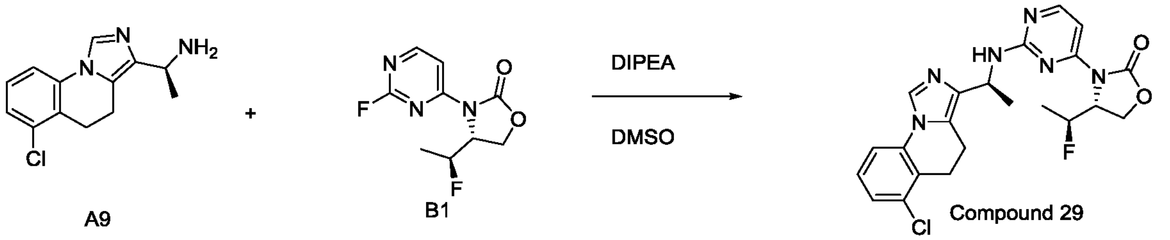

A9 B1 Compound 29

A9 (44.5mg, 0.18mmol) and B1 (46mg, 0.20mmol) were dissolved in 1.2ml DMSO, and DIPEA (70mg, 0.54mmol) was added. The mixture was stirred at 100 °C for 1.5 hours. The mixture was extracted with water and ethyl acetate, and the organic phase was dried with anhydrous sodium sulfate, filtered, concentrated, then separated by Combi-Flash flash silica gel chromatography, and lyophilized to obtain

(R)-3-(2-(((S)-1-(6-chloro-4,5-dihydroimidazo[1,5-*a*]quinolin-3-yl)ethyl)amino)pyrimidin-4-yl)-4-((S)-1-fluoroethyl)oxazolidin-2-one (Compound 29, white solid, 57.5mg, 70.0%).

LCMS: m/z= 457.1$(M+H)^+$, RT=4.47 min. $^1H$ NMR (400 MHz, Chloroform-*d*) δ 8.21 (d, *J* = 5.7 Hz, 1H), 7.95 (s, 1H), 7.46 (d, *J* = 5.7 Hz, 1H), 7.34 (dd, *J* = 7.0, 2.2 Hz, 1H), 7.31 - 7.19 (m, 2H), 5.81 (br, 1H), 5.18 (br, 2H), 4.85 - 4.60 (m, 1H), 4.54 (dd, *J* = 8.9, 3.4 Hz, 1H), 4.40 (t, *J* = 8.9 Hz, 1H), 3.25 - 2.73 (m, 4H), 1.59 (d, *J* = 6.8 Hz, 3H), 1.47 - 1.18 (m, 3H).

## Testing example 1: Effect of the compound of the disclosure on IDH1 enzyme activity at the molecular level

Control compounds: AG120, CAS:1448346-63-1; IDH305, CAS:1628805-46-8 Reagents, consumables and instruments:

Substrate α-KG, NADPH, Diphhorase and Resazurin were purchased from Sigma; all the other reagents were purchased from Sinopharm Chemical Reagent Co., Ltd.

The reaction microwell 96 plate (COSTAR, 3915) was purchased from Costar.

The multifunctional enzyme reader for reading plate in the experiment was the product of Molecular Devices, model: spectramax i3.

The experimental water was Millipore distilled water.

Compound preparation: 12000g compound was centrifuged for 5min, and then added with DMSO to prepare 20mM stock solution. After the vortex was uniform, ultrasonic wave was conducted for 10min and the stock solution was stored at -20 °C.

Test method: The enzyme activity of IDH1 converting α-KG to 2HG was measured by the consumption of NADPH. After the enzymatic reaction finished, the amount of residual NADPH can be reflected by fluorescence signal generated by the addition of catalytically excessive diphhorase and reazurin.

Detection of the effect on IDH1 R132H enzyme activity: 50μL enzyme system (150mM NaCl, 20mM Tris, pH = 7.5, 10mM $MgCl_2$, 0.05%(w/v) bovine serum albumin, 100ng IDH1 R132H) and 1μL compound were added into the 96 well plate and incubated for 60min in dark at room temperature. 50μL mixed liquid of substrate α-KG and NADPH (final concentration of substrate α-KG was ImM, final concentration of NADPH was 20μM), and the mixture was incubated in dark for 60min at room temperature. Detection reaction: 50μL 1×detection buffer diluted 0.01 unit diabase and 30μM resazurin were added into each well, and the mixture was incubated in dark at room temperature for 10min. Reading plate: PerkinElmer at Ex 544Em 590 was used to read the plate. IC50 value was calculated by GraphPad Prism software.

Detection of the effect on IDH1 R132C enzyme activity: 50μL enzyme system (50mM $K_2HPO_4$ (pH=6.5), 40mM $NaHCO_3$, 10% glycerol, 5mM $MgCl_2$, 0.03%(w/v) bovine serum albumin, 80ng IDH1 R132C) and 1μL compound were added into the 96 well plate and incubated for 60min in dark at room temperature. 50μL mixed liquid of substrate α-KG and NADPH (final concentration of substrate α-KG was 2mM, final concentration of NADPH was 20μM), and the plate was incubated in dark for 60min at room temperature. Detection reaction: 50μL 1×detection buffer diluted 0.01 unit diabase and 30μM resazurin were added into each well, and the mixture was incubated in dark at room temperature for 10min. Reading plate: spectramax i3 at Ex 544Em 590 was used to read the plate. IC50 value was calculated by GraphPad Prism software.

Results

Table 1 shows the $IC_{50}$ values of some compounds of the disclosure.

The letter A represents that $IC_{50}$ is less than 50nM;
the letter B represents that $IC_{50}$ is 50nM to 500nM;
the letter C represents that IC50 is more than 500nM;
the results are shown in the following table.

| Compound number | IDH1 R132C | IDH1 R132H |
|---|---|---|
| 1 | A | A |
| 2 | A | A |
| 3 | A | A |
| 4 | A | A |
| 5 | A | A |
| 6 | A | A |
| 7 | A | A |
| 8 | A | A |
| 9 | A | B |
| 10 | B | B |
| 11 | A | B |
| 12 | A | A |
| 13 | A | A |
| 14 | B | B |
| 15 | B | B |
| 16 | A | A |
| 17 | A | A |
| 18 | A | B |
| 19 | C | C |
| 20 | A | A |
| 21 | A | A |
| 22 | A | B |
| 23 | A | A |
| 24 | A | A |
| 25 | B | B |
| 26 | A | A |
| 27 | B | B |
| 28 | A | A |
| 29 | B | A |
| AG120 | A | A |
| IDH305 | N/A | N/A |

The results show that the compounds of the disclosure can effectively inhibit the activities of various IDH1 mutants (including IDH1 R132C mutant and IDH1 R132H mutant) at very low concentration (≤100nm).

**Testing example 2: Determination of the inhibition of the compound of the disclosure on 2HG of fibrosarcoma cell line HT1080**

In this experiment, the inhibitory activity of the compound of the disclosure on the concentration level of 2HG of the fibrosarcoma cell line HT1080 was determined by the following method.

Cell sample preparation: Human fibrosarcoma cell line HT1080 expressing IDH1 mutant IDH1-R132C was

cultured in DMEM medium containing 10% fetal bovine serum. The cells were digested with trypsin and inoculated into a 24 well cell culture plate with a density of 10000 cells/well. The cells were cultured in a 5% $CO_2$ cell incubator at 37 °C. After overnight culture, the test compound was added to the cells. The final concentration of DMSO was 0.1%, and DMSO was used as control. The plate was then placed in a cell incubator and cultured for 48 hours.

Detection of 2HG: 2-HG was measured by PicoProbeTM D-2-Hydroxyglutarate (D2HG) Assay Kit of BioVision. 500uL cell supernatant was taken from each well and added to a 10kD centrifuge column, 12000g, and centrifuged for 10 minutes to remove the effect of enzyme activity. 2.5uL of each sample was added to a 384 well white flat-bottom reaction plate (CORNING, 3576). 25uL reaction system (21.425uL D2HG Assay Buffer, 0.5uL D2HG Enzyme, 0.5uL D2HG Developer, 0.075uL PicoProbe) was mixed well and incubate at 37 °C for 60 minutes Reading plate: spectramax i3 at Ex 544Em 590 was used to read the plate. IC50 value was calculated by GraphPad Prism software.

**Calculation of inhibition rate and $IC_{50}$**

The inhibition rate of the sample was obtained by the following formula:

$$\text{Inhibition rate (\%)} = \left(1 - \frac{\text{2HG content of compound well}}{\text{2HG content of negative control}}\right) \times 100\%$$

$IC_{50}$ value was calculated by GraphPad Prism software. The inhibitory activities of some compounds on the 2HG level of HT1080 were listed in Table 2.

Table 2:

| **Compound number** | **HT1080 2HG inhibitory $IC_{50}$(nM)** |
|---|---|
| Compound 1 | 60 |
| Compound 2 | 12 |
| Compound 3 | 13 |
| Compound 4 | 54 |
| Compound 5 | 16 |
| Compound 6 | 20 |
| Compound 7 | 35 |
| Compound 8 | 10 |
| Compound 9 | 45 |
| Compound 12 | 24 |
| Compound 13 | 35 |
| Compound 16 | 140 |
| Compound 17 | 87 |
| Compound 18 | 26 |
| Compound 20 | 7 |
| Compound 21 | 17 |
| Compound 22 | 104 |
| Compound 23 | 22 |
| Compound 26 | 167 |
| Compound 28 | 13 |
| Positive agent: AG120, $IC_{50}$ is 25 nM | |

The results show that the compounds of the disclosure exhibited inhibitory activity against 2HG in different cell

lines, and their semi active inhibitory concentrations were all below 100nM, and some compounds could be as low as about 10nM.

**Testing example 3: Pharmacokinetic determination of the compound of the disclosure**

The following method was used to determine the pharmacokinetic parameters of the compound of the disclosure.
Healthy male adult rats/mice were used in the study, and each group of animal was given 5-100mg/Kg by gavage. Fasting lasted from 10 hours before administration to 4 hours after administration. Blood samples were collected at different time points after administration, and the plasma content of compounds was determined (LC-MS/MS). The plasma concentration-time relationship was analyzed by professional software (winnonlin), and the pharmacokinetic parameters of the compound were calculated.
The results show that the compounds of the disclosure have excellent pharmacokinetic properties and brain permeability.
All publications mentioned herein are cited by reference in the present disclosure to the same extent as if each publication is separately cited by reference. In addition, it should be understood that after reading the above contents of the disclosure, those skilled in the art can make various modifications to the disclosure, and these equivalent forms also fall within the scope of the claims attached to the disclosure.

## Claims

1. A compound of Formula I, or a stereoisomer, racemate thereof, or a pharmaceutically acceptable salt thereof, or an isotope-substituted derivative thereof:

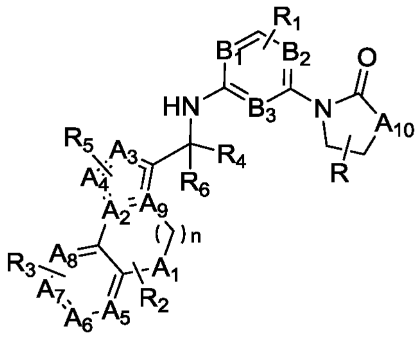

I

wherein,

n is 0, 1, 2 or 3;
$A_1$ is selected from the group consisting of: a bond, $-CH_2-$, -CH(R)-, $-C(R)_2-$, $-CH_2O-$, -CH(R)O-, $-C(R)_2O-$, $-CH_2N(R)-$, -CH(R)N(R)-, $-C(R)_2N(R)-$, -CH=CH-, -C(R)=CH-, -C(R)=C(R)-, -CH=N-, -C(R)=N-, -NR-, -O-, -S-,

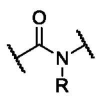 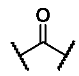 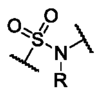 ;

$A_2$, $A_3$, $A_4$, $A_5$, $A_6$, $A_7$, $A_8$, $A_9$, $A_{10}$ are independently selected from the group consisting of: $C(R)_{2-}$, CH(R), NR, O, S, CR or N;
$B_1$, $B_2$, $B_3$ are independently selected from the group consisting of: CR or N;
the dotted line represents a double bond or null;
R is selected from the group consisting of: H, halogen, CN, substituted or unsubstituted C1-C4 alkyl, substituted or unsubstituted C1-C4 alkoxy, substituted or unsubstituted $C_3$-$C_{10}$ cycloalkyl, substituted or unsubstituted $C_6$-$C_{12}$ aryl, and substituted or unsubstituted 3 to 12-membered heteroaryl; or two R groups together form a group selected from the group consisting of: substituted or unsubstituted $-(CH_2)_m-$ structure, substituted or unsubstituted $-CH_2-O-CH_2-$ structure, and substituted or unsubstituted $-O-CH_2-O-$ structure; wherein, m is 2 or

3; or when two R groups are connected to the same carbon atom, the two R groups form a C=O double bond (carbonyl group) with the carbon atom;

$R_4$, $R_6$ are independently selected from the group consisting of: hydrogen, deuterium, methyl, trideuteriomethyl, dideuteriomethyl, monodeuteriomethyl, ethyl, trifluoromethyl, difluoromethyl, monofluoromethyl, substituted or unsubstituted C1-C4 alkyl, and substituted or unsubstituted C1-C4 alkoxy; or $R_4$ and $R_6$ together form a group selected from the group consisting of: =O (with the carbon atom it connects thereto forming a carbonyl group), substituted or unsubstituted $-(CH_2)_m-$ structure, and substituted or unsubstituted $-CH_2-O-CH_2-$structure; wherein, m is 2 or 3;

$R_1$, $R_2$, $R_3$, $R_5$ are independently one or more substituents corresponding to any position on the five-membered or six-membered ring, and are selected from the group consisting of: H, hydroxyl, cyano, halogen, trifluoromethyl, substituted or unsubstituted C1-C4 alkyl, substituted or unsubstituted C1-C4 alkoxy, substituted or unsubstituted $C_3$-$C_{10}$ cycloalkyl, substituted or unsubstituted $C_6$-$C_{12}$ aryl, and substituted or unsubstituted 3 to 12-membered heteroaryl;

wherein each chiral center can be independently R configuration or S configuration;

said "substituted" refers to that one or more hydrogen atoms on the group are substituted by substituent(s) selected from the group consisting of: halogen, amino, cyano, $C_1$-$C_6$ alkyl, $C_1$-$C_6$ alkoxy, $C_1$-$C_6$ halogenated alkyl, carbonyl($C_{2-10}$)alkoxy, carbonyl($C_{7-10}$)aryloxy, acylamino($C_{2-10}$)alkyl, unsubstituted $C_6$-$C_{12}$ aryl or 3 to 12-membered heteroaryl, or $C_6$-$C_{12}$ aryl or 3 to 12-membered heteroaryl substituted by 1-3 substituents selected from the group consisting of: halogen, unsubstituted or halogenated $C_1$-$C_6$ alkyl, and $C_1$-$C_6$ alkoxy.

2. The compound, the stereoisomer, racemate thereof, or the pharmaceutically acceptable salt thereof of claim 1, wherein the compound has the structure of Formula II:

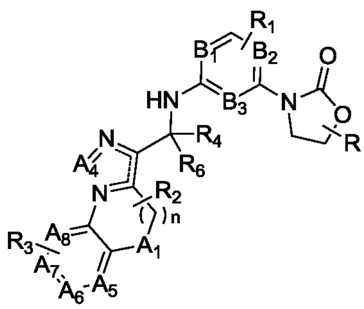

II

wherein the groups are as defined in claim 1.

3. The compound, the stereoisomer, racemate thereof, or the pharmaceutically acceptable salt thereof of claim 2, wherein the compound has the structure of Formula III:

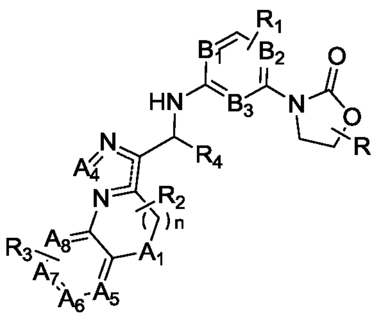

III

wherein the groups are as defined in claim 1.

4. The compound, the stereoisomer, racemate thereof, or the pharmaceutically acceptable salt thereof of claim 3, wherein the compound has the structure of Formula IV:

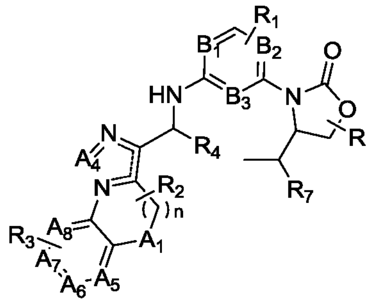

IV

wherein the groups are as defined in claim 1;

$R_7$ is selected from the group consisting of: H, hydroxyl, cyano, amino, halogen, substituted or unsubstituted C1-C4 alkyl, substituted or unsubstituted C1-C4 alkoxy, substituted or unsubstituted $C_3$-$C_{10}$ cycloalkyl, substituted or unsubstituted $C_6$-$C_{12}$ aryl, and substituted or unsubstituted 3 to 12-membered heteroaryl;
wherein each chiral center can be independently R configuration or S configuration;
said substituted refers to that one or more hydrogen atoms on the group are substituted by substituent(s) selected from the group consisting of: halogen, amino, cyano, $C_1$-$C_6$ alkyl, $C_1$-$C_6$ alkoxy, $C_1$-$C_6$ halogenated alkyl, carbonyl($C_{2-10}$)alkoxy, carbonyl($C_{7-10}$)aryloxy, acylamino($C_{2-10}$)alkyl, unsubstituted $C_6$-$C_{12}$ aryl or 3 to 12-membered heteroaryl, or $C_6$-$C_{12}$ aryl or 3 to 12-membered heteroaryl substituted by 1-3 substituents selected from the group consisting of: halogen, unsubstituted or halogenated $C_1$-$C_6$ alkyl, and $C_1$-$C_6$ alkoxy.
In another preferred embodiment, n=1, and $A_1$ is $CR_2$ or O.

**5.** The compound, the stereoisomer, racemate thereof, or the pharmaceutically acceptable salt thereof of claim 1, wherein R is selected from the group consisting of: H, halogen, CN, substituted or unsubstituted C1-C4 alkyl, substituted or unsubstituted C1-C4 alkoxy, substituted or unsubstituted $C_3$-$C_{10}$ cycloalkyl, substituted or unsubstituted $C_6$-$C_{12}$ aryl, and substituted or unsubstituted 3 to 12-membered heteroaryl; or two R groups together form a group selected from the group consisting of: substituted or unsubstituted $-(CH_2)_2-$ structure, and substituted or unsubstituted $-CH_2-O-CH_2-$ structure; or when two R groups are connected to the same carbon atom, the two R groups form a C=O double bond (carbonyl group) with the carbon atom;
$R_1$, $R_2$, $R_3$, $R_4$, $R_5$ are independently one or more groups that correspond to any position on the five-membered or six-membered ring and that are selected from the group consisting of: H, halogen, substituted or unsubstituted C1-C4 alkyl, and substituted or unsubstituted C1-C4 alkoxy.

**6.** The compound, the stereoisomer, racemate thereof, or the pharmaceutically acceptable salt thereof of claim 1, wherein the compound is selected from the group consisting of:

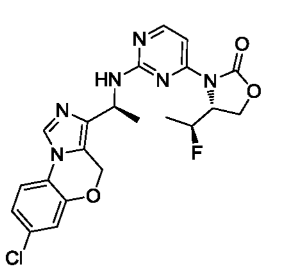

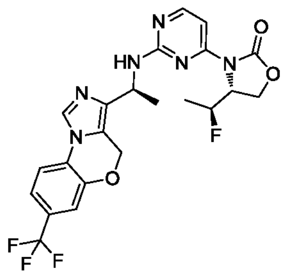

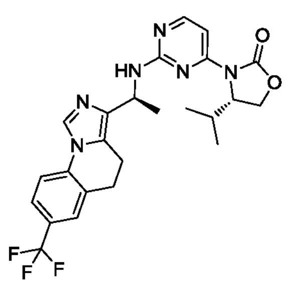

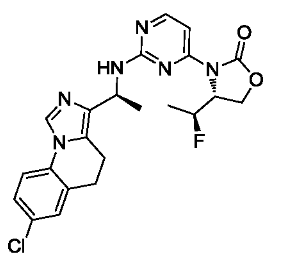

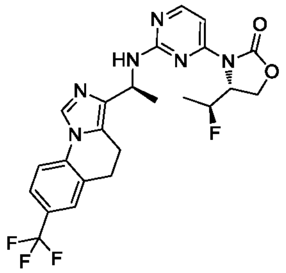

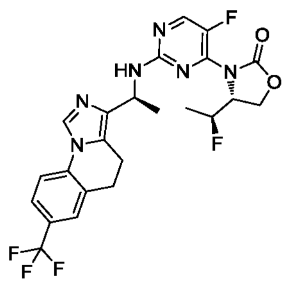

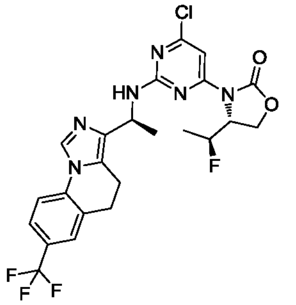

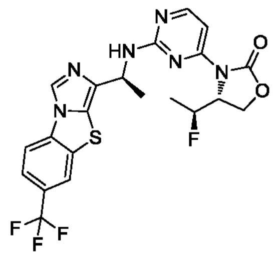

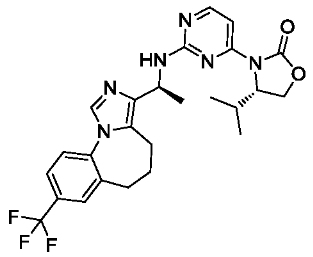

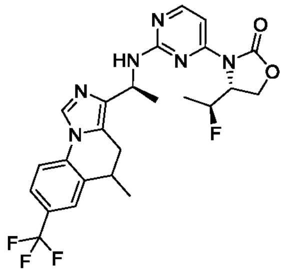

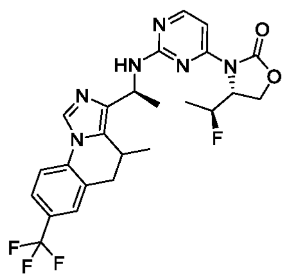

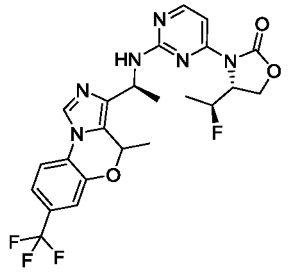

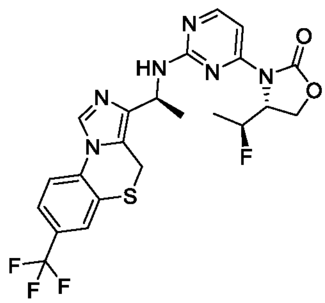

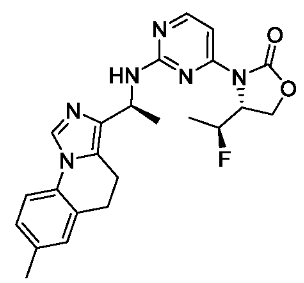

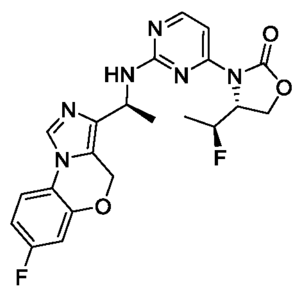

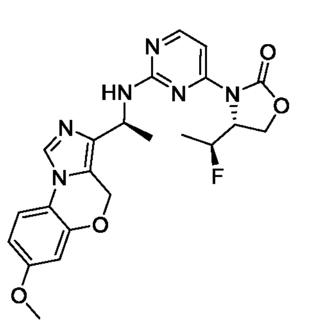

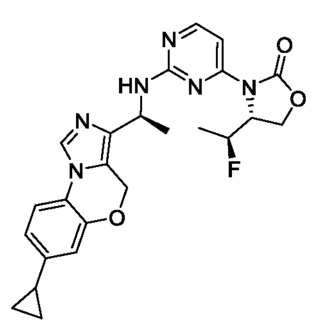

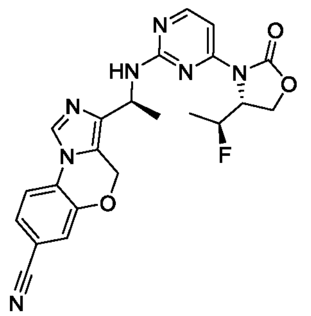

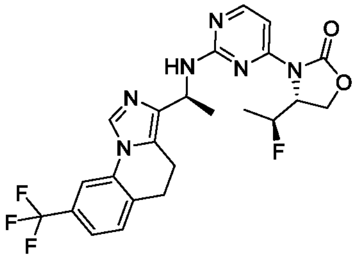
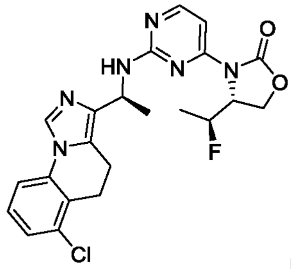
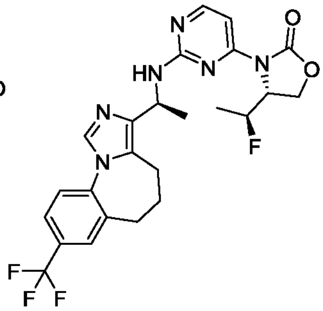

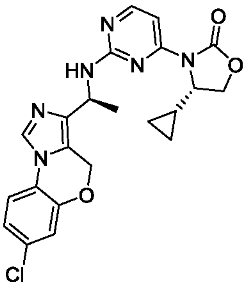
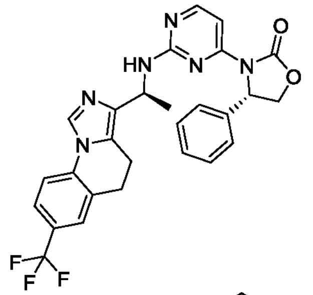
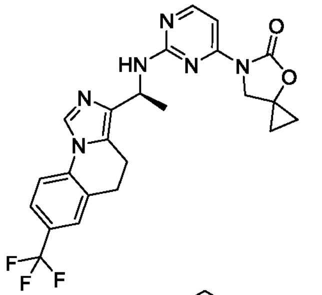

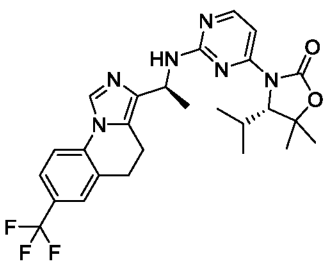
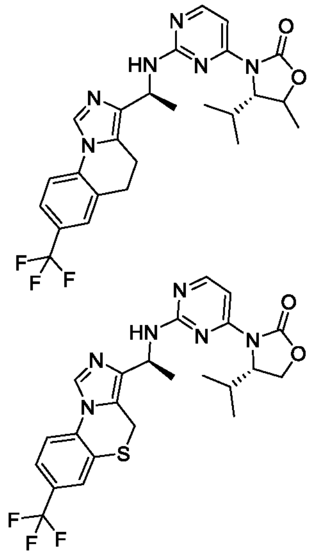
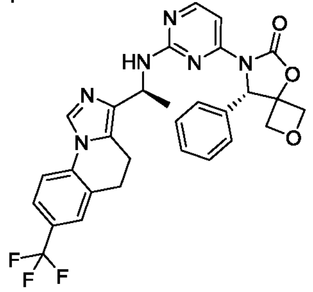

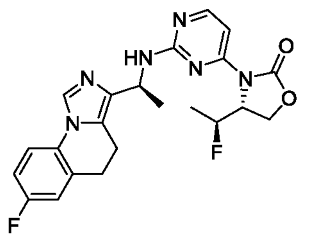

.

7. A pharmaceutical composition, wherein the pharmaceutical composition comprises: (a) the compound of Formula I of any one of claims 1-6 as an active ingredient, or a racemate, R-isomer, S-isomer or pharmaceutically acceptable salt thereof, or their mixture, or an isotope-substituted derivative thereof, and (b) a pharmaceutically acceptable excipient.

8. The pharmaceutical composition of claim 7, wherein the pharmaceutical composition further comprises (c) a second active ingredient.

9. The pharmaceutical composition of claim 7, wherein the pharmaceutical composition is used for the treatment or prevention of solid tumor(s) carrying IDH1 mutation, preferably, the pharmaceutical composition is used for the treatment or prevention of indication(s) selected from the group consisting of: brain glioma, glioblastoma, paraneuroma, acute leukemia, prostate cancer, thyroid cancer, colorectal cancer, chondrosarcoma, cholangiocarcinoma, leukemia, and melanoma.

10. Use of a compound of Formula I, or a racemate, R-isomer, S-isomer or pharmaceutically acceptable salt thereof, or an isotope-substituted derivative thereof, or their mixture in: (1) preparing a pharmaceutical composition for the treatment or prevention of diseases related to the activity or expression of mutant IDH; (2) preparing a inhibitor of mutant IDH.

**11.** Use of claim 10, wherein the diseases are solid tumor(s) carrying IDH1 mutation, preferably selected from the group consisting of: brain glioma, glioblastoma, paraneuroma, acute leukemia, prostate cancer, thyroid cancer, colorectal cancer, chondrosarcoma, cholangiocarcinoma, leukemia, and melanoma.

**12.** The pharmaceutical composition of claim 10, wherein the tumor(s) are selected from the group consisting of: neuroglioma, acute myeloid leukemia, sarcoma, prostate cancer, melanoma, non-small cell lung cancer, articular chondroma, and cholangioma.

**INTERNATIONAL SEARCH REPORT**

International application No. **PCT/CN2019/084373**

**A. CLASSIFICATION OF SUBJECT MATTER**

C07D 498/04(2006.01)i; C07D 513/04(2006.01)i; C07D 487/04(2006.01)i; A61K 31/542(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D498/-; C07D513/-; C07D487/-; A61K31/-; A61P35/-

Documentation searched other than minimum documentation to the extent that such documents are included in the fields searched

Electronic data base consulted during the international search (name of data base and, where practicable, search terms used)

CNABS, CNKI, DWPI, SIPOABS, REG(STN), CAPLUS(STN): 氨基嘧啶, 嘧啶氨基, 异柠檬酸脱氢酶, IDH1, 抑制剂, 瘤, 癌, pyrimidine, inhibitor, tumor, cancer, search according to the compound of claims 1 and 6

**C. DOCUMENTS CONSIDERED TO BE RELEVANT**

| Category* | Citation of document, with indication, where appropriate, of the relevant passages | Relevant to claim No. |
|---|---|---|
| A | CN 103958506 A (NOVARTIS AG) 30 July 2014 (2014-07-30)<br>claim 1, and description, pages 5, 175, 177, 178, 182, 212-213 and 281 | 1-12 |
| A | CN 105263929 A (NOVARTIS AG) 20 January 2016 (2016-01-20)<br>claim 1 | 1-12 |
| A | WO 2017140758 A1 (DEBIOPHARM INT S A ET AL.) 24 August 2017 (2017-08-24)<br>entire document | 1-12 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **20 July 2019** | **05 August 2019** |

| Name and mailing address of the ISA/CN | Authorized officer |
|---|---|
| **National Intellectual Property Administration, PRC (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2019/084373**

| Patent document cited in search report | Publication date (day/month/year) | Patent family member(s) | Publication date (day/month/year) |
|---|---|---|---|
| CN 103958506 A | 30 July 2014 | EP 2771337 B1 | 02 August 2017 |
| | | KR 20140069235 A | 09 June 2014 |
| | | GE P20166432 B | 10 February 2016 |
| | | CA 2849995 A1 | 04 April 2013 |
| | | MX 342326 B | 26 September 2016 |
| | | GT 201400057 A | 28 September 2017 |
| | | ZA 201402144 B | 27 September 2017 |
| | | CL 2014000746 A1 | 03 October 2014 |
| | | SG 11201400989T A | 28 April 2014 |
| | | TN 2014000128 A1 | 01 July 2015 |
| | | UY 34352 A | 30 April 2013 |
| | | US 8957068 B2 | 17 February 2015 |
| | | CU 24269 B1 | 08 August 2017 |
| | | EA 025183 B1 | 30 November 2016 |
| | | JP 2014528951 A | 30 October 2014 |
| | | DO P2014000058 A | 01 June 2014 |
| | | CR 20140143 A | 16 October 2014 |
| | | EP 2771337 A1 | 03 September 2014 |
| | | CN 103958506 B | 22 February 2017 |
| | | TW I547493 B | 01 September 2016 |
| | | AU 2012313888 A1 | 15 May 2014 |
| | | CO 6920291 A2 | 10 April 2014 |
| | | ES 2645968 T3 | 11 December 2017 |
| | | JP 6026544 B2 | 16 November 2016 |
| | | AP 3907 A | 23 November 2016 |
| | | CU 20140036 A7 | 30 July 2014 |
| | | AP 201407602 A0 | 30 April 2014 |
| | | MX 2014003752 A | 22 August 2014 |
| | | IL 231786 D0 | 28 May 2014 |
| | | AR 088175 A1 | 14 May 2014 |
| | | EA 201490696 A1 | 29 August 2014 |
| | | NZ 624040 A | 29 January 2016 |
| | | US 2014235620 A1 | 21 August 2014 |
| | | UA 111503 C2 | 10 May 2016 |
| | | WO 2013046136 A1 | 04 April 2013 |
| | | AU 2012313888 B2 | 31 March 2016 |
| | | MA 35452 B1 | 01 September 2014 |
| | | TW 201321375 A | 01 June 2013 |
| | | IL 231786 A | 29 June 2017 |
| | | BR 112014007310 A2 | 04 April 2017 |
| | | AP 201407602 D0 | 30 April 2014 |
| | | PE 15812014 A1 | 14 November 2014 |
| | | AU 2012313888 A8 | 24 July 2014 |
| CN 105263929 A | 20 January 2016 | AU 2014229313 A1 | 01 October 2015 |
| | | HK 1213251 A1 | 30 June 2016 |
| | | ES 2665619 T3 | 26 April 2018 |
| | | US 2016318915 A1 | 03 November 2016 |
| | | DO P2015000240 A | 15 December 2015 |
| | | TN 2015000392 A1 | 03 January 2017 |
| | | TW 201444831 A | 01 December 2014 |

Form PCT/ISA/210 (patent family annex) (January 2015)

**INTERNATIONAL SEARCH REPORT**
**Information on patent family members**

International application No.
**PCT/CN2019/084373**

| Patent document cited in search report | | | Publication date (day/month/year) | Patent family member(s) | | | Publication date (day/month/year) |
|---|---|---|---|---|---|---|---|
| | | | | PH | 12015502018 | A1 | 11 January 2016 |
| | | | | AP | 201508707 | D0 | 30 September 2015 |
| | | | | CN | 105263929 | B | 28 August 2018 |
| | | | | UY | 35470 | A | 31 October 2014 |
| | | | | AU | 2014229313 | B2 | 28 July 2016 |
| | | | | US | 2015152093 | A1 | 04 June 2015 |
| | | | | SG | 11201507063Y | A | 29 October 2015 |
| | | | | MX | 355945 | B | 07 May 2018 |
| | | | | AR | 095311 | A1 | 07 October 2015 |
| | | | | ZA | 201506452 | B | 27 July 2016 |
| | | | | AP | 201508707 | A0 | 30 September 2015 |
| | | | | CR | 20150491 | A | 29 January 2016 |
| | | | | CL | 2015002472 | A1 | 04 January 2016 |
| | | | | GE | P201706699 | B | 10 July 2017 |
| | | | | US | 2018051015 | A1 | 22 February 2018 |
| | | | | BR | 112015023412 | A2 | 18 July 2017 |
| | | | | KR | 20150131224 | A | 24 November 2015 |
| | | | | IL | 240972 | D0 | 30 November 2015 |
| | | | | EP | 2970240 | B1 | 10 January 2018 |
| | | | | JP | 6387360 | B2 | 05 September 2018 |
| | | | | CA | 2903979 | A1 | 18 September 2014 |
| | | | | MX | 2015012552 | A | 18 April 2016 |
| | | | | WO | 2014141104 | A1 | 18 September 2014 |
| | | | | PE | 19392015 | A1 | 08 January 2016 |
| | | | | US | 9688672 | B2 | 27 June 2017 |
| | | | | US | 10112931 | B2 | 30 October 2018 |
| | | | | US | 2014275083 | A1 | 18 September 2014 |
| | | | | EA | 201591745 | A1 | 29 January 2016 |
| | | | | JP | 2016513633 | A | 16 May 2016 |
| | | | | EP | 2970240 | A1 | 20 January 2016 |
| | | | | US | 9434719 | B2 | 06 September 2016 |
| | | | | BR | 112015023412 | A8 | 23 January 2018 |
| | | | | CU | 20150119 | A7 | 29 June 2016 |
| | | | | EA | 028033 | B1 | 29 September 2017 |
| WO | 2017140758 | A1 | 24 August 2017 | None | | | |

Form PCT/ISA/210 (patent family annex) (January 2015)